# EUROPEAN PATENT APPLICATION

(11) **EP 4 534 532 A1**
(43) Date of publication of application: **09.04.2025**
(21) Application number: 23812204.8
(22) Date of filing: 26.05.2023
(51) Int. Cl.: C07D 401/14, A61K 31/506, A61K 31/519, A61K 31/5377, A61K 31/439, A61P 35/00, C07D 498/22, C07D 405/14, C07D 471/10, C07D 409/14

(54) **HETEROARYL DERIVATIVE COMPOUND AND USES THEREOF**

(30) Priority: 27.05.2022 KR 20220065605
(71) Applicant: Voronoi Inc., Incheon 21984 (KR)
(72) Inventor: LEE, Younho, Incheon 21984 (KR); HWANG, Seonah, Incheon 21984 (KR); SON, Jungbeom, Incheon 21984 (KR); KIM, Namdoo, Incheon 21984 (KR); KIM, Sunghwan, Incheon 21984 (KR); KIM, Daekwon, Incheon 21984 (KR)
(74) Representative: Viering, Jentschura & Partner mbB Patent- und Rechtsanwälte
(86) International application number: PCT/KR2023/007286
(87) International publication number: WO 2023/229430

(57) **Abstract**

The present invention relates to a heteroaryl derivative and uses thereof. The heteroaryl derivative of the present invention exhibits excellent inhibitory activity against EGFR, and thus can be effectively used as a therapeutic agent for EGFR-related diseases.

## Description

### [Technical Field]

The present invention relates to a heteroaryl derivative compound and medical uses thereof. Specifically, the present invention relates to a heteroaryl derivative compound having EGFR inhibitory activity.

### [Background Art]

Protein kinases act as molecular switches to participate in signal transduction pathways, and the transition between active and inactive states of target proteins by kinases in cells should be smoothly controlled. If the transition between the active and inactive states is abnormally controlled, intracellular signal transduction is excessively activated or deactivated to induce uncontrollable cell division and proliferation. In particular, abnormal activation by mutation, amplification and/or overexpression of protein kinase genes causes the development and progression of various tumors or plays a crucial role in the development of various diseases such as inflammatory diseases, degenerative brain diseases, and autoimmune diseases.

Epidermal growth factor receptor (EGFR), a receptor tyrosine kinase of the ErbB family, is abnormally activated in many epithelial tumors, including non-small cell lung carcinoma (NSCLC), breast cancer, glioma, squamous cell carcinoma of the head and neck, colorectal cancer, rectal adenocarcinoma, head and neck cancer, stomach cancer, and prostate cancer, which is known that activation of the EGFR-tyrosine kinase causes sustained cell proliferation, invasion of surrounding tissues, distant metastasis, angiogenesis, and increases cell survival.

Meanwhile, it has been known that the EGFR mutation, EGFR Del19 or EGFR L858R, is a major cause of non-small cell lung cancer and head and neck cancer, and Iressa and Tarceva as therapeutic agents thereof have been developed and are currently used in clinical practice. However, when these drugs are used in patients, acquired resistance, which causes EGFR secondary mutations based on the structure of the drug, was observed, and it was also found that this resistance is a major cause of actual drug resistance. When first-generation EGFR inhibitors are administered for about 10 months on average, acquired resistance, called the T790M mutation located in the gatekeeper of EGFR kinase, occurs, and thus the first-generation EGFR inhibitors show no drug efficacy. In other words, EGFR Del19/T790M or EGFR L858R/T790M double mutations occur, preventing the existing therapeutic agents from exhibiting drug efficacy. In this regard, Osimertinib, a third-generation EGFR-TKI target drug that shows high reactivity against drug resistance due to the EGFR T790M mutation, has been developed, but this drug has also been reported to have drug resistance (Clin Cancer Res, 2015, 17:21). The EGFR C797S mutation has been suggested as one of the main mechanisms that cause drug resistance to osimertinib, and about 40% of clinical trial patients have been reported to have the EGFR C797S mutation (Nature Medicine, 2015, 21:560-562).

There is a growing unmet need for novel compounds capable of being utilized in the treatment of EGFR-related diseases by modulating EGFR activity (particularly C797S mutations such as EGFR Del19/T790M/C797S or EGFR L858R/T790M/C797S) .

### [Disclosure]

### [Technical Problem]

An object of the present invention is to provide a heteroaryl derivative having a novel structure, a stereoisomer thereof, or a pharmaceutically acceptable salt thereof.

Another object of the present invention is to provide a method for preparing the heteroaryl derivative compound.

Still another object of the present invention is to provide a pharmaceutical use of the heteroaryl derivative compound, and more particularly, to provide a pharmaceutical composition for treating or preventing EGFR-related diseases comprising the heteroaryl derivative compound as an active ingredient, the use of the compound for treating or preventing EGFR-related diseases using the compound, or a method for treating or preventing EGFR-related diseases comprising administering the compound.

### [Technical Solution]

In order to achieve the above purpose, the present inventors conducted extensive research with considerable efforts and completed the present invention by confirming that heteroaryl derivative compounds represented by Chemical Formula 1 described below inhibited the proliferation of EGFR-activated cells.

### Heteroaryl derivative compound

The present invention provides a compound represented by the following Chemical Formula 1, a stereoisomer thereof, or a pharmaceutically acceptable salt thereof: 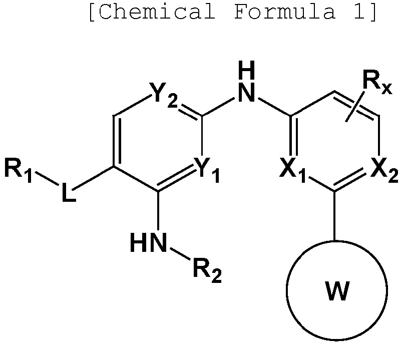 in Chemical Formula 1 above,
X₁ and X₂ are each independently CH or N;
Rx is -H, -C₁₋₆alkyl, -C₁₋₆hydroxyalkyl, -NH₂, -OH, -O-C₁₋₆alkyl, or -halo;
Y₁ and Y₂ are each independently CH or N;
ring W is cycloalkyl, heterocycloalkyl, aryl, or heteroaryl {wherein at least one H of the cycloalkyl, heterocycloalkyl, aryl, or heteroaryl ring may be substituted with -C₁₋₆alkyl, -C₁₋₆alkenyl, -C₁₋₆alkynyl, -C₁-₆alkyl-cycloalkyl, -C₁₋₆hydroxyalkyl, -OH, -O-C₁₋₆alkyl, =O, - C(=O)-H, -C(=O)-C₁₋₆alkyl, -C(=O)-cycloalkyl, -S(=O)₂-C₁-₆alkyl, -S(=O)₂-cycloalkyl, -halo, or cycloalkyl, and -CH₂-of the heterocycloalkyl ring may be substituted with - S(=O)₂-};
L is -C(=O)-, -C (=O)-NRₐ-, -C(=O)-O-, -NRₐ-C(=O)-, or -O-C(=O)-;
R₁ is -H, -C₁₋₆alkyl, -C₁₋₆cyanoalkyl, -C₁₋₆aminoalkyl, - C₁₋₆hydroxyalkyl, -C₁₋₆alkyl-O-C₁₋₆alkyl, -C₁₋₆hydroxyhaloalkyl, -C₁₋₆haloalkyl, -(CH₂)m-C(=O)-OC₁₋₆alkyl, -(CH₂)m-S(=O)₂-R_{b}, - (CH₂)m-cycloalkyl, -(CH₂)m-heterocycloalkyl, -(CH₂)m-aryl, or -(CH₂)m-heteroaryl {wherein at least one H of the -(CH₂)m-cycloalkyl, -(CH₂)m-heterocycloalkyl, -(CH₂)m-aryl, or - (CH₂)m-heteroaryl may be substituted with -C₁₋₆alkyl, -C₁₋₆cyanoalkyl, -C₁₋₆aminoalkyl, -C₁₋₆hydroxyalkyl, -C₁₋₆haloalkyl, -CN, -NO₂, -NH₂, -NH-C₁₋₆alkyl, -N(C₁₋₆alkyl)(C₁₋₆alkyl), -OH, -O-C₁₋₆alkyl, =O, -C(=O)-C₁₋₆alkyl, -C(=O)-OC₁₋₆alkyl, -S(=O)₂-C₁₋₆alkyl, -S(=O)₂-halo, -S(=O)₂-cycloalkyl, -C₁₋₆alkylS(=O)₂-C₁₋₆alkyl, -NR_{c}-S(=O)₂-C₁₋₆alkyl, -halo, -(CH₂)n-cycloalkyl, -(CH₂)n-heterocycloalkyl, -(CH₂)n-aryl, or - (CH₂)n-heteroaryl [wherein at least one H of the -(CH₂)n-cycloalkyl, -(CH₂)n-heterocycloalkyl, -(CH₂)n-aryl, or - (CH₂)n-heteroaryl ring may be substituted with -C₁₋₆alkyl, - C₁₋₆aminoalkyl, -C₁₋₆hydroxyalkyl, -C₁₋₆haloalkyl, =O, -S(=O)₂-C₁₋₆alkyl, -C₁₋₆alkyl-S(=O)₂-C₁₋₆alkyl, -halo, cycloalkyl, or heterocycloalkyl], and -CH₂- of the -(CH₂)m-cycloalkyl or - (CH₂)m-heterocycloalkyl may be substituted with -S(=O)₂-};
R₂ is -C₁₋₆alkyl, cycloalkyl, heterocycloalkyl, aryl, or heteroaryl {wherein at least one H of the cycloalkyl, heterocycloalkyl, aryl, or heteroaryl ring may be substituted with -C₁₋₆alkyl, -C₁₋₆aminoalkyl, -C₁₋₆hydroxyalkyl, -CN, -NO₂, -NH₂, -NH-C₁₋₆alkyl, -N(C₁₋₆alkyl) (C₁₋₆alkyl), -OH, -O-C₁₋₆alkyl, or -halo}, or R₂ may be connected to ring W to form a ring {wherein at least one H of the ring may be substituted with -C₁₋₆alkyl, -C₁₋₆aminoalkyl, -C₁₋₆hydroxyalkyl, -CN, -NO₂, -NH₂, -NH-C₁₋₆alkyl, -N(C₁₋₆alkyl)(C₁₋₆alkyl), -OH, -O-C₁₋₆alkyl, or -halo};
Rₐ is -H or -C₁₋₆alkyl;
R_{b} is -H, -C₁₋₆alkyl, -NH₂, -NH-C₁₋₆alkyl, or -N(C₁₋₆alkyl)(C₁₋₆alkyl);
R_{c} is -H or -C₁₋₆alkyl; and
m and n are each independently 0, 1, 2, 3, or 4.

According to an embodiment of the present invention, the compound represented by Chemical Formula 1, a stereoisomer thereof, or a pharmaceutically acceptable salt thereof may be in the following ranges:
X₁ and X₂ are each independently CH or N {wherein at least one of X₁ or X₂ is N};
Rx is -H or -C₁₋₆alkyl;
Y₁ and Y₂ are each independently CH or N {wherein at least one of Y₁ or Y₂ is N};
ring W is heterocycloalkyl, aryl or heteroaryl {wherein at least one H of the heterocycloalkyl, aryl or heteroaryl ring may be substituted with -C₁₋₆alkyl, -C₁₋₆alkyl-cycloalkyl, -OH, -O-C₁₋₆alkyl, =O, -C(=O)-H, -C(=O)-C₁₋₆alkyl, -C(=O)-cycloalkyl, -S(=O)₂-C₁₋₆alkyl, -S(=O)₂-cycloalkyl, -halo, or cycloalkyl, and -CH₂- of the heterocycloalkyl ring may be substituted with -S(=O)₂-};
L is -C(=O)-, -C(=O)-NRₐ-, -C(=O)-O-, -NRₐ-C(=O)-, or -O-C(=O)-;
R₁ is -H, -C₁₋₆alkyl, -C₁₋₆cyanoalkyl, -C₁₋₆aminoalkyl, - C₁₋₆hydroxyalkyl, -C₁₋₆alkyl-O-C₁₋₆alkyl, -C₁₋₆hydroxyhaloalkyl, -C₁₋₆haloalkyl, -(CH₂)m-C(=O)-OC₁₋₆alkyl, -(CH₂)m-S(=O)₂-R_{b}, - (CH₂)m-cycloalkyl, -(CH₂)m-heterocycloalkyl, -(CH₂)m-aryl, or -(CH₂)m-heteroaryl {wherein at least one H of the -(CH₂)m-cycloalkyl, -(CH₂)m-heterocycloalkyl, -(CH₂)m-aryl, or - (CH₂)m-heteroaryl may be substituted with -C₁₋₆alkyl, -C₁₋₆cyanoalkyl, -C₁₋₆aminoalkyl, -C₁₋₆hydroxyalkyl, -C₁₋₆haloalkyl, -CN, -NH₂, -NH-C₁₋₆alkyl, -N(C₁₋₆alkyl)(C₁₋₆alkyl), -C(=O)-C₁₋₆alkyl, -C(=O)-OC₁₋₆alkyl, -S(=O)₂-C₁₋₆alkyl, -S(=O)₂-halo, - S(=O)₂-cycloalkyl, -C₁₋₆alkyl-S(=O)₂-C₁₋₆alkyl, -NR_{c}-S(=O)₂-C₁₋₆alkyl, -halo, or -(CH₂)n-heterocycloalkyl [wherein at least one H of the -(CH₂)n-heterocycloalkyl ring may be substituted with -C₁₋₆alkyl or heterocycloalkyl], and -CH₂- of the - (CH₂)m-cycloalkyl or -(CH₂)m-heterocycloalkyl may be substituted with -S(=O)₂-};
R₂ is -C₁₋₆alkyl, cycloalkyl, heterocycloalkyl, or heteroaryl {wherein at least one H of the cycloalkyl, heterocycloalkyl, or heteroaryl ring may be substituted with -C₁₋₆alkyl}, or R₂ may be connected to ring W to form a ring {wherein at least one H of the ring may be substituted with -C₁₋₆alkyl};
Rₐ is -H or -C₁₋₆alkyl;
R_{b} is -H, -C₁₋₆alkyl, -NH₂, -NH-C₁₋₆alkyl, or -N(C₁₋₆alkyl)(C₁₋₆alkyl);
R_{c} is -H or -C₁₋₆alkyl; and
m and n are each independently 0, 1, 2, 3, or 4.

According to an embodiment of the present invention, the compound represented by Chemical Formula 1, a stereoisomer thereof, or a pharmaceutically acceptable salt thereof may be in the following ranges:
ring W is heterocycloalkyl selected from the group consisting of 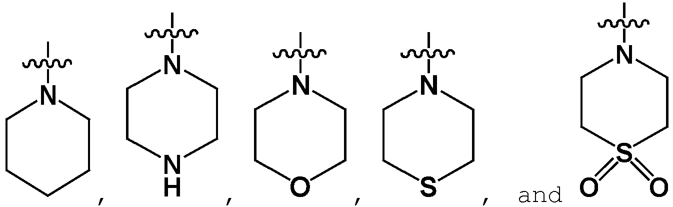 {wherein at least one H of the heterocycloalkyl ring may be substituted with -C₁₋₆alkyl, -OH, -O-C₁₋₆alkyl, =O, -S(=O)₂-C₁₋₆alkyl, -S(=O)₂-cycloalkyl, or -halo}.

According to an embodiment of the present invention, the compound represented by Chemical Formula 1, a stereoisomer thereof, or a pharmaceutically acceptable salt thereof may be in the following ranges:
ring W is an aryl or heteroaryl selected from the group consisting of phenyl and 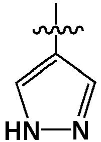 {wherein at least one H of the heteroaryl ring may be substituted with -C₁₋₆alkyl, -C₁₋₆hydroxyalkyl, -O-C₁₋₆alkyl, -S(=O)₂-C₁₋₆alkyl, or -S(=O)₂-cycloalkyl}.

According to an embodiment of the present invention, the compound represented by Chemical Formula 1 above may be selected from the group consisting of compounds listed in Table 2 below.

According to an embodiment of the present invention, the compound represented by Chemical Formula 1, a stereoisomer thereof, or a pharmaceutically acceptable salt thereof may be a compound represented by the following Chemical Formula A, a stereoisomer thereof, or a pharmaceutically acceptable salt thereof: 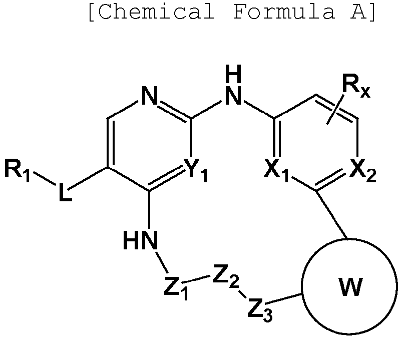 in Chemical Formula A above,
X₁ and X₂ are each independently CH or N;
Rx is -H, -C₁₋₆alkyl, -C₁₋₆hydroxyalkyl, -NH₂, -OH, -O-C₁₋₆alkyl, or -halo;
Y₁ is CH or N;
ring W is a 5-6 membered heteroaryl or phenyl {wherein at least one H of the 5-6 membered heteroaryl or phenyl ring may be substituted with -C₁₋₆alkyl, -C₁₋₆alkenyl, -C₁₋₆alkynyl, -C₁₋₆alkyl-cycloalkyl, -C₁₋₆hydroxyalkyl, -OH, -O-C₁₋₆alkyl, =O, -halo, or cycloalkyl, and -CH₂- of the heterocycloalkyl ring may be substituted with -S(=O)₂-};
Z₁ is -(CH₂CH₂)-, -(CH₂CH₂CH₂)-, or -(CH₂CH₂CH₂CH₂)-{wherein at least one H of the -(CH₂CH₂)-, -(CH₂CH₂CH₂), or - (CH₂CH₂CH₂CH₂) - may be substituted with -C₁₋₃alkyl};
Z₂ is -(CH₂)- or -O-;
Z₃ is a bond (null), -(CH₂)-, or -(CH₂CH₂)-,
L is -C(=O)-, -C(=O)-NRₐ-, -C(=O)-O-, -NRₐ-C(=O)-, or -O-C(=O)-;
R₁ is -H, -C₁₋₆alkyl, -C₁₋₆cyanoalkyl, -C₁₋₆aminoalkyl, - C₁₋₆hydroxyalkyl, -C₁₋₆alkyl-O-C₁₋₆alkyl, -C₁₋₆hydroxyhaloalkyl, -C₁₋₆haloalkyl, -(CH₂)m-C(=O)-OC₁₋₆alkyl, -(CH₂)m-S(=O)₂-R_{b}, - (CH₂)m-cycloalkyl, -(CH₂)m-heterocycloalkyl, -(CH₂)m-aryl, or -(CH₂)m-heteroaryl {wherein at least one H of the -(CH₂)m-cycloalkyl, -(CH₂)m-heterocycloalkyl, -(CH₂)m-aryl, or - (CH₂)m-heteroaryl may be substituted with -C₁₋₆alkyl, -C₁₋₆cyanoalkyl, -C₁₋₆aminoalkyl, -C₁₋₆hydroxyalkyl, -C₁₋₆haloalkyl, -CN, -NO₂, -NH₂, -NH-C₁₋₆alkyl, -N(C₁₋₆alkyl)(C₁₋₆alkyl), -OH, -O-C₁₋₆alkyl, =O, -S(=O)₂-C₁₋₆alkyl, -S(=O)₂-halo, -S(=O)₂-cycloalkyl, -C₁₋₆alkyl-S(=O)₂-C₁₋₆alkyl, -NR_{c}-S(=O)₂-C₁₋₆alkyl, -halo, -(CH₂)n-cycloalkyl, -(CH₂)n-heterocycloalkyl, - (CH₂)n-aryl, or -(CH₂)n-heteroaryl [wherein at least one H of the (CH₂)n-cycloalkyl, -(CH₂)n-heterocycloalkyl, -(CH₂)n-aryl, or -(CH₂)n-heteroaryl ring may be substituted with - C₁₋₆alkyl, -C₁₋₆aminoalkyl, -C₁₋₆hydroxyalkyl, -C₁₋₆haloalkyl, =O, -S(=O)₂-C₁₋₆alkyl, -C₁₋₆alkyl-S(=O)₂-C₁₋₆alkyl, -halo, cycloalkyl, or heterocycloalkyl], and -CH₂- of the -(CH₂)m-cycloalkyl or -(CH₂)m-heterocycloalkyl may be substituted with -S(=O)₂-};
Rₐ is -H or -C₁₋₆alkyl;
R_{b} is -H, -C₁₋₆alkyl, -NH₂, -NH-C₁₋₆alkyl, or -N(C₁₋₆alkyl)(C₁₋₆alkyl);
R_{c} is -H or -C₁₋₆alkyl; and
m and n are each independently 0, 1, 2, or 3.

According to an embodiment of the present invention, the compound represented by Chemical Formula A, a stereoisomer thereof, or a pharmaceutically acceptable salt thereof may be in the following ranges:
X₁ and X₂ are each independently CH or N {wherein at least one of X₁ or X₂ is N};
Rx is -H or -C₁₋₆alkyl;
Y₁ is CH or N;
ring W is a 5-6 membered heteroaryl {wherein at least one H of the 5-6 membered heteroaryl ring may be substituted with -C₁₋₆alkyl or cycloalkyl};
Z₁ is -(CH₂CH₂)-, -(CH₂CH₂CH₂)-, or -(CH₂CH₂CH₂CH₂)-{wherein at least one H of the -(CH₂CH₂)-, -(CH₂CH₂CH₂), or - (CH₂CH₂CH₂CH₂)- may be substituted with -C₁₋₃alkyl};
Z₂ is -(CH₂)- or -O-;
Z₃ is a bond (null), -(CH₂)-, or -(CH₂CH₂)-,
L is -C(=O)-, -C (=O) -NRₐ-, or -C (=O) -O-;
R₁ is -H, -C₁₋₆alkyl, -C₁₋₆cyanoalkyl, -C₁₋₆haloalkyl, - (CH₂)m-S(=O)₂-R_{b}, -(CH₂)m-cycloalkyl, -(CH₂)m-heterocycloalkyl, or -(CH₂)m-aryl {wherein at least one H of the -(CH₂)m-cycloalkyl, -(CH₂)m-heterocycloalkyl, or -(CH₂)m-aryl may be substituted with -C₁₋₆alkyl, -C₁₋₆cyanoalkyl, -C₁₋₆aminoalkyl, -C₁₋₆hydroxyalkyl, -C₁₋₆haloalkyl, -S(=O)₂-C₁₋₆alkyl, -C₁₋₆alkyl-S(=O)₂-C₁₋₆alkyl, -NR_{c}-S(=O)₂-C₁₋₆alkyl, - halo, or -(CH₂)n-heterocycloalkyl [wherein at least one H of the -(CH₂)n-heterocycloalkyl ring may be substituted with - C₁₋₆alkyl or heterocycloalkyl], and -CH₂- of the -(CH₂)m-cycloalkyl or -(CH₂)m-heterocycloalkyl may be substituted with -S(=O)₂-};
Rₐ is -H or -C₁₋₆alkyl;
R_{b} is -H or -C₁₋₆alkyl;
R_{c} is -H or -C₁₋₆alkyl; and
m and n are each independently 0, 1, 2, or 3.

According to an embodiment of the present invention, the compound represented by Chemical Formula A, a stereoisomer thereof, or a pharmaceutically acceptable salt thereof may be in the following ranges:
ring W is 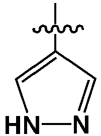 or phenyl {wherein at least one H of the 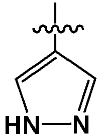 or phenyl ring may be substituted with -C₁₋₆alkyl, -C₁₋₆hydroxyalkyl, -O-C₁₋₆alkyl, -S(=O)₂-C₁₋₆alkyl, or -S(=O)₂-cycloalkyl}.

In the present invention, "alkyl" may refer to, unless otherwise indicated, a straight or branched chain acyclic, cyclic, or saturated hydrocarbon to which they are bonded. For example, "C₁₋₆alkyl" may refer to an alkyl containing 1 to 6 carbon atoms. The acyclic alkyl may include, for example, methyl, ethyl, n-propyl, n-butyl, isopropyl, sec-butyl, isobutyl, tert-butyl, and the like, but is not limited thereto. The cyclic alkyl may be used interchangeably with "cycloalkyl" in the present specification, and may include, as an example, cyclopropyl, cyclobutyl, cyclopentyl, cyclohexyl, cycloheptyl, cyclooctyl, or the like, but is not limited thereto.

In the present invention, "alkenyl" may refer to, unless otherwise indicated, an unsaturated hydrocarbon comprising at least one double bond. For example, "C₂₋₆alkenyl" may include from 2 to 6 carbon atoms and may include at least one double bond. As an example, it may include ethenyl, prop-1-enyl, but-1-enyl, pent-1-enyl, hex-1-enyl, and the like, but is not limited thereto.

In the present invention, "alkynyl" may refer to, unless otherwise indicated, an unsaturated hydrocarbon comprising at least one triple bond. For example, "C₂₋₆alkynyl" may include from 2 to 6 carbon atoms and may include at least one triple bond. As an example, it may include ethynyl, prop-1-ynyl, but-1-ynyl, pent-1-ynyl, hex-1-ynyl, and the like, but is not limited thereto.

In the present invention, "alkoxy" may refer to -(O-alkyl) as an alkyl ether group, wherein the alkyl is the same as defined above. For example, "C₁₋₆alkoxy" may refer to an alkoxy containing C₁₋₆alkyl, i.e., -(O-C₁₋₆alkyl); and as an example, the alkoxy may include, but are not limited to, methoxy, ethoxy, n-propoxy, isopropoxy, n-butoxy, isobutoxy, sec-butoxy, tert-butoxy, and the like.

In the present invention, "halo" may be F, Cl, Br, or I.

In the present invention, "haloalkyl" may refer to a straight or branched chain alkyl (hydrocarbon) having one or more carbon atoms substituted with halo as defined herein. Examples of the haloalkyl include methyl, ethyl, propyl, isopropyl, isobutyl or n-butyl, independently substituted with one or more halogens, such as F, Cl, Br, or I, but are not limited to.

In the present invention, "hydroxyalkyl" may refer to a straight or branched chain alkyl (hydrocarbon) having a carbon atom substituted with hydroxyl (-OH). Examples of the hydroxyalkyl include methyl, ethyl, propyl, isopropyl, isobutyl or n-butyl independently substituted with -OH, but are not limited to.

In the present invention, "aminoalkyl" may refer to a straight or branched chain alkyl (hydrocarbon) having a carbon atom substituted with amino- (NR'R"). Here, R' and R" may each independently be selected from the group consisting of hydrogen, C₁₋₆alkyl, and N protecting groups (e.g., Boc), wherein the selected R' and R" may each independently be substituted or unsubstituted.

In the present invention, "cyanoalkyl" may refer to a straight or branched chain alkyl (hydrocarbon) having a carbon atom substituted with cyano (-CN).

In the present invention, "heterocycloalkyl" may refer to a ring containing one or more selected from N, O, P, P(=O), and S within the ring, and may be saturated or partially unsaturated. Here, when unsaturated, the heterocycloalkyl may be referred to as a heterocycloalkene. Unless otherwise stated, the heterocycloalkyl may be a single ring or multiple rings such as spiro rings, bridged rings, or fused rings. Further, "3-12 membered heterocycloalkyl" may refer to a heterocycloalkyl containing 3 to 12 atoms forming a ring, and as an example, the heterocycloalkyl may include pyrrolidine, piperidine, imidazolidine, pyrazolidine, butyrolactam, valerolactam, imidazolidinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, quinuclidine, tropane, 2-azaspiro[3.3]heptane, (1R,5S)-3-azabicyclo[3.2.1]octane, (1S,4S)-2-azabicyclo[2.2.2]octane, or (1R,4R)-2-oxa-5-azabicyclo[2.2.2]octane, and the like, but is not limited to.

In the present invention, "arene" may refer to an aromatic hydrocarbon ring. The arene may be monocyclic arene or polycyclic arene. The number of carbon atoms forming the arene ring may be 5 or more and 30 or less, 5 or more and 20 or less, or 5 or more and 15 or less. Examples of arene may include benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like, but are not limited to. In the present specification, a moiety obtained by removing one hydrogen atom from the above "arene" is referred to as "aryl".

In the present invention, "heteroarene" may be a ring containing one or more of O, N, P, Si, and S as heteroatoms. The number of carbon atoms forming the heteroarene ring may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of the heteroarene may include thiophene, purine, pyrrole, pyrazole, imidazole, thiazole, oxazole, isothiazole, oxadiazole, triazole, pyridine, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine or pyrazolopyridine, N-arylcarbazole, N-heteroarylcarbazole, N-alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilole, dibenzofuran, and the like, but are not limited to. In an embodiment of the present invention, the heteroarene may also include a bicyclic heterocyclo-arene including an arene ring fused to a heterocycloalkyl ring or a heteroarene fused to a cycloalkyl ring. In the present specification, a moiety obtained by removing one hydrogen atom from the above "heteroarene" is referred to as "heteroaryl".

In the present invention, "stereoisomer" refers to compounds that have the same chemical or molecular formula but are sterically different. Stereoisomer in the present specification include optical isomer, enantiomer, diastereomer, cis/trans isomer, rotamer, and atropisomer, and each of these isomers, racemates, and mixtures thereof are also included within the scope of the present invention. For example, since the stereochemical structure of Chemical Formula 1 of the present invention is not specified, the stereoisomers of Chemical Formula 1 may be included. Unless otherwise specified, a solid bond ( 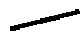 ) connected to an asymmetric carbon atom may include a wedge solid bond ( 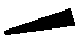 ) or wedge dashed bond ( 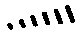 ) representing the absolute arrangement of stereocenters.

The compound represented by Chemical Formula 1 of the present invention may be present in the form of a "pharmaceutically acceptable salt". Thus, pharmaceutically acceptable salts of the compound represented by Chemical Formula 1 above are included in the scope of the compound of the present invention. The term "pharmaceutically acceptable salt" of the present invention refers to any organic or inorganic acid addition salt of the compound represented by Chemical Formula 1 at a concentration having a relatively non-toxic and harmless effective effect on patients in which side effects caused by these salts do not reduce the beneficial efficacy of the compound.

In particular, the pharmaceutically acceptable salt may be an acid addition salt formed from a free acid. Here, the acid addition salts may be obtained from inorganic acids such as hydrochloric acid, nitric acid, phosphoric acid, sulfuric acid, hydrobromic acid, hydroiodic acid, nitrous acid, hypophosphorous acid, and the like, non-toxic organic acids such as aliphatic mono- and dicarboxylates, phenyl-substituted alkanoates, hydroxy alkanoates and alkanedioates, aromatic acids, aliphatic and aromatic sulfonic acids, and the like, organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, and the like.

Examples of the pharmaceutically acceptable salt may include sulfate, sulfite, nitrate, phosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, benzoate, phthalate, benzenesulfonate, toluene sulfonate, phenylacetate, phenylpropionate, phenylbutyrate, citrate, lactate, glycolate, malate, tartrate, mandelate, and the like.

The acid addition salt may be prepared by a conventional method, for example, may be prepared by dissolving the derivative represented by Chemical Formula 1 in an organic solvent such as methanol, ethanol, acetone, methylene chloride, acetonitrile, or the like, and adding an organic acid or an inorganic acid to form a precipitate, followed by filtering and drying the resulting precipitate, or may be prepared by distillation using a solvent and excess acid under reduced pressure, followed by drying and crystallizing the reaction product in an organic solvent.

Further, the pharmaceutically acceptable salt may be a salt obtained using a base or a metal salt. As an example of the metal salt, an alkali metal salt or an alkaline earth metal salt may be obtained by dissolving the compound in an excess alkali metal hydroxide or alkaline earth metal hydroxide solution, and filtering an insoluble compound salt, followed by evaporating and drying the filtrate. Sodium, potassium or calcium salts may be pharmaceutically suitable as alkali metal salts. In addition, the corresponding salts may be obtained by reacting an alkali metal or alkaline earth metal salt with a suitable silver salt (for example, silver nitrate) or may be prepared by salt production methods known in the art.

### Use of heteroaryl derivative compound

The present invention provides use of a compound represented by the following Chemical Formula 1, a stereoisomer thereof, or a pharmaceutically acceptable salt thereof:

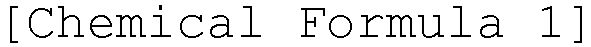

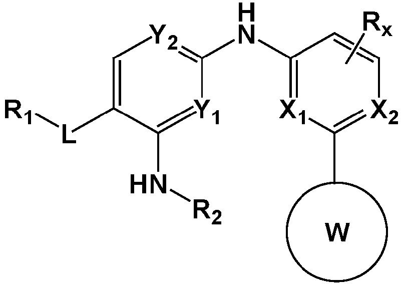

The compound represented by Chemical Formula 1 of the present invention, a stereoisomer thereof, or a pharmaceutically acceptable salt thereof exhibits inhibitory activity against various kinases.

According to an embodiment of the present invention, the heteroaryl derivative represented by Chemical Formula 1 above exhibits excellent inhibitory activity against EGFR kinase, and therefore can be effectively used for the treatment or prevention of EGFR-related diseases, particularly cancer. In particular, the heteroaryl derivative compound of the present invention exhibits excellent inhibitory activity against EGFR mutations (e.g., EGFR Del19/C797S, EGFR L858R/C797S, EGFR Del19/T790M/C797S, or EGFR L858R/T790M/C797S, etc.), and therefore can be usefully employed for the treatment or prevention of carcinomas induced by EGFR.

In the present invention, cancer includes all cancers capable of exhibiting preventive or therapeutic efficacy due to inhibition of EGFR kinase activity, and may be a solid cancer or a hematological cancer. For example, the cancer may be one or more types selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone tumor, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid tumor, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, hematological cancer, and thymic carcinoma, but is not limited thereto. In addition, the cancer includes primary cancer as well as metastatic cancer.

According to an embodiment of the present invention, the present invention provides a pharmaceutical composition for treating or preventing EGFR-related diseases, comprising the compound represented by Chemical Formula 1 as described above, a stereoisomer thereof, or a pharmaceutically acceptable salt thereof, as an active ingredient. Specifically, the EGFR-related disease may be cancer. The types of cancer are as described above.

The pharmaceutical composition of the present invention may further include at least one active ingredient exhibiting the same or similar efficacy in addition to the compound represented by Chemical Formula 1 as described above, a stereoisomer or a pharmaceutically acceptable salt thereof.

The pharmaceutical composition of the present invention may be used for clinical administration and may be prepared for administration in various oral and parenteral dosage forms.

In addition, according to an embodiment of the present invention, the present invention provides the use of the compound represented by Chemical Formula 1 as described above, a stereoisomer thereof, or a pharmaceutically acceptable salt thereof, for the manufacture of a medicament for the treatment or prevention of EGFR-related diseases. Specifically, the EGFR-related disease may be cancer. The types of cancer are as described above.

Further, according to an embodiment of the present invention, the present invention provides the use of the compound represented by Chemical Formula 1 as described above, a stereoisomer thereof, or a pharmaceutically acceptable salt thereof, for use in the manufacture of a medicament for the treatment or prevention of cancer. The types of cancer are as described above.

Further, according to an embodiment of the present invention, the present invention provides a method for treating or preventing EGFR-related diseases, comprising administering to a subject in need thereof a therapeutically effective amount of the compound represented by Chemical Formula 1 as described above, a stereoisomer thereof, or a pharmaceutically acceptable salt thereof. The subject may be a mammal including a human. Specifically, the EGFR-related disease may be cancer. The types of cancer are as described above.

Further, according to an embodiment of the present invention, the present invention provides a method for treating or preventing cancer, comprising administering to a subject in need thereof a therapeutically effective amount of the compound represented by Chemical Formula 1 as described above, a stereoisomer thereof, or a pharmaceutically acceptable salt thereof. The types of cancer are as described above.

Further, according to an embodiment of the present invention, the present invention provides a method for inhibiting EGFR, comprising administering to a subject in need thereof a therapeutically effective amount of the compound represented by Chemical Formula 1 as described above, a stereoisomer thereof, or a pharmaceutically acceptable salt thereof.

As used herein, the term "therapeutically effective amount" refers to an amount of the compound represented by Chemical Formula 1 that is effective for the treatment or prevention of EGFR-related diseases. Specifically, the "therapeutically effective amount" means an amount sufficient to treat a disease at a reasonable benefit/risk ratio applicable to medical treatment, and the effective dose level may be determined depending on factors including the subject type and severity, age, sex, type of diseases, the activity of the drug, the sensitivity to the drug, the time of administration, the route of administration, the rate of excretion, the duration of treatment, drugs used concurrently, and other factors well known in the medical field. The pharmaceutical composition of the present invention may be administered as an individual therapeutic agent or in combination with other therapeutic agents, or may be administered sequentially or simultaneously with a commercially available therapeutic agent. In addition, the pharmaceutical composition of the present invention may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present invention may be determined by specialists according to various factors such as the patient's condition, age, sex, complications, and the like. Since the active ingredient of the pharmaceutical composition of the present invention has excellent safety, the active ingredient may be used even above the predetermined dose.

In the present invention, the term "prevention" refers to any action that suppresses or delays the occurrence, spread, and recurrence of the disease by administering the compound, and term "treatment" refers to any action in which the symptoms of the disease are improved or beneficially changed by administration of the compound.

Further, according to an embodiment of the present invention, the pharmaceutical composition may further comprise a pharmaceutically acceptable carrier, diluent, or excipient. In an embodiment, the present invention provides a pharmaceutical composition comprising the compound represented by Chemical Formula 1, or a pharmaceutically acceptable salt or stereoisomer thereof, and a pharmaceutically acceptable additive.

Examples of additives used in the pharmaceutical composition may include sweeteners, binders, solvents, solubilizers, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The pharmaceutical composition may be combined in various formulation forms for oral administration (for example, tablets, pills, powders, capsules, syrups or emulsions) or parenteral administration (for example, intramuscular, intravenous or subcutaneous injection).

For example, the pharmaceutical composition may be combined into a formulation for oral administration, and additives used herein may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, and the like. Specifically, solid preparations for oral administration may include tablets, pills, powders, granules, capsules, and the like, and these solid preparations may be formulated by mixing at least one excipient in the composition, such as starch, calcium carbonate, sucrose, lactose, gelatin, etc. In addition, lubricants such as magnesium stearate and talc may be used in addition to simple excipients. Further, examples of liquid preparations for oral administration may include suspensions, emulsions, syrups, and the like. Various excipients such as wetting agents, sweeteners, aromatics, preservatives, and the like, may be included in addition to water and liquid paraffin that are commonly used simple diluents.

Further, preparations for parenteral administration may include sterilized aqueous solutions, non-aqueous solutions, suspensions, emulsions, freeze-dried preparations, and suppositories. As the non-aqueous solvents and the suspensions, propylene glycol, polyethylene glycol, vegetable oils such as olive oil, and injectable esters such as ethyl oleate, and the like, may be used. As the base of the suppository, witepsol, macrogol, tween 61, cacao butter, laurin butter, glycerogelatin and the like, may be used. Meanwhile, conventional additives such as solubilizers, isotonic agents, suspending agents, emulsifiers, stabilizers, preservatives, and the like, may be contained in the injection.

It may also be formulated in combination formulations with other active agents to achieve synergistic action of the active ingredients.

Matters described in the use, composition, and treatment method of the present invention are equally applied unless they contradict each other.

### [Advantageous Effects]

The heteroaryl derivative compound of the present invention may exhibit excellent inhibitory activity against EGFR, and may therefore be useful in the treatment or prevention of the EGFR-related diseases.

### [Best Mode]

Hereinafter, the present invention will be described in detail by Examples and Experimental Examples. However, the following Examples and Experimental Examples are only provided to illustrate the present invention, and the scope of the present invention is not limited thereto.

### <Analysis and purification conditions>

Compounds synthesized in Examples of the present invention were purified or subjected to structural analysis under the following conditions.

### 1. HPLC and MPLC

### HPLC for analysis (ACQUITY UPLC H-Class Core System)

Waters UPLC system (ACQUITY UPLC PDA Detector) equipped with a mass QDa detector from Waters was used. Waters' ACQUITY UPLC^{®} BEH C18 (1.7 µm, 2.1 × 50 mm) column was used, and the column temperature was set at 30°C.

Water containing 0.1% formic acid was used as mobile phase A, and acetonitrile containing 0.1% formic acid was used as mobile phase B.

Gradient condition (10-100% B for 3 minutes, flow rate = 0.6 ml/min)

### Preparative HPLC System for purification (Preparative-Liquid chromatography UV spectrometry)

The ACCQPrep HP150 equipment manufactured by Teledyne ISCO Inc., was used. Waters' XTERRA^{®} Prep RP18 OBD^{™} (10 µm, 30 × 300 mm) column was used, and the column temperature was set to room temperature.

Gradient condition (10-100% B for 120 minutes, flow rate = 42 ml/min)

### Medium pressure liquid chromatography (MPLC) for purification

Medium pressure liquid chromatography was performed using Teledyne ISCO's CombiFlash Rf +UV.

### 2. NMR analysis

NMR analysis was performed using AVANCE III 400 or AVANCE III 400 HD manufactured by Bruker, and data were expressed in parts per million (δ) (ppm).

The commercial reagents used were used without further purification. In the present invention, ambient temperature or room temperature refers to a temperature of about 5°C to 40°C, as an example, 10°C to 30°C, and as another example, 20°C to 27°C, and is not strictly limited to the above range. Concentration under reduced pressure or solvent distillation was performed using a rotary evaporator.

### Preparation Example: Preparation of intermediate compound of the present invention

### <Preparation Example 1> Preparation of 2-(cis-3-fluoro-4-methoxypiperidin-1-yl)pyrimidin-4-amine

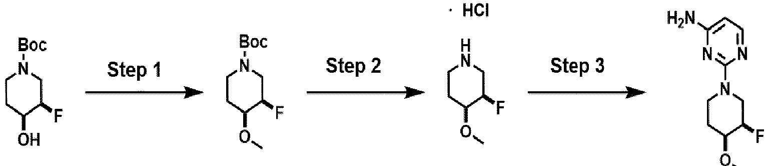

### Step 1: Preparation of cis-tert-butyl 3-fluoro-4-methoxypiperidin-1-carboxylate

Cis-tert-butyl 3-fluoro-4-hydroxypiperidin-1-carboxylate (1 g, 1.0 eq.) was dissolved in tetrahydrofuran (THF; 23 mL), and sodium hydride (NaH; 0.22 g, 60% purity, 1.2 eq.) was added under a nitrogen atmosphere at 0°C. The reaction mixture was stirred at 0°C for 30 minutes, and methyl iodide (0.31 mL, 1.1 eq.) was added dropwise at 0°C. Then, the reaction mixture was stirred at room temperature for 3 hours. The LCMS analysis confirmed the complete consumption of the starting material and the detection of the target compound. The reaction mixture was concentrated under reduced pressure, and ethyl acetate (EA; 100 mL) and saturated aqueous sodium bicarbonate solution (200 mL * 2) were added to extract the organic layer. The organic layer was dried over sodium sulfate and concentrated under reduced pressure to obtain the target compound (1.0 g, 94% yield) as a yellow oil.
MS: m/z 234.3 [M+H]⁺

### Step 2: Preparation of cis-3-fluoro-4-methoxypiperidine hydrochloride

The cis-tert-butyl 3-fluoro-4-methoxypiperidin-1-carboxylate (1.0 g, 1.0 eq.) obtained in Step 1 was dissolved in dichloromethane (DCM; 24 mL), and then HCl/dioxane (4M, 3.7 mL, 3.0 eq.) was added at 0°C. Then, the reaction mixture was stirred at room temperature for 12 hours. The LCMS analysis confirmed the complete consumption of the starting material and the detection of the target compound. The reaction mixture was concentrated under reduced pressure and diethyl ether (60 mL) was added to obtain a solid. The precipitate was filtered and dried to afford the target compound (0.56 g, 77% yield) as a white solid.
MS: m/z 134.3 [M+H]⁺

### Step 3: Preparation of 2-(cis-3-fluoro-4-methoxypiperidin-1-yl)pyrimidin-4-amine

The cis-3-fluoro-4-methoxypiperidine hydrochloride (0.56 g, 1.0 eq.) and 2-chloropyrimidin-4-amine (0.32 g, 0.8 eq.) obtained in Step 2 were dissolved in isopropyl alcohol (IPA; 8 mL) at room temperature, and N,N-diisopropylethylamine (DIPEA; 2.52 mL, 4.0 eq.) was added. The reaction mixture was stirred at 120°C for 12 hours. The LCMS analysis confirmed the complete consumption of the starting material and the detection of the target compound. The reaction mixture was concentrated under reduced pressure and the concentrated compound was purified by MPLC (hexane/ethyl acetate) to afford the target compound (0.59 g, 83% yield) as a white solid.

¹H NMR (400 MHz, CDCl₃) δ 7.92 (d, *J* = 5.6 Hz, 1H), 5.75 (d, *J* = 5.6 Hz, 1H), 4.77 (ddtd, *J* = 48.3, 6.2, 2.6, 0.9 Hz, 1H), 4.62 (s, 2H), 4.50 (dddd, *J* = 14.0, 7.8, 6.2, 1.4 Hz, 1H), 4.21 (dddd, *J* = 13.5, 6.5, 3.9, 1.4 Hz, 1H), 3.67-3.49 (m, 2H), 3.47 (s, 3H), 3.40 (dddd, *J* = 13.7, 8.8, 3.7, 1.8 Hz, 1H), 1.99-1.88 (m, 1H), 1.82-1.72 (m, 1H).

MS: m/z 227.3 [M+H]⁺

### <Preparation Example 2> Preparation of 2-(1-(cyclopropylsulfonyl)-1H-pyrazol-4-yl)pyrimidin-4-amine

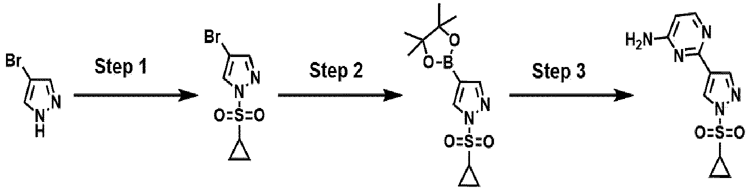

### Step 1: Preparation of 4-bromo-1-(cyclopropylsulfonyl)-1H-pyrazole

4-Bromo-1H-pyrazole (10 g, 1 eq.) was dissolved in dichloromethane (DCM; 170 mL), and cyclopropanesulfonyl chloride (7.62 mL, 1.1 eq.) and triethylamine (TEA; 12.33 mL, 1.3 eq.) were added thereto. The reaction mixture was stirred at room temperature for 19 hours. The LCMS analysis confirmed the complete consumption of the starting material and the detection of the target compound. Water (100 mL) was added to the reaction mixture, and the resulting mixture was extracted using dichloromethane (DCM; 200 mL). The organic layer was concentrated under reduced pressure and purified by MPLC (hexane/ethyl acetate) to obtain the target compound (10 g, 59% yield).
MS: m/z 250.3 [M+H]⁺

### Step 2: Preparation of 1-(cyclopropylsulfonyl)-4-(4,4,5,5-tetramethyl-1,3,2-dioxaborolan-2-yl)-1H-pyrazole

The 4-bromo-1-(cyclopropylsulfonyl)-1H-pyrazole (10 g, 1 eq.) obtained in Step 1 was dissolved in 1,4-dioxane (133 mL), and then bis(pinacolato)diboron (12.14 g, 1.2 eq.) and potassium acetate (7.82 g, 2 eq.) were added. The reaction mixture was degassed for 10 minutes, and [1,1'-bis(diphenylphosphino)ferrocene]palladium(II) dichloride (1.17 g, 0.04 eq.) was then added. Next, the reaction mixture was stirred at 110°C for 16 hours under a nitrogen atmosphere. LCMS analysis results showed that all starting materials disappeared and the target compound was detected. The reaction mixture was passed through diatomaceous earth for filtration. The filtrate was concentrated under reduced pressure to obtain the target compound (11 g, 93%), which was used in the next reaction without purification.
MS: m/z 299.3 [M+H]⁺

### Step 3: Preparation of 2-(1-(cyclopropylsulfonyl)-1H-pyrazol-4-yl)pyrimidin-4-amine

The 1-(cyclopropylsulfonyl)-4-(4,4,5,5-tetramethyl-1,3,2-dioxaborolan-2-yl)-1H-pyrazole (2.3 g, 1 eq.) obtained in Step 2 above was dissolved in acetonitrile (ACN; 15 mL), and then 2-chloropyrimidin-4-amine (1 g, 1 eq.) and aqueous sodium carbonate solution (2 M, 11.57 mL, 3 eq.) were added. The reaction mixture was degassed for 10 minutes, and bis[di-tert-butyl(4-dimethylaminophenyl)phosphine]dichloropalladium (0.109 g, 0.02 eq.) was then added. The reaction mixture was stirred at 100°C for 2 hours under a nitrogen atmosphere. LCMS analysis results showed that all starting materials disappeared and the target compound was detected. The reaction mixture was passed through diatomaceous earth for filtration. The filtrate was concentrated under reduced pressure and purified by MPLC (ethyl acetate/dichloromethane) to obtain the target compound (1 g, 49% yield).
MS: m/z 266.3 [M+H]⁺

### <Preparation Examples 3 to 15>

Compounds of Preparation Examples 3 to 9 below were prepared using a method similar to the above <Preparation Example 1>. Further, compounds of Preparation Examples 10 to 15 below were prepared using a method similar to the above <Preparation Example 2>. The compound names and chemical structures of Preparation Examples 3 to 15 are summarized in Table 1.

**[Table 1]**

| Preparat ion Example | Structure | Compound Name |
|---|---|---|
| 3 | 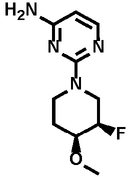 | 2-((3R,4S)-3-fluoro-4-methoxypiperidin-1-yl)pyrimidin-4-amine |
| 4 | 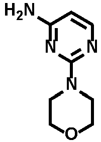 | 2-morpholinopyrimidin-4-amine |
| 5 | 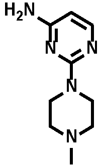 | 2-(4-methylpiperazin-1-yl)pyrimidin-4-amine |
| 6 | 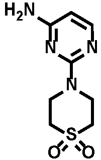 | 4-(4-aminopyrimidin-2-yl)thiomorpholine 1,1-dioxide |
| 7 | 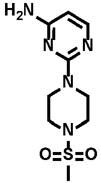 | 2-(4-(methylsulfonyl)piperazin-1-yl)pyrimidin-4-amine |
| 8 | 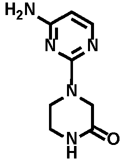 | 4-(4-aminopyrimidin-2-yl)piperazin-2-one |
| 9 | 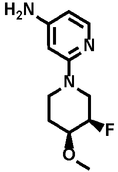 | 2-((3R,4S)-3-fluoro-4-methoxypiperidin-1-yl)pyridin-4-amine |
| 10 | 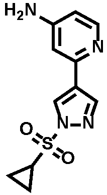 | 2-(1-(cyclopropylsulfonyl)-lH-pyrazol-4-yl)pyridin-4-amine |
| 11 | 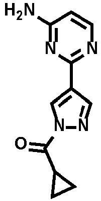 | (4-(4-aminopyrimidin-2-yl)-1H-pyrazol-1-yl) (cyclopropyl)methanone |
| 12 | 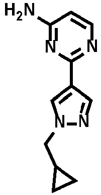 | 2-(1-(cyclopropylmethyl)-1H-pyrazol-4-yl)pyrimidin-4-amine |
| 13 | 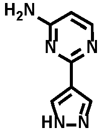 | 2-(1H-pyrazol-4-yl)pyrimidin-4-amine |
| 14 | 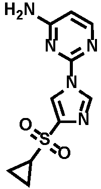 | 2-(4-(cyclopropylsulfonyl)-1H-imidazol-1-yl)pyrimidin-4-amine |
| 15 | 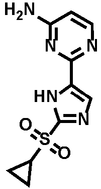 | 2-(2-(cyclopropylsulfonyl)-1H-imidazol-5-yl)pyrimidin-4-amine |

### <Preparation Example 16> Preparation of 4-bromo-2-methyl-1-((2-(trimethylsilyl)ethoxy)methyl)-1,2-dihydro-3H-pyrazol-3-one

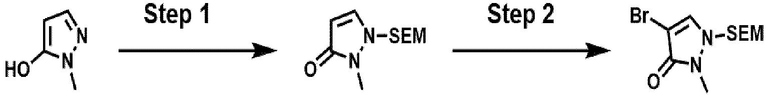

### Step 1: 2-Methyl-1-((2-(trimethylsilyl)ethoxy)methyl)-1,2-dihydro-3H-pyrazol-3-one

1-Methyl-1H-pyrazol-5-ol (6 g, 1 eq.) was dissolved in acetonitrile (ACN; 204 mL), and potassium carbonate (25.4 g, 3 eq.) was added. The reaction mixture was stirred at room temperature for 30 minutes, then 2-(trimethylsilyl)ethoxymethyl chloride (14.1 mL, 1.3 eq.) was slowly added and stirred at room temperature for 3 hours. LCMS analysis results showed that all starting materials disappeared and the target compound was detected. The reaction mixture was passed through diatomaceous earth for filtration. The filtrate was concentrated under reduced pressure, hexane was poured, and the precipitated solid was filtered and dried to obtain the target compound (14 g, 100% yield).
MS: m/z 229.3 [M+H]⁺

### Step 2: Preparation of 4-bromo-2-methyl-1-((2-(trimethylsilyl)ethoxy)methyl)-1,2-dihydro-3H-pyrazol-3-one

The 2-methyl-1-((2-(trimethylsilyl)ethoxy)methyl)-1,2-dihydro-3H-pyrazol-3-one (5 g, 1 eq.) obtained in Step 1 above was dissolved in acetonitrile (ACN; 110 mL), and then N-bromosuccinimide (NBS; 5.07 g, 1.3 eq.) was slowly added at 0°C. The reaction mixture was stirred at 15°C for 1 hour. LCMS analysis results showed that all starting materials disappeared and the target compound was detected. To the reaction mixture, saturated aqueous sodium thiosulfate solution (100 mL) was added to terminate the reaction. The organic layer was extracted using ethyl acetate (EA; 200 mL) and washed several times with a saturated aqueous sodium chloride solution (100 ml * 2). Next, the organic layer was dried with magnesium sulfate and concentrated under reduced pressure. The concentrated compound was purified by chromatography (ethyl acetate/hexane) to obtain the target compound (6 g, 89% yield) .

MS: m/z 307.3 [M+H]⁺

### <Preparation Example 17> Preparation of 4-(4-aminopyrimidin-2-yl)-2-methyl-1-((2-(trimethylsilyl)ethoxy)methyl)-1,2-dihydro-3H-pyrazol-3-one

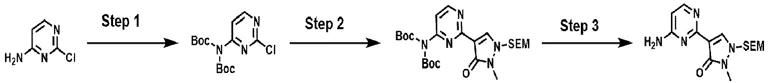

### Step 1: Preparation of tert-butyl (tert-butoxycarbonyl) (2-chloropyrimidin-4-yl)carbamate

2-Chloropyrimidin-4-amine (10 g, 1 eq.) was dissolved in tetrahydrofuran (THF; 77 mL), and then di-tert-butyl dicarbonate (44.8 mL, 2.5 eq.), triethylamine (TEA; 32.3 mL, 3 eq.), and 4-(dimethylamino)pyridine (0.943 g, 0.1 eq.) were sequentially added at 0°C. The reaction mixture was stirred at 50°C for 1 hour. LCMS analysis results showed that all starting materials disappeared and the target compound was detected. The organic layer was extracted using ethyl acetate (EA; 200 mL) and a saturated aqueous ammonium chloride solution (100 mL), and the organic layer was washed several times with a saturated aqueous sodium chloride solution (100 mL * 2). The organic layer was dried over magnesium sulfate and concentrated under reduced pressure. The concentrated compound was purified by MPLC (hexane/ethyl acetate) to obtain the target compound (22.5 g, 88% yield).
MS: m/z 330.3 [M+H]⁺

### Step 2: Preparation of tert-butyl (tert-butoxycarbonyl) (2-(2-methyl-3-oxo-1-((2-(trimethylsilyl)ethoxy)methyl)-2,3-dihydro-1H-pyrazol-4-yl)pyrimidin-4-yl)carbamate

The tert-butyl (tert-butoxycarbonyl) (2-chloropyrimidin-4-yl)carbamate (12.88 g, 6 eq.) obtained in Step 1 above, the 4-bromo-2-methyl-1-((2-(trimethylsilyl)ethoxy)methyl)-1,2-dihydro-3H-pyrazol-3-one (2 g, 1 eq.) obtained in <Preparation Example 16> above, bis(pinacolato)diboron (9.92 g, 6 eq.), and sodium carbonate (3.45 g, 5 eq.) were dissolved in acetonitrile (ACN; 100 mL) and water (10 mL), followed by degassing for 10 minutes. Bis(di-tert-butyl(4-dimethylaminophenyl)phosphine)dichloropalladium(II) (Pd(amphos)Cl₂; 0.69 g, 0.15 eq.) was added to the reaction mixture, and the reaction mixture was stirred at 100°C for 2 hours under a nitrogen atmosphere. LCMS analysis results showed that all starting materials disappeared and the target compound was detected. The reaction mixture was passed through diatomaceous earth for filtration. The filtrate was evaporated to dryness under reduced pressure, and the concentrated compound was purified by MPLC (methanol/dichloromethane) to obtain the target compound (2.5 g, 75% purity, 55% yield).
MS: m/z 522.3 [M+H]⁺

### Step 3: Preparation of 4-(4-aminopyrimidin-2-yl)-2-methyl-1-((2-(trimethylsilyl)ethoxy)methyl)-1,2-dihydro-3H-pyrazol-3-one

The tert-butyl (tert-butoxycarbonyl) (2-(2-methyl-3-oxo-1-((2-(trimethylsilyl)ethoxy)methyl)-2,3-dihydro-1H-pyrazol-4-yl)pyrimidin-4-yl)carbamate (3 g, 5.75 mmol) obtained in Step 2 above was dissolved in 1,1,1,3,3,3-hexafluoro-2-propanol (HFIP; 30 mL), and trifluoroacetic acid (TFA; 6.65 mL, 15 eq) was then added and stirred at 15°C for 3 hours. LCMS analysis results showed that all starting materials disappeared and the target compound was detected. Saturated aqueous sodium bicarbonate solution (100 mL) was slowly added to the reaction mixture, and then extracted using ethyl acetate (EA; 200 mL). The organic layer was washed several times with a saturated sodium chloride solution (100 mL * 2). Next, the organic layer was dried with magnesium sulfate and concentrated under reduced pressure. The concentrated compound was purified by MPLC (methanol/dichloromethane) to obtain the target compound (1.1 g, 59.5% yield).

MS: m/z 322.3 [M+H]⁺

### <Preparation Example 18> Preparation of 4-(4-aminopyrimidin-2-yl)-2-methyl-1-((2-(trimethylsilyl)ethoxy)methyl)-1,2-dihydro-3H-pyrazol-3-one

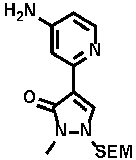

A compound of Preparation Example 18 was prepared in a similar manner to Preparation Example 17 above.
MS: m/z 321.3 [M+H]⁺

### Example: Preparation of compounds of the present invention

### <Example 1> Preparation of (6-((2-(1-(cyclopropylsulfonyl)-1H-pyrazol-4-yl)pyrimidin-4-yl)amino)-4-(isopropylamino)pyridin-3-yl)(3-((methylsulfonyl)methyl)azetidin-1-yl)methanone

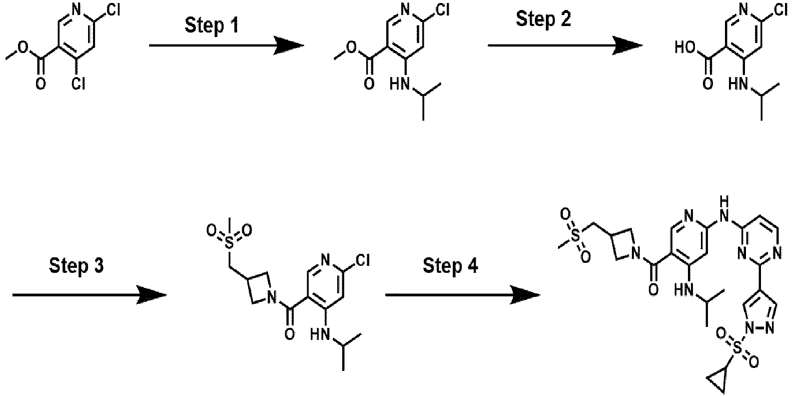

### Step 1: Preparation of methyl 6-chloro-4-(isopropylamino) nicotinate

Methyl 4,6-dichloronicotinate (6 g, 1 eq.) was dissolved in acetonitrile (ACN; 58 mL), and then isopropylamine (2.74 mL, 1.1 eq.) and diisopropylethylamine (DIPEA; 10.17 mL, 2 eq.) were added. The reaction mixture was stirred at 80°C for 16 hours. The reaction mixture was concentrated under reduced pressure, water (100 mL) was added, and then the reaction mixture was extracted with ethyl acetate (EA; 200 mL). The organic layer was washed several times with a saturated sodium chloride solution (100 mL * 2). Next, the organic layer was dried with magnesium sulfate and concentrated under reduced pressure. The concentrated compound was purified by chromatography (ethyl acetate/hexane) to obtain the target compound (6.4 g, 96% yield) .

### Step 2: Preparation of 6-chloro-4-(isopropylamino)nicotinic acid

The methyl 6-chloro-4-(isopropylamino)nicotinate (6.4 g, 1 eq.) obtained in Step 1 was dissolved in tetrahydrofuran (THF; 50 ml), methanol (50 ml), and water (50 ml), and then aqueous sodium hydroxide solution (6 M; 23.32 mL, 5 eq.) was added. The reaction mixture was stirred at 80°C for 2 hours. LCMS analysis results showed that all starting materials disappeared and the target compound was detected. The reaction mixture was titrated with 6N hydrochloric acid solution until the pH reached 4. The precipitated solid was filtered and washed with water. The obtained solid was dried in a reduced pressure oven to obtain the target compound (5.3 g, 88% yield).

### Step 3: Preparation of (6-chloro-4-(isopropylamino) pyridin-3-yl)(3-((methylsulfonyl)methyl)azetidin-1-yl)methanone

The 6-chloro-4-(isopropylamino) nicotinic acid (600 mg, 1 eq.) obtained in Step 2 above was dissolved in dichloromethane (DCM; 5 mL), and then 3-((methylsulfonyl)methyl)azetidine (809 mg, 1.1 eq.), 1-[bis(dimethylamino)methylene]-1H-1,2,3-triazol[4,5-b]pyridinium 3-oxide hexafluorophosphate (HATU; 2.1 g, 2 eq.), and diisopropylethylamine (DIPEA; 2 mL, 5 eq.) were sequentially added. The reaction mixture was stirred at room temperature for 6 hours. LCMS analysis results showed that all starting materials disappeared and the target compound was detected. The reaction mixture was concentrated under reduced pressure, saturated aqueous sodium bicarbonate solution (10 mL) was added, and then the reaction mixture was extracted with ethyl acetate (EA; 10 mL). The organic layer was washed with saturated aqueous sodium chloride solution (10 mL * 2). Next, the organic layer was dried with magnesium sulfate and concentrated under reduced pressure. The concentrated compound was purified by chromatography (ethyl acetate/hexane) to obtain the target compound (500 mg, 52% yield).

### Step 4: Preparation of (6-((2-(1-(cyclopropylsulfonyl)-1H-pyrazol-4-yl)pyrimidin-4-yl)amino)-4-(isopropylamino)pyridin-3-yl)(3-((methylsulfonyl)methyl)azetidin-1-yl)methanone

The (6-chloro-4-(isopropylamino)pyridin-3-yl)(3-((methylsulfonyl)methyl)azetidin-1-yl)methanone (70 mg, 1 eq.) obtained in Step 3 was dissolved in 1,4-dioxane (2 mL), and the 2-(1-(cyclopropylsulfonyl)-1H-pyrazol-4-yl)pyrimidin-4-amine (64 mg, 1.2 eq.) obtained in Preparation Example 2 above and cesium carbonate (198 mg, 3 eq.) were then added. The reaction mixture was degassed for 5 minutes, and Brettphos Pd(II) G3 (20 mg, 0.1 eq.) was added. Then, the reaction mixture was stirred at 120°C for 12 hours. LCMS analysis results showed that all starting materials disappeared and the target compound was detected. The reaction mixture was passed through diatomaceous earth for filtration. The filtrate was concentrated under reduced pressure and purified by Prep-HPLC to obtain the target compound (30 mg, 26% yield).

¹H NMR (400 MHz, Chloroform-d) δ 9.81 (s, 1H), 8.62 (d, J = 0.6 Hz, 1H), 8.44 (d, J = 0.6 Hz, 1H), 8.41 (d, J = 5.8 Hz, 1H), 8.22 (d, J = 7.2 Hz, 1H), 7.91 (s, 1H), 7.71 (s, 1H), 6.96 (d, *J* = 5.8 Hz, 1H), 4.55 (t, *J* = 8.9 Hz, 2H), 4.18 (s, 2H), 3.85 (h, *J* = 6.4 Hz, 1H), 3.40 (d, *J* = 7.1 Hz, 2H), 3.34 (qd, *J* = 8.1, 5.6 Hz, 1H), 2.96 (s, 3H), 2.83 (tt, *J* = 8.0, 4.7 Hz, 1H), 1.57-1.49 (m, 2H), 1.38 (d, *J* = 6.4 Hz, 6H), 1.26-1.20 (m, 2H); MS m/z: 575.31 [M+H]⁺.

### <Example 80> Preparation of (S)-N-(cyanomethyl)-1¹,6-dimethyl-1¹H-9-oxa-3,5-diaza-2(2,4)-pyrimidina-4(2,4)-pyridina-1(4,5)-pyrazolacyclononaphane-4⁵-carboxamide

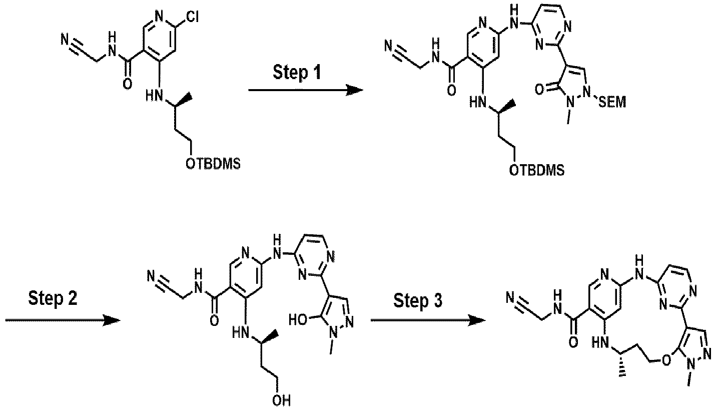

### Step 1: Preparation of (S)-4-((4-((tertbutyldimethylsilyl)oxy)butan-2-yl)amino)-N-(cyanomethyl)-6-((2-(2-methyl-3-oxo-1-((2-(trimethylsilyl)ethoxy)methyl)-2,3-dihydro-1H-pyrazol-4-yl)pyrimidin-4-yl)amino)nicotinamide

The (S)-4-((4-((tert-butyldimethylsilyl)oxy)butan-2-yl)amino)-6-chloro-N-(cyanomethyl)nicotinamide (0.37 g, 1 eq.) obtained in a similar manner to Example 1 was dissolved in 1,4-dioxane (4.67 mL), and then the 4-(4-aminopyrimidin-2-yl)-2-methyl-1-((2-(trimethylsilyl)ethoxy)methyl)-1,2-dihydro-3H-pyrazol-3-one (0.3 g, 1 eq.) obtained in Preparation Example 17 above and cesium carbonate (0.912 g, 3 eq.) were added. The reaction mixture was degassed for 10 minutes, and Brettphos Pd(II) G3 (0.28 g, 0.3 eq.) was added. Next, the reaction mixture was stirred at 120°C for 3 hours under a nitrogen atmosphere. LCMS analysis results showed that all starting materials disappeared and the target compound was detected. The reaction mixture was passed through diatomaceous earth for filtration. The filtrate was concentrated under reduced pressure and purified by chromatography (methanol/dichloromethane) to obtain the target compound (0.17 g, 27% yield).

### Step 2: Preparation of (S)-N-(cyanomethyl)-6-((2-(5-hydroxy-1-methyl-1H-pyrazol-4-yl)pyrimidin-4-yl)amino)-4-((4-hydroxybutan-2-yl)amino)nicotinamide

The (S)-4-((4-((tert-butyldimethylsilyl)oxy)butan-2-yl)amino)-N-(cyanomethyl)-6-((2-(2-methyl-3-oxo-1-((2-(trimethylsilyl)ethoxy)methyl)-2,3-dihydro-1H-pyrazol-4-yl)pyrimidin-4-yl)amino)nicotinamide (0.16 g, 1 eq.) obtained in Step 1 was dissolved in tetrahydrofuran (THF; 5 mL), and then tetrabutylammonium fluoride (1 M, 0.469 mL, 2 eq.) was added at 0°C. Then, the reaction mixture was stirred at 80°C for 3 hours. LCMS analysis results showed that all starting materials disappeared and the target compound was detected. The reaction mixture was concentrated under reduced pressure and purified by reverse phase chromatography (0.035% trifluoroacetic acid in methanol/water) to obtain the target compound (0.06 g, 58% yield) .

### Step 3: Preparation of (S)-N-(cyanomethyl)-1¹,6-dimethyl-1¹H-9-oxa-3,5-diaza-2(2,4)-pyrimidina-4(2,4)-pyridina-1(4,5)-pyrazolacyclononaphane-4⁵-carboxamide

The (S)-N-(cyanomethyl)-6-((2-(5-hydroxy-1-methyl-1H-pyrazol-4-yl)pyrimidin-4-yl)amino)-4-((4-hydroxybutan-2-yl)amino)nicotinamide (2 mg, 1 eq.) obtained in Step 2 was dissolved in tetrahydrofuran (THF; 0.5 mL), and then cyanomethylenetributylphosphorane (CMBP; 0.05 mL, 4 eq.) was added. The reaction mixture was degassed for 10 minutes and stirred at 60°C for 2 hours under a nitrogen atmosphere. LCMS analysis results showed that all starting materials disappeared and the target compound was detected. The reaction mixture was concentrated under reduced pressure and purified by reverse phase chromatography (0.035% trifluoroacetic acid in methanol/water) to obtain the target compound (5 mg, 26% yield).

¹H NMR (400 MHz, Chloroform-d) δ 8.89 (s, 1H), 8.45 (d, *J* = 7.3 Hz, 1H), 8.36 (d, *J* = 5.7 Hz, 1H), 8.35 (s, 1H), 8.13 (s, 1H), 6.51 (d, *J* = 5.8 Hz, 1H), 6.43-6.37 (m, 1H), 4.68 (td, *J* = 10.0, 3.2 Hz, 1H), 4.31 (dd, *J* = 9.2, 5.7 Hz, 2H), 4.28-4.23 (m, 1H), 4.09 (dt, *J* = 9.2, 4.4 Hz, 1H), 3.80 (s, 3H), 2.20-2.13 (m, 2H), 1.47 (d, *J* = 6.6 Hz, 3H); MS m/z: 420.17 [M+H]⁺.

### <Example 2> to <Example 79>, and <Example 81> to <Example 134>

All compounds according to Examples of the present invention (Examples 1 to 134 compounds) were prepared in a similar manner to Example 1 or 80 above, and the compound names, chemical structural formulas, NMR, and LCMS analysis results of respective compounds according to Examples above are summarized and shown in Table 2 below.

**[Table 2]**

| Exa mpl e com pou nd | Structure | Compound name | ¹H NMR; MS [M+H]⁺ |
|---|---|---|---|
| 1 | 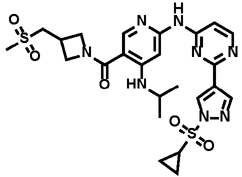 | (6-((2-(1-(cyclopropylsulfonyl )-1H-pyrazol-4-yl)pyrimidin-4-yl)amino)-4-(isopropylamino)pyri din-3-yl) (3-((methylsulfonyl)met hyl)azetidin-1-yl)methanone | ¹H NMR (400 MHz, Chloroform-*d*) δ 9.81 (s, 1H), 8.62 (d, *J* = 0.6 Hz, 1H), 8.44 (d, *J* = 0.6 Hz, 1H), 8.41 (d, *J* = 5.8 Hz, 1H), 8.22 (d, *J* = 7.2 Hz, 1H), 7.91 (s, 1H), 7.71 (s, 1H), 6.96 (d, *J* = 5.8 Hz, 1H), 4.55 (t, *J* = 8.9 Hz, 2H), 4.18 (s, 2H), 3.85 (h, *J* = 6.4 Hz, 1H), 3.40 (d, *J* = 7.1 Hz, 2H), 3.34 (qd, *J* = 8.1, 5.6 Hz, 1H), 2.96 (s, 3H), 2.83 (tt, *J* = 8.0, 4.7 Hz, 1H), 1.57-1.49 (m, 2H), 1.38 (d, *J* = 6.4 Hz, 6H), 1.26-1.20 (m, 2H); 575.31 [M+H]⁺ |
| 2 | 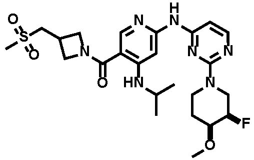 | (6-((2-(cis-3-fluoro-4-methoxypiperidin-1-yl)pyrimidin-4-yl)amino)-4-(isopropylamino)pyri din-3-yl) (3-((methylsulfonyl)met hyl)azetidin-1-yl)methanone | ¹H NMR (400 MHz, DMSO) δ 9.66 (s, 1H), 8.05 (s, 1H), 8.00 (d, *J* = 5.6 Hz, 1H), 7.94 (d, *J* = 7.5 Hz, 1H), 7.33 (s, 1H), 6.62 (d, *J* = 5.6 Hz, 1H), 4.91 (d, *J* = 49.1 Hz, 1H), 4.69-4.53 (m, 1H), 4.37 (d, *J* = 13.0 Hz, 2H), 3.73-3.36 (m, 7H), 3.35 (s, 3H), 3.17 (ddd, *J* = 21.7, 19.0, 9.7 Hz, 3H), 2.97 (s, 3H), 1.75 (ddd, *J* = 33.8, 14.5, 7.3 Hz, 2H), 1.21 (dt, *J* = 8.0, 4.0 Hz, 6H); 536.3 [M+H]⁺ |
| 3 | 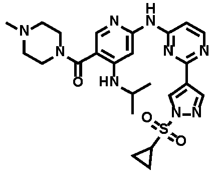 | (6-((2-(1-(cyclopropylsulfonyl )-1H-pyrazol-4-yl)pyrimidin-4-yl)amino)-4-(isopropylamino)pyri din-3-yl) (4-methylpiperazin-1-yl)methanone | ¹H NMR (400 MHz, Chloroform-*d*) δ 8.64 (s, 1H), 8.46-8.36 (m, 2H), 7.92 (s, 1H), 7.70 (s, 1H), 7.24-7.01 (m, 2H), 6.19 (d, *J* = 7.2 Hz, 1H), 3.77 (dq, *J* = 12.9, 6.4 Hz, 1H), 3.71-3.62 (m, 4H), 2.83 (dq, *J* = 8.0, 4.7 Hz, 1H), 2.51-2.42 (m, 4H), 2.34 (s, 3H), 1.56-1.48 (m, 2H), 1.34 (d, *J* = 6.3 Hz, 6H), 1.23-1.19 (m, 2H); 526.3 [M+H]⁺ |
| 4 | 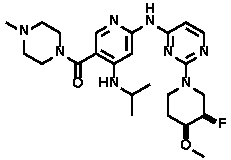 | (6-((2-(cis-3-fluoro-4-methoxypiperidin-1-yl)pyrimidin-4-yl)amino)-4-(isopropylamino)pyri din-3-yl) (4-methylpiperazin-1-yl)methanone | ¹H NMR (400 MHz, Chloroform-*d*) δ 8.07 (d, *J* = 5.6 Hz, 1H), 7.86 (s, 1H), 7.33 (s, 1H), 6.24 (dd, *J* = 16.6, 6.5 Hz, 2H), 4.87-4.65 (m, 1H), 4.51-4.36 (m, 1H), 4.11 (t, *J* = 7.2 Hz, 1H), 3.87-3.55 (m, 8H), 3.50 (s, 3H), 2.52-2.40 (m, 4H), 2.33 (s, 3H), 2.03-1.94 (m, 1H), 1.83-1.75 (m, 1H), 1.30-1.27 (m, 6H); 487.3 [M+H]⁺ |
| 5 | 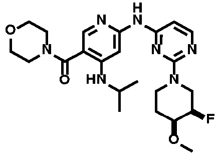 | (6-((2-(cis-3-fluoro-4-methoxypiperidin-1-yl)pyrimidin-4-yl)amino)-4-(isopropylamino)pyri din-3-yl) (morpholino)metha none | ¹H NMR (400 MHz, Chloroform-*d*) δ 8.06 (d, *J* = 5.6 Hz, 1H), 7.87 (s, 1H), 7.31 (d, *J* = 6.0 Hz, 2H), 6.29-6.19 (m, 2H), 4.88-4.68 (m, 1H), 4.49-4.37 (m, 1H), 4.15 (dd, *J* = 10.1, 4.8 Hz, 1H), 3.85-3.57 (m, 12H), 3.49 (d, *J* = 9.9 Hz, 3H), 2.00 (ddd, *J* = 12.1, 8.3, 4.5 Hz, 1H), 1.79 (ddt, *J* = 18.1, 14.6, 7.2 Hz, 1H), 1.28 (dd, *J* = 6.3, 1.4 Hz, 6H); 474.3 [M+H]⁺ |
| 6 | 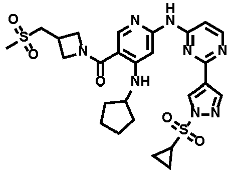 | (4-(cyclopentylamino)-6-((2-(1-(cyclopropylsulfonyl )-1H-pyrazol-4-yl)pyrimidin-4-yl)amino)pyridin-3-yl) (3-((methylsulfonyl)methyl)azetidin-1-yl)methanone | ¹H NMR (400 MHz, Chloroform-*d*) δ 8.65 (s, 1H), 8.44 (t, *J* = 2.9 Hz, 2H), 8.13 (d, *J* = 6.3 Hz, 1H), 8.06 (s, 1H), 7.48 (s, 1H), 6.99 (s, 1H), 4.59-4.49 (m, 2H), 4.18 (s, 2H), 3.96-3.86 (m, 1H), 3.40 (d, *J* = 7.3 Hz, 2H), 3.37-3.29 (m, 1H), 2.95 (s, 3H), 2.85-2.78 (m, 1H), 2.15-2.03 (m, 2H), 1.80-1.62 (m, 6H), 1.53-1.50 (m, 2H), 1.24-1.18 (m, 2H); 601.30 [M+H]⁺ |
| 7 | 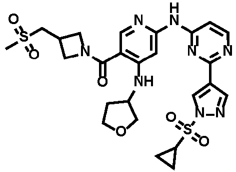 | (6-((2-(1-(cyclopropylsulfonyl )-1H-pyrazol-4-yl)pyrimidin-4-yl)amino)-4-((tetrahydrofuran-3-yl)amino)pyridin-3-yl) (3-((methylsulfonyl)met hyl)azetidin-1-yl)methanone | ¹H NMR (400 MHz, DMSO-*d*6) δ 10.14 (s, 1H), 8.66 (s, 1H), 8.47 (d, *J* = 5.9 Hz, 1H), 8.44 (s, 1H), 8.14 (d, *J* = 4.0 Hz, 2H), 7.58 (d, *J* = 5.2 Hz, 1H), 7.22 (s, 1H), 4.16 (s, 2H), 3.90 (dd, *J* = 9.2, 5.3 Hz, 1H), 3.84 (t, *J* = 7.6 Hz, 1H), 3.76 (td, *J* = 8.5, 5.4 Hz, 1H), 3.63 (dd, *J* = 9.1, 2.8 Hz, 1H), 3.56 (d, *J* = 7.4 Hz, 2H), 3.28-3.23 (m, 1H), 3.19-3.11 (m, 1H), 2.97 (s, 3H), 2.36-2.26 (m, 2H), 1.87-1.79 (m, 1H), 1.37-1.22 (m, 6H); 603.28 [M+H]⁺ |
| 8 | 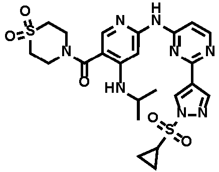 | (6-((2-(1-(cyclopropylsulfonyl )-1H-pyrazol-4-yl)pyrimidin-4-yl)amino)-4-(isopropylamino)pyri din-3-yl) (1,1-dioxydothiomorpholin o)methanone | ¹H NMR (400 MHz, Chloroform-*d*) δ 8.64 (d, *J* = 0.6 Hz, 1H), 8.46-8.43 (m, 2H), 7.96 (s, 1H), 7.46 (s, 1H), 7.12 (d, *J* = 5.9 Hz, 1H), 6.34 (d, *J* = 7.0 Hz, 1H), 4.15 (t, 4H), 3.83-3.73 (m, 1H), 3.12 (t, *J* = 5.4 Hz, 4H), 2.83 (tt, *J* = 8.0, 4.7 Hz, 1H), 1.56-1.51 (m, 3H), 1.35 (d, *J* = 6.3 Hz, 6H), 1.24-1.21 (m, 2H); 561.19 [M+H]⁺ |
| 9 | 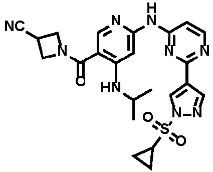 | 1-(6-((2-(1-(cyclopropylsulfonyl )-1H-pyrazol-4-yl)pyrimidin-4-yl)amino)-4-(isopropylamino)nico tinoyl)azetidine-3-carbonitrile | ¹H NMR (400 MHz, Chloroform-*d*) δ 8.64 (d, *J* = 0.6 Hz, 1H), 8.44 (d, *J* = 4.8 Hz, 2H), 8.02 (s, 1H), 7.92 (d, *J* = 7.2 Hz, 1H), 7.39 (s, 1H), 7.21 (s, 1H), 7.11 (d, *J* = 5.9 Hz, 1H), 4.60-4.47 (m, 4H), 3.79 (h, *J* = 6.5 Hz, 1H), 3.58 (tt, *J* = 9.1, 6.3 Hz, 1H), 2.87-2.78 (m, 1H), 1.54-1.52 (m, 2H), 1.36 (d, *J* = 6.4 Hz, 6H), 1.24-1.21 (m, 2H); 508.20 [M+H]⁺ |
| 10 | 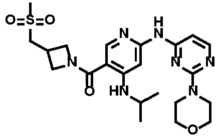 | (4-(isopropylamino)-6-((2-morpholinopyrimidin-4-yl)amino)pyridin-3-yl) (3-((methylsulfonyl)met hyl)azetidin-1-yl)methanone | ¹H NMR (400 MHz, DMSO-*d*6) δ 9.68 (s, 1H), 8.05 (s, 1H), 8.04 (d, *J* = 5.6 Hz, 1H), 7.93 (d, *J* = 7.5 Hz, 1H), 7.35 (s, 1H), 6.65 (d, *J* = 5.7 Hz, 1H), 4.32 (s, 2H, br), 4.07 (s, 2H, br), 3.72-3.65 (m, 8H), 3.64-3.57 (m, 1H), 3.55 (d, *J* = 7.5 Hz, 2H), 3.19-3.08 (m, 1H), 2.97 (s, 3H), 1.20 (d, *J* = 6.3 Hz, 6H); 490.20 [M+H]⁺ |
| 11 | 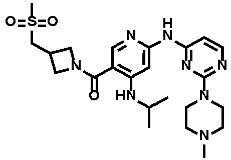 | (4-(isopyropylamino)-4-((2-(4-methylpiperazin-1-yl)pyrimidin-4-yl)amino)pyridin-3-yl) (3-((methylsulfonyl)met hyl)azetidin-1-yl)methanone | ¹H NMR (400 MHz, Chloroform-*d*) δ 8.07 (d, *J* = 5.6 Hz, 1H), 8.03-7.98 (m, 2H), 7.93 (d, 1H), 7.36 (s, 1H), 7.29 (s, 1H), 4.54 (s, 2H), 4.16 (s, 2H), 3.86 (t, *J* = 5.1 Hz, 4H), 3.75-3.66 (m, 1H), 3.39 (d, *J* = 7.3 Hz, 2H), 3.37-3.26 (m, 1H), 2.95 (s, 3H), 2.48 (t, *J* = 5.1 Hz, 4H), 2.35 (s, 3H), 1.28 (d, *J* = 6.4 Hz, 6H) ; 503.26[M+H]⁺ |
| 12 | 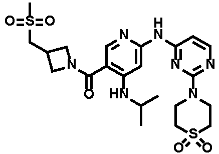 | (6-((2-(1,1-dioxydothiomorpholin o)pyrimidin-4-yl)amino)-4-(isopropylamino)pyri din-3-yl) (3-((methylsulfonyl)met hyl)azetidin-1-yl)methanone | ¹H NMR (400 MHz, Chloroform-*d*) δ 8.12 (d, *J* = 5.7 Hz, 1H), 8.04 (s, 1H), 8.02 (d, *J* = 7.5 Hz, 1H), 7.24 (s, 1H), 6.83 (s, 1H), 6.66 (d, *J* = 5.7 Hz, 1H), 4.57-4.51 (m, 2H), 4.40-4.35 (m, 4H), 4.17 (s, 2H), 3.66-3.56 (m, 1H), 3.39 (d, *J* = 7.3 Hz, 2H), 3.34-3.32 (m, 1H), 3.05 (t, *J* = 5.4 Hz, 4H), 2.95 (s, 3H), 1.29 (d, *J* = 6.4 Hz, 6H); 538.20 [M+H]⁺ |
| 13 | 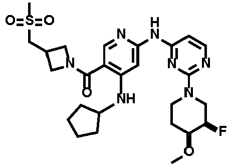 | (4-(cyclopentylamino)-6-((2-(cis-3-fluoro-4-methoxypiperidin-1-yl)pyrimidin-4-yl)amino)pyridin-3-yl) (3-((methylsulfonyl)met hyl)azetidin-1-yl)methanone | ¹H NMR (400 MHz, Chloroform-*d*) δ 8.21 (s, 1H), 8.19 (s, 1H), 8.07 (d, *J* = 5.6 Hz, 1H), 7.94 (s, 1H), 7.31 (s, 1H), 6.31 (d, *J* = 5.6 Hz, 1H), 4.85-4.68 (m, 1H), 4.53 (t, *J* = 8.9 Hz, 2H), 4.49-4.39 (m, 1H), 4.20-4.10 (m, 3H), 3.94-3.88 (m, 1H), 3.81 (ddd, *J* = 23.0, 14.1, 2.9 Hz, 1H), 3.65-3.55 (m, 2H), 3.49 (s, 3H), 3.36-3.26 (m, 1H), 2.95 (s, 3H), 2.04-1.98 (m, 3H), 1.79 (dq, *J* = 10.7, 3.7 Hz, 6H), 1.68-1.62 (m, 3H); 562.39 [M+H]⁺ |
| 14 | 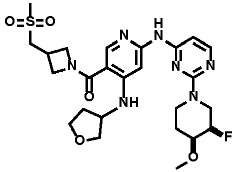 | (6-((2-(cis-3-fluoro-4-methoxypiperidin-1-yl)pyrimidin-4-yl)amino)-4-((tetrahydrofuran-3-yl)amino)pyridin-3-yl) (3-((methylsulfonyl)met hyl)azetidin-1-yl)methanone | 564.37[M+H]⁺ |
| 15 | 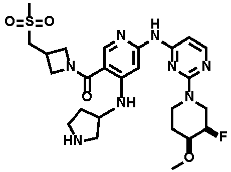 | (6-((2-(cis-3-fluoro-4-methoxypiperidin-1-yl)pyrimidin-4-yl)amino)-4-(pyrolidin-3-ylamino)pyridin-3-yl) (3-((methylsulfonyl)met hyl)azetidin-1-yl)methanone | 563.45[M+H]⁺ |
| 16 | 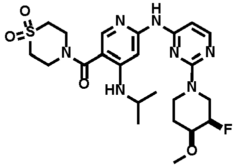 | (1,1-dioxydothiomorpholin o)(6-((2-(cis-3-fluoro-4-methoxypiperidin-1-yl)pyrimidin-4-yl)amino)-4-(isopropylamino)pyri din-3-yl)methanone | ¹H NMR (400 MHz, Chloroform-*d*) δ 8.08 (d, *J* = 5.6 Hz, 1H), 7.91 (s, 1H), 7.36 (s, 1H), 7.13 (s, 1H), 6.32 (d, *J* = 7.5 Hz, 1H), 6.23 (d, *J* = 5.6 Hz, 1H), 4.85-4.69 (m, 1H), 4.44 (dt, *J* = 14.0, 7.0 Hz, 1H), 4.18-4.09 (m, 5H), 3.87-3.70 (m, 2H), 3.66-3.56 (m, 2H), 3.50 (s, 3H), 3.10 (t, *J* = 5.4 Hz, 4H), 2.05-1.95 (m, 1H), 1.84-1.76 (m, 1H), 1.29 (dd, *J* = 6.4, 1.7 Hz, 6H); 522.32 [M+H]⁺ |
| 17 | 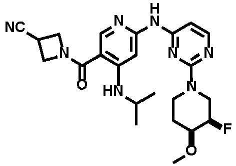 | 1-(6-((2-(cis-3-fluoro-4-methoxypiperidin-1-yl)pyrimidin-4-yl)amino)-4-(isopropylamino)nico tinoyl)azetidine-3-carbonitrile | ¹H NMR (400 MHz, Chloroform-*d*) δ 8.37 (s, 1H), 8.07 (d, *J* = 5.6 Hz, 1H), 7.98 (d, *J* = 7.6 Hz, 1H), 7.90 (s, 1H), 7.48 (s, 1H), 6.23 (d, *J* = 5.6 Hz, 1H), 4.77 (ddt, *J* = 47.8, 5.7, 2.5 Hz, 1H), 4.59-4.45 (m, 4H), 4.41 (dt, *J* = 13.9, 7.1 Hz, 1H), 4.16-4.07 (m, 1H), 3.90-3.72 (m, 2H), 3.67-3.53 (m, 3H), 3.50 (s, 3H), 2.05-1.95 (m, 1H), 1.84-1.74 (m, 1H), 1.30 (dd, *J* = 6.4, 2.3 Hz, 6H); 469.33 [M+H]⁺ |
| 18 | 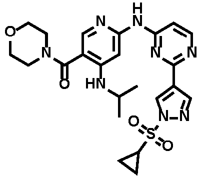 | (6-((2-(1-(cyclopropylsulfonyl )-1H-pyrazol-4-yl)pyrimidin-4-yl)amino)-4-(isopropylamino)pyri din-3-yl) (morpholino)metha none | ¹H NMR (400 MHz, Chloroform-*d*) δ 8.64 (s, 1H), 8.47-8.38 (m, 2H), 7.91 (s, 1H), 7.52 (d, *J* = 3.8 Hz, 1H), 7.21-7.11 (m, 2H), 6.25 (d, *J* = 7.2 Hz, 1H), 3.83-3.63 (m, 9H), 2.83 (tt, *J* = 8.0, 4.7 Hz, 1H), 1.52 (tt, *J* = 6.0, 3.1 Hz, 2H), 1.34 (d, *J* = 6.3 Hz, 6H), 1.24-1.17 (m, 2H); 513.3 [M+H]⁺ |
| 19 | 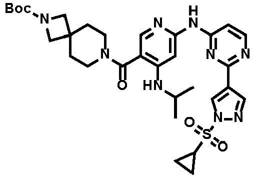 | tert-butyl 7-(6-((2-(1-(cyclopropylsulfonyl )-1H-pyrazol-4-yl)pyrimidin-4-yl)amino)-4-(isopropylamino)nico tinoyl)-2,7-diazaspiro[3.5]nonan e-2-carboxylate | ¹H NMR (400 MHz, Chloroform-*d*) δ 8.64 (s, 1H), 8.49-8.40 (m, 2H), 7.88 (d, *J* = 5.3 Hz, 1H), 7.51 (d, *J* = 9.2 Hz, 1H), 7.22-7.08 (m, 2H), 6.20 (d, *J* = 7.2 Hz, 1H), 3.76 (dd, *J* = 13.1, 6.5 Hz, 1H), 3.70 (s, 4H), 3.60-3.55 (m, 4H), 2.82 (td, *J* = 7.9, 4.0 Hz, 1H), 1.84-1.79 (m, 4H), 1.53 (dd, *J* = 4.7, 2.2 Hz, 2H), 1.45 (s, 9H), 1.32 (d, *J* = 6.3 Hz, 6H), 1.23-1.20 (m, 2H); 652.4 [M+H]⁺ |
| 20 | 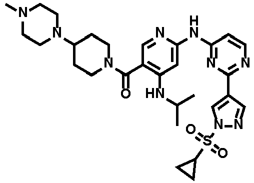 | (6-((2-(1-(cyclopropylsulfonyl )-1H-pyrazol-4-yl)pyrimidin-4-yl)amino)-4-(isopropylamino)pyri din-3-yl) (4-(4-methylpiperazin-1-yl)piperidin-1-yl)methanone | ¹H NMR (400 MHz, Chloroform-*d*) δ 8.64 (s, 1H), 8.49-8.35 (m, 2H), 7.90 (s, 1H), 7.52 (s, 1H), 7.21-7.09 (m, 2H), 6.20 (d, *J* = 7.1 Hz, 1H), 4.34 (d, *J* = 12.5 Hz, 2H), 3.75 (dt, *J* = 12.9, 6.5 Hz, 1H), 2.96 (t, *J* = 12.2 Hz, 2H), 2.90-2.78 (m, 1H), 2.52 (dd, *J* = 39.0, 28.3 Hz, 8H), 2.31 (s, 3H), 1.95 (d, *J* = 12.0 Hz, 2H), 1.54 (ddd, *J* = 9.5, 7.6, 4.4 Hz, 5H), 1.33 (d, *J* = 6.3 Hz, 6H), 1.21 (dt, *J* = 7.0, 3.5 Hz, 2H); 609.4 [M+H]⁺ |
| 21 | 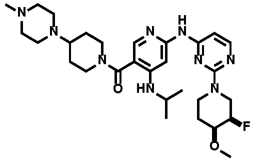 | (6-((2-(cis-3-fluoro-4-methoxypiperidin-1-yl)pyrimidin-4-yl)amino)-4-(isopropylamino)pyri din-3-yl) (4-(4-methylpiperazin-1-yl)piperidin-1-yl)methanone | ¹H NMR (400 MHz, Chloroform-*d*) δ 8.05 (d, *J* = 5.6 Hz, 1H), 7.86 (s, 1H), 7.44 (s, 1H), 7.32 (s, 1H), 6.21 (dd, *J* = 15.1, 6.6 Hz, 2H), 4.86-4.68 (m, 1H), 4.49-4.38 (m, 1H), 4.34 (d, *J* = 12.9 Hz, 2H), 4.19-4.07 (m, 1H), 3.88-3.68 (m, 2H), 3.67-3.54 (m, 2H), 3.53-3.46 (m, 3H), 2.95 (t, *J* = 12.1 Hz, 2H), 2.71-2.34 (m, 8H), 2.29 (s, 3H), 2.04-1.97 (m, 1H), 1.94 (d, *J* = 13.1 Hz, 2H), 1.57-1.43 (m, 2H), 1.34-1.23 (m, 8H); 570.5 [M+H]⁺ |
| 22 | 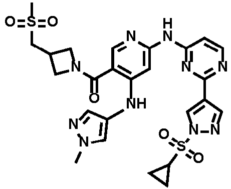 | (6-((2-(1-(cyclopropylsulfonyl )-1H-pyrazol-4-yl)pyrimidin-4-yl)amino)-4-((1-methyl-1H-pyrazol-4-yl)amino)pyridin-3-yl) (3-((methylsulfonyl)met hyl)azetidin-1-yl)methanone | 613.3[M+H]⁺ |
| 23 | 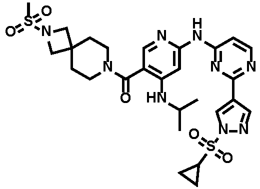 | (6-((2-(1-(cyclopropylsulfonyl )-1H-pyrazol-4-yl)pyrimidin-4-yl)amino)-4-(isopropylamino)pyri din-3-yl) (2-(methylsulfonyl)-2,7-diazaspiro[3.5]nonan -7-yl)methanone | ¹H NMR (400 MHz, Chloroform-*d*) δ 8.68-8.60 (m, 1H), 8.43 (d, *J* = 6.0 Hz, 2H), 7.87 (s, 1H), 7.77 (s, 1H), 7.14 (d, *J* = 5.7 Hz, 1H), 6.30 (d, *J* = 7.0 Hz, 1H), 3.82-3.72 (m, 5H), 3.62-3.56 (m, 4H), 2.88 (d, *J* = 2.0 Hz, 3H), 2.83 (ddd, *J* = 16.0, 8.0, 4.7 Hz, 1H), 1.91-1.85 (m, 4H), 1.53 (dd, *J* = 4.8, 2.1 Hz, 2H), 1.33 (d, *J* = 6.3 Hz, 6H), 1.22 (dd, *J* = 8.1, 1.9 Hz, 2H); 630.3 [M+H]⁺ |
| 24 | 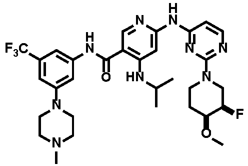 | 6-((2-(cis-3-fluoro-4-methoxypiperidin-1-yl)pyrimidin-4-yl)amino)-4-(isopropylamino)-N-(3-(4-methylpiperazin-1-yl)-5-(trifluoromethyl)phe nyl)nicotinamide | ¹H NMR (400 MHz, Chloroform-*d*) δ 8.30 (s, 1H), 8.09 (t, *J* = 9.3 Hz, 2H), 7.70 (s, 1H), 7.38 (d, *J* = 20.8 Hz, 2H), 7.19 (s, 2H), 6.91 (s, 1H), 6.20 (d, *J* = 5.6 Hz, 1H), 4.78 (d, *J* = 48.5 Hz, 1H), 4.43 (d, *J* = 7.4 Hz, 1H), 4.16 (s, 1H), 3.79 (d, *J* = 12.7 Hz, 2H), 3.61 (d, *J* = 10.6 Hz, 2H), 3.50 (s, 3H), 3.32-3.28 (m, 4H), 2.61-2.57 (m, 4H), 2.37 (s, 3H), 2.06-1.94 (m, 2H), 1.31 (d, *J* = 4.8 Hz, 6H); 646.3 [M+H]⁺ |
| 25 | 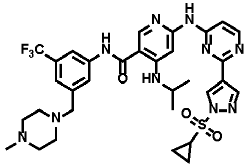 | 6-((2-(1-(cyclopropylsulfonyl )-1H-pyrazol-4-yl)pyrimidin-4-yl)amino)-4-(isopropylamino) -N-(3-((4-methylpiperazin-1-yl)methyl)-5-(trifluoromethyl)phe nyl)nicotinamide | ¹H NMR (400 MHz, Chloroform-*d*) δ 8.67 (s, 1H), 8.47 (s, 1H), 8.40 (d, *J* = 3.6 Hz, 2H), 7.97 (s, 1H), 7.70 (s, 1H), 7.59 (s, 1H), 7.34 (s, 4H), 7.02 (d, *J* = 5.6 Hz, 1H), 3.94-3.77 (m, 1H), 3.61 (s, 2H), 3.38 (d, *J* = 1.6 Hz, 8H), 2.91-2.84 (m, 1H), 2.46 (s, 3H), 1.52 (s, 2H), 1.39 (d, *J* = 6.3 Hz, 6H), 1.30 (s, 2H); 699.4 [M+H]⁺ |
| 26 | 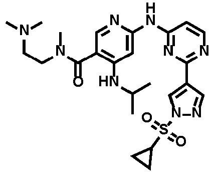 | 6-((2-(1-(cyclopropylsulfonyl )-1H-pyrazol-4-yl)pyrimidin-4-yl)amino)-N-(2-(dimethylamino)ethyl )-4-(isopropylamino)-N-methylnicotinamide | ¹H NMR (400 MHz, Chloroform-*d*) δ 8.63 (s, 1H), 8.52 (s, 1H), 8.44 (s, 1H), 8.40 (d, *J* = 5.8 Hz, 1H), 7.90 (s, 1H), 7.41 (s, 1H), 7.06 (d, *J* = 5.8 Hz, 1H), 6.03 (d, *J* = 7.4 Hz, 1H), 3.88-3.78 (m, 1H), 3.64 (t, *J* = 6.3 Hz, 2H), 3.06 (s, 3H), 2.83 (tt, *J* = 8.0, 4.7 Hz, 1H), 2.54 (t, *J* = 6.4 Hz, 2H), 2.25 (s, 6H), 1.56-1.50 (m, 2H), 1.34 (d, *J* = 6.4 Hz, 6H), 1.25-1.19 (m, 2H); 528.23 [M+H]⁺ |
| 27 | 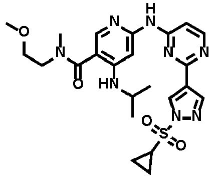 | 6-((2-(1-(cyclopropylsulfonyl )-1H-pyrazol-4-yl)pyrimidin-4-yl)amino)-4-(isopropylamino)-N-(2-methoxyethyl)-N-methylnicotinamide | ¹H NMR (400 MHz, Chloroform-*d*) δ 9.40 (s, 1H), 8.63 (s, 1H), 8.44 (s, 1H), 8.40 (d, *J* = 5.8 Hz, 1H), 7.89 (s, 1H), 7.59 (s, 1H), 7.02 (d, *J* = 5.8 Hz, 1H), 6.16 (d, *J* = 7.3 Hz, 1H), 3.90-3.77 (m, *J* = 6.4 Hz, 1H), 3.69 (t, *J* = 5.1 Hz, 2H), 3.61 (t, *J* = 5.2 Hz, 2H), 3.37 (s, 3H), 3.12 (s, 3H), 2.83 (tt, *J* = 8.0, 4.7 Hz, 1H), 1.56-1.49 (m, 2H), 1.35 (d, *J* = 6.4 Hz, 6H), 1.25-1.19 (m, 2H); 515.22 [M+H]⁺ |
| 28 | 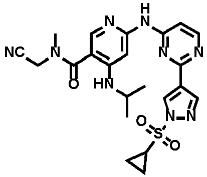 | N-(cyanomethyl)-6-((2-(1-(cyclopropylsulfonyl )-1H-pyrazol-4-yl)pyrimidin-4-yl)amino)-4-(isopropylamino)-N-methylnicotinamide | ¹H NMR (400 MHz, Chloroform-*d*) δ 8.64 (d, *J* = 0.6 Hz, 1H), 8.45 (d, *J* = 0.6 Hz, 1H), 8.43 (d, *J* = 5.8 Hz, 1H), 8.32 (s, 1H), 7.96 (s, 1H), 7.44 (s, 1H), 7.07 (d, *J* = 5.8 Hz, 1H), 6.56 (d, *J* = 7.1 Hz, 1H), 4.39 (s, 2H), 3.86-3.77 (m, 1H), 3.27 (s, 3H), 2.83 (tt, *J* = 8.0, 4.7 Hz, 1H), 1.56-1.51 (m, 2H), 1.36 (d, *J* = 6.4 Hz, 6H), 1.25-1.19 (m, 2H); 496.20 [M+H]⁺ |
| 29 | 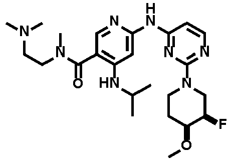 | N-(2-(dimethylamino)ethyl )-6-((2-(cis-3-fluoro-4-methoxypiperidin-1-yl)pyrimidin-4-yl)amino)-4-(isopropylamino)-N-methylnicotinamide | ¹H NMR (400 MHz, Chloroform-*d*) δ 8.05 (d, *J* = 5.6 Hz, 1H), 7.90 (s, 1H), 7.31 (s, 1H), 6.23 (d, *J* = 5.7 Hz, 1H), 5.91 (d, *J* = 7.7 Hz, 1H), 4.77 (ddt, *J* = 47.7, 6.1, 2.8 Hz, 1H), 4.42 (dt, *J* = 14.0, 7.0 Hz, 1H), 4.13 (dq, *J* = 11.1, 4.1 Hz, 1H), 3.88-3.72 (m, 2H), 3.62 (t, *J* = 6.5 Hz, 4H), 3.50 (s, 3H), 3.05 (s, 3H), 2.52 (t, *J* = 6.5 Hz, 2H), 2.24 (s, 6H), 2.05-1.95 (m, 1H), 1.83-1.75 (m, 1H), 1.26 (dd, *J* = 6.3, 1.5 Hz, 6H); 489.32 [M+H]⁺ |
| 30 | 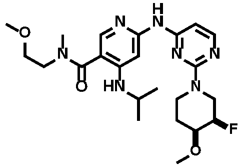 | 6-((2-(cis-3-fluoro-4-methoxypiperidin-1-yl)pyrimidin-4-yl)amino)-4-(isopropylamino)-N-(2-methoxyethyl)-N-methylnicotinamide | ¹H NMR (400 MHz, Chloroform-*d*) δ 8.11 (s, 1H, br), 8.06 (d, *J* = 5.6 Hz, 1H), 7.89 (s, 1H), 7.42 (s, 1H), 6.26 (d, *J* = 5.6 Hz, 1H), 6.08 (d, *J* = 7.6 Hz, 1H), 4.86-4.69 (m, 1H), 4.45-4.36 (m, 1H), 4.15-4.08 (m, 1H), 3.89-3.56 (m, 8H), 3.50 (s, 3H), 3.36 (s, 3H), 3.12 (s, 3H), 2.05-1.95 (m, 1H), 1.83-1.75 (m, 1H), 1.27 (dd, *J* = 6.3, 1.8 Hz, 6H); 476.30 [M+H]⁺ |
| 31 | 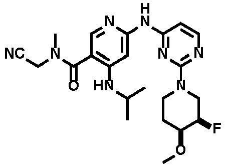 | N-(cyanomethyl)-6-((2-(cis-3-fluoro-4-methoxypiperidin-1-yl)pyrimidin-4-yl)amino)-4-(isopropylamino)-N-methylnicotinamide | ¹H NMR (400 MHz, Chloroform-*d*) δ 12.19 (s, 1H), 8.18 (d, *J* = 5.5 Hz, 1H), 7.94 (s, 1H), 7.75 (s, 1H), 7.38 (d, *J* = 7.7 Hz, 1H), 6.50 (d, *J* = 5.5 Hz, 1H), 4.85-4.67 (m, 1H), 4.36 (s, 2H), 4.35-4.27 (m, 1H), 4.08-3.99 (m, 1H), 3.94-3.81 (m, 2H), 3.76-3.61 (m, 2H), 3.51 (s, 3H), 3.29 (s, 3H), 2.06-1.96 (m, 1H), 1.84-1.76 (m, 1H), 1.36 (dd, *J* = 6.4, 4.6 Hz, 6H); 457.24 [M+H]⁺ |
| 32 | 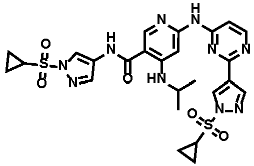 | N-(1-(cyclopropylsulfonyl )-1H-pyrazol-4-yl)-6-((2-(1-(cyclopropylsulfonyl )-1H-pyrazol-4-yl)pyrimidin-4-yl)amino)-4-(isopropylamino)nico tinamide | ¹H NMR (400 MHz, Chloroform-*d*) δ 8.64 (s, 1H), 8.52 (s, 1H), 8.45 (s, 1H), 8.43 (d, *J* = 5.7 Hz, 1H), 8.35 (d, *J* = 7.3 Hz, 1H), 8.30 (s, 1H), 8.26 (s, 1H), 7.93 (s, 1H), 7.80 (s, 1H), 7.50 (s, 1H), 7.02-6.98 (m, 1H), 3.91-3.82 (m, 1H), 2.87-2.73 (m, 2H), 1.56-1.48 (m, 4H), 1.40 (d, *J* = 6.4 Hz, 6H), 1.26-1.14 (m, 4H); 613.21 [M+H]⁺ |
| 33 | 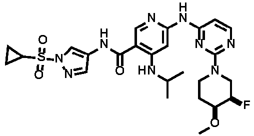 | N-(1-(cyclopropylsulfonyl )-1H-pyrazol-4-yl)-6-((2-(cis-3-fluoro-4-methoxypiperidin-1-yl)pyrimidin-4-yl)amino)-4-(isopropylamino)nico tinamide | ¹H NMR (400 MHz, Chloroform-*d*) δ 9.36 (s, 1H), 8.53 (d, *J* = 7.6 Hz, 1H), 8.51 (s, 1H), 8.35 (s, 1H), 8.23 (s, 1H), 8.07 (d, *J* = 5.5 Hz, 1H), 7.94 (d, *J* = 0.8 Hz, 1H), 7.62 (s, 1H), 6.22 (d, *J* = 5.6 Hz, 1H), 4.86-4.69 (m, 1H), 4.45-4.35 (m, 1H), 4.16-4.05 (m, 1H), 3.90-3.77 (m, 2H), 3.70-3.58 (m, 2H), 3.51 (s, 3H), 2.76 (tt, *J* = 8.0, 4.7 Hz, 1H), 2.07-1.94 (m, 1H), 1.85-1.74 (m, 1H), 1.54-1.46 (m, 2H), 1.33 (dd, *J* = 6.4, 3.5 Hz, 6H), 1.21-1.14 (m, 2H); 574.25 [M+H]⁺ |
| 34 | 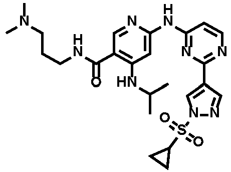 | 6-((2-(1-(cyclopropylsulfonyl )-1H-pyrazol-4-yl)pyrimidin-4-yl)amino)-N-(3-(dimethylamino)propy l)-4-(isopropylamino)nico tinamide | ¹H NMR (400 MHz, Chloroform-*d*) δ 12.50 (s, 1H), 9.49 (d, *J* = 7.3 Hz, 1H), 8.58 (t, *J* = 4.3 Hz, 1H), 8.53 (d, *J* = 6.2 Hz, 2H), 8.44 (d, *J* = 5.7 Hz, 1H), 8.37 (s, 1H), 7.90 (s, 1H), 7.05 (d, *J* = 5.8 Hz, 1H), 3.86 (h, *J* = 6.6 Hz, 1H), 3.56-3.45 (m, 2H), 3.23 (t, *J* = 7.6 Hz, 2H), 2.92-2.82 (m, 7H), 2.15-2.10 (m, 2H), 1.58-1.52 (m, 2H), 1.41 (d, *J* = 6.4 Hz, 6H), 1.28-1.22 (m, 2H); 528.23 [M+H]⁺ |
| 35 | 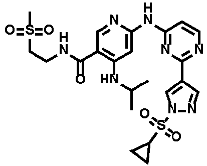 | 6-((2-(1-(cyclopropylsulfonyl )-1H-pyrazol-4-yl)pyrimidin-4-yl)amino)-4-(isopropylamino)-N-(2-(methylsulfonyl)ethy l)nicotinamide | ¹H NMR (400 MHz, Chloroform-*d*) δ 9.22 (s, 1H), 8.64-8.60 (m, 1H), 8.46-8.39 (m, 3H), 8.13 (s, 1H), 7.61 (s, 1H), 6.97 (d, *J* = 5.8 Hz, 1H), 6.84 (t, *J* = 5.9 Hz, 1H), 3.99-3.92 (m, 2H), 3.90-3.80 (m, 1H), 3.38-3.31 (m, 2H), 3.01 (s, 3H), 2.87-2.79 (m, 1H), 1.56-1.50 (m, 2H), 1.39 (d, *J* = 6.4 Hz, 6H), 1.25-1.19 (m, 2H); 549.14 [M+H]⁺ |
| 36 | 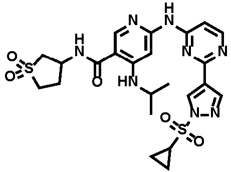 | 6-((2-(1-(cyclopropylsulfonyl )-1H-pyrazol-4-yl)pyrimidin-4-yl)amino)-N-(1,1-dioxydotetrahydrothi ophen-3-yl)-4-(isopropylamino)nico tinamide | ¹H NMR (400 MHz, DMSO-*d*6) δ 10.20 (s, 1H), 8.62 (s, 1H), 8.61 (d, *J* = 6.9 Hz, 1H), 8.47-8.41 (m, 4H), 7.53 (s, 1H), 7.32 (d, *J* = 5.2 Hz, 1H), 4.66 (h, *J* = 7.6 Hz, 1H), 3.79 (h, *J* = 6.6 Hz, 1H), 3.49 (dd, *J* = 13.4, 7.8 Hz, 1H), 3.38 (ddd, *J* = 13.0, 7.8, 5.0 Hz, 1H), 3.30-3.24 (m, 1H), 3.18 (ddd, *J* = 13.3, 9.3, 7.6 Hz, 1H), 3.06 (dd, *J* = 13.4, 7.9 Hz, 1H), 2.47-2.39 (m, 1H), 2.27-2.16 (m, 1H), 1.37-1.31 (m, 2H), 1.29-1.23 (m, 8H); 561.14 [M+H]⁺ |
| 37 | 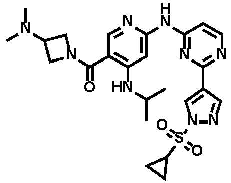 | (6-((2-(1-(cyclopropylsulfonyl )-1H-pyrazol-4-yl)pyrimidin-4-yl)amino)-4-(isopropylamino)pyri din-3-yl) (3-(dimethylamino)azeti din-1-yl)methanone | ¹H NMR (400 MHz, Chloroform-*d*) δ 8.78 (s, 1H), 8.61 (s, 1H), 8.49 (d, *J* = 5.8 Hz, 1H), 8.43 (s, 1H), 7.93 (s, 1H), 7.84 (s, 1H), 7.13 (d, *J* = 5.7 Hz, 1H), 3.88 (dd, *J* = 13.2, 6.5 Hz, 1H), 3.23-3.06 (m, 1H), 2.91-2.75 (m, 1H), 2.19 (d, *J* = 13.2 Hz, 6H), 1.56-1.52 (m, 2H), 1.42 (d, *J* = 6.4 Hz, 6H), 1.23 (dd, *J* = 7.7, 5.9 Hz, 6H); 526.3 [M+H]⁺ |
| 38 | 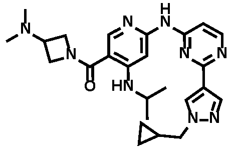 | (6-((2-(1-(cyclopropylmethyl)-1H-pyrazol-4-yl)pyrimidin-4-yl)amino)-4-(isopropylamino)pyri din-3-yl) (3-(dimethylamino)azeti din-1-yl)methanone | ¹H NMR (400 MHz, Chloroform-*d*) δ 8.38 (d, *J* = 5.8 Hz, 1H), 8.21 (s, 1H), 8.17 (d, *J* = 8.5 Hz, 1H), 8.08 (d, *J* = 7.8 Hz, 2H), 7.95 (d, *J* = 7.2 Hz, 1H), 7.14 (d, *J* = 10.5 Hz, 1H), 7.08 (d, *J* = 5.8 Hz, 1H), 4.04 (d, *J* = 7.1 Hz, 2H), 3.76 (dq, *J* = 13.0, 6.4 Hz, 1H), 3.14-3.04 (m, 1H), 2.18 (d, *J* = 5.3 Hz, 6H), 1.34-1.24 (m, 11H), 0.75-0.63 (m, 2H), 0.42 (q, *J* = 4.9 Hz, 2H); 476.3 [M+H]⁺ |
| 39 | 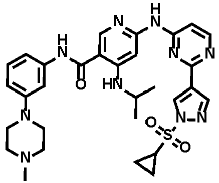 | 6-((2-(1-(cyclopropylsulfonyl )-1H-pyrazol-4-yl)pyrimidin-4-yl)amino)-4-(isopropylamino)-N-(3-(4-methylpiperazin-1-yl)phenyl)nicotinami de | ¹H NMR (400 MHz, Chloroform-*d*) δ 8.59 (d, *J* = 25.4 Hz, 2H), 8.36 (dd, *J* = 15.2, 8.3 Hz, 3H), 8.19 (d, *J* = 6.8 Hz, 2H), 7.48 (s, 1H), 7.24 (t, *J* = 5.2 Hz, 2H), 6.98 (d, *J* = 8.9 Hz, 1H), 6.83 (d, *J* = 5.5 Hz, 1H), 6.74 (dd, *J* = 8.3, 2.0 Hz, 1H), 3.79 (dd, *J* = 13.0, 6.5 Hz, 1H), 3.26-3.20 (m, 4H), 2.82 (td, *J* = 7.9, 4.0 Hz, 1H), 2.61-2.55 (m, 4H), 2.36 (s, 3H), 1.55-1.48 (m, 2H), 1.35 (d, *J* = 6.3 Hz, 6H), 1.20 (dd, *J* = 7.9, 1.8 Hz, 2H); 617.3 [M+H]⁺ |
| 40 | 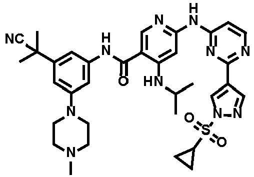 | N-(3-(2-cyanopropan-2-yl)-5-(4-methylpiperazin-1-yl)phenyl)-6-((2-(1-(cyclopropylsulfonyl )-1H-pyrazol-4-yl)pyrimidin-4-yl)amino)-4-(isopropylamino)nico tinamide | ¹H NMR (400 MHz, Chloroform-*d*) δ 8.65 (s, 1H), 8.47-8.41 (m, 2H), 8.35 (d, *J* = 8.4 Hz, 1H), 8.13 (d, *J* = 7.3 Hz, 1H), 7.69 (s, 1H), 7.33 (s, 1H), 7.19 (s, 1H), 7.06 (d, *J* = 6.0 Hz, 1H), 7.00 (s, 1H), 6.86 (s, 1H), 3.83 (dd, *J* = 13.0, 6.4 Hz, 1H), 3.31-3.27 (m, 4H), 2.88-2.78 (m, 1H), 2.61 (s, 4H), 2.38 (s, 3H), 1.74 (s, 6H), 1.54-1.51 (m, 2H), 1.38 (d, *J* = 6.4 Hz, 6H), 1.24-1.21 (m, 2H); 684.4 [M+H]⁺ |
| 41 | 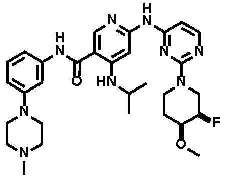 | 6-((2-(cis-3-fluoro-4-methoxypiperidin-1-yl)pyrimidin-4-yl)amino)-4-(isopropylamino)-N-(3-(4-methylpiperazin-1-yl)phenyl)nicotinami de | ¹H NMR (400 MHz, DMSO) δ 9.94 (s, 1H), 9.78 (s, 1H), 8.50 (s, 1H), 8.13 (d, *J* = 7.6 Hz, 1H), 8.02 (d, *J* = 5.6 Hz, 1H), 7.42 (s, 1H), 7.25 (s, 1H), 7.20-7.10 (m, 2H), 6.68 (dd, *J* = 7.2, 2.2 Hz, 1H), 6.59 (d, *J* = 5.5 Hz, 1H), 5.02-4.80 (m, 1H), 4.67 (d, *J* = 13.8 Hz, 1H), 4.40 (d, *J* = 13.4 Hz, 1H), 3.74-3.17 (m, 4H), 3.13-3.07 (m, 4H), 2.49-2.43 (m, 4H), 2.22 (s, 3H), 1.88 (s, 3H), 1.81-1.66 (m, 2H), 1.24 (dd, *J* = 6.2, 2.8 Hz, 6H); 578.4 [M+H]⁺ |
| 42 | 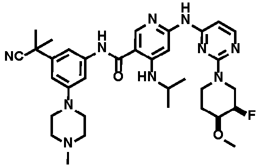 | N-(3-(2-cyanopropan-2-yl)-5-(4-methylpiperazin-1-yl)phenyl)-6-((2-(cis-3-fluoro-4-methoxypiperidin-1-yl)pyrimidin-4-yl)amino)-4-(isopropylamino)nico tinamide | ¹H NMR (400 MHz, Chloroform-*d*) δ 8.32 (s, 1H), 8.08 (dd, *J* = 16.4, 6.6 Hz, 2H), 7.90 (s, 1H), 7.52 (s, 1H), 7.40 (s, 1H), 7.18 (s, 1H), 7.01 (s, 1H), 6.85 (s, 1H), 6.14 (d, *J* = 5.6 Hz, 1H), 4.78 (dd, *J* = 48.0, 6.1 Hz, 1H), 4.52-4.36 (m, 1H), 4.14 (d, *J* = 7.2 Hz, 1H), 3.78 (dt, *J* = 12.7, 8.9 Hz, 2H), 3.61 (dd, *J* = 19.0, 7.8 Hz, 2H), 3.50 (s, 3H), 3.31-3.23 (m, 4H), 2.61-2.53 (m, 4H), 2.36 (s, 3H), 2.06-1.92 (m, 2H), 1.72 (d, *J* = 9.9 Hz, 6H), 1.35-1.29 (m, 6H); 645.4 [M+H]⁺ |
| 43 | 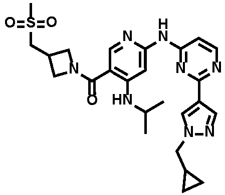 | (6-((2-(1-(cyclopropylmethyl)-1H-pyrazol-4-yl)pyrimidin-4-yl)amino)-4-(isopropylamino)pyri din-3-yl) (3-((methylsulfonyl)met hyl)azetidin-1-yl)methanone | ¹H NMR (400 MHz, Chloroform-*d*) δ 8.39 (d, *J* = 5.8 Hz, 1H), 8.21 (s, 1H), 8.15 (s, 1H), 8.06-8.02 (m, 2H), 7.79 (s, 1H), 7.21 (s, 1H), 7.06 (d, *J* = 5.8 Hz, 1H), 4.55 (t, *J* = 8.8 Hz, 2H), 4.18 (s, 2H), 4.04 (d, *J* = 7.1 Hz, 2H), 3.86-3.75 (m, 1H), 3.39 (d, *J* = 7.3 Hz, 2H), 3.37-3.29 (m, 1H), 2.95 (s, 3H), 1.34 (d, *J* = 6.4 Hz, 6H), 0.86-0.81 (m, 1H), 0.75-0.65 (m, 2H), 0.42 (dt, *J* = 6.0, 4.8 Hz, 2H); 525.32 [M+H]⁺ |
| 44 | 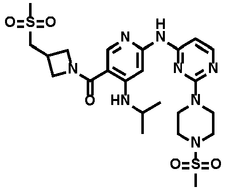 | (4-(isopropylamino)-6-((2-(4-(methylsulfonyl) pipe razin-1-yl)pyrimidin-4-yl)amino)pyridin-3-yl) (3-((methylsulfonyl)met hyl)azetidin-1-yl)methanone | ¹H NMR (400 MHz, Chloroform-*d*) δ 12.14 (s, 1H), 8.95 (d, *J* = 7.6 Hz, 1H), 8.16 (d, *J* = 5.5 Hz, 1H), 7.79 (s, 1H), 7.75 (s, 1H), 6.52 (d, *J* = 5.5 Hz, 1H), 4.63-4.50 (m, 2H), 4.22 (s, 2H), 3.96 (t, *J* = 5.1 Hz, 4H), 3.80-3.68 (m, 1H), 3.45-3.33 (m, 3H), 3.29 (t, *J* = 5.1 Hz, 4H), 2.99 (s, 3H), 2.81 (s, 3H), 1.36 (d, *J* = 6.4 Hz, 6H); 567.24 [M+H]⁺ |
| 45 | 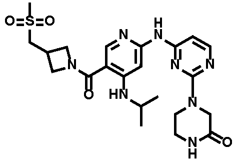 | 4-(4-((4-(isopropylamino)-5-(3-((methylsulfonyl)met hyl)azetidine-1-carbonyl)pyridin-2-yl)amino)pyrimidin-2-yl)piperazin-2-one | 503.21[M+H]⁺ |
| 46 | 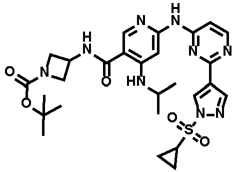 | tert-butyl 3-(6-((2-(1-(cyclopropylsulfonyl )-1H-pyrazol-4-yl)pyrimidin-4-yl)amino)-4-(isopropylamino)nico tinamido)azetidine-1-carboxylate | ¹H NMR (400 MHz, Chloroform-*d*) δ 10.38 (s, 1H), 8.67 (d, *J* = 7.3 Hz, 1H), 8.61 (d, *J* = 0.6 Hz, 1H), 8.44 (s, 1H), 8.42 (d, *J* = 5.8 Hz, 1H), 8.28 (s, 1H), 7.82 (s, 1H), 6.92 (d, *J* = 5.8 Hz, 1H), 6.68 (d, *J* = 6.7 Hz, 1H), 4.75-4.64 (m, 1H), 4.33 (dd, *J* = 9.4, 7.6 Hz, 2H), 3.92-3.82 (m, 3H), 2.83 (tt, *J* = 7.9, 4.7 Hz, 1H), 1.56-1.50 (m, 2H), 1.46 (s, 9H), 1.39 (d, *J* = 6.4 Hz, 6H), 1.27-1.19 (m, 2H); 598.25 [M+H]⁺ |
| 47 | 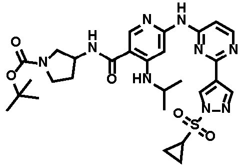 | tert-butyl 3-(6-((2-(1-(cyclopropylsulfonyl )-1H-pyrazol-4-yl)pyrimidin-4-yl)amino)-4-(isopropylamino)nico tinamido)pyrrolidine -1-carboxylate | ¹H NMR (400 MHz, Chloroform-*d*) δ 10.51 (s, 1H), 8.72 (d, *J* = 7.3 Hz, 1H), 8.61 (s, 1H), 8.44 (s, 1H), 8.41 (d, *J* = 5.8 Hz, 1H), 8.18 (s, 1H), 7.82 (s, 1H), 6.92 (d, *J* = 5.8 Hz, 1H), 6.39 (s, 1H), 4.61-4.52 (m, 1H), 3.92-3.80 (m, 1H), 3.70 (s, 1H), 3.50 (s, 2H), 3.04-2.99 (m, 1H), 2.84 (tt, *J* = 7.9, 4.7 Hz, 1H), 2.30-2.19 (m, 1H), 1.96 (s, 1H), 1.56-1.50 (m, 2H), 1.48 (s, 9H), 1.40 (d, *J* = 6.4 Hz, 6H), 1.26-1.20 (m, 2H); 612.24 [M+H]⁺ |
| 48 | 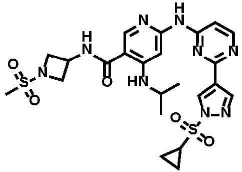 | 6-((2-(1-(cyclopropylsulfonyl )-1H-pyrazol-4-yl)pyrimidin-4-yl)amino)-4-(isopropylamino)-N-(1-(methylsulfonyl)azet idin-3-yl)nicotinamide | 576.19[M+H]⁺ |
| 49 | 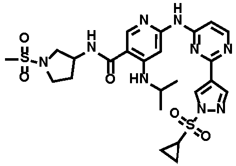 | 6-((2-(1-(cyclopropylsulfonyl )-1H-pyrazol-4-yl)pyrimidin-4-yl)amino)-4-(isopropylamino)-N-(1-(methylsulfonyl)pyrr olidin-3-yl)nicotinamide | ¹H NMR (400 MHz, Chloroform-*d*) δ 8.87 (s, 1H), 8.62 (d, *J* = 0.6 Hz, 1H), 8.43 (d, *J* = 4.4 Hz, 2H), 8.41 (d, *J* = 5.8 Hz, 1H), 8.15 (s, 1H), 7.53 (s, 1H), 6.99 (d, *J* = 5.8 Hz, 1H), 6.39 (d, *J* = 6.9 Hz, 1H), 4.64 (dq, *J* = 10.6, 6.3, 5.3 Hz, 1H), 3.89-3.78 (m, 1H), 3.65 (dt, *J* = 10.3, 7.3 Hz, 1H), 3.59 (dd, *J* = 10.9, 5.8 Hz, 1H), 3.46 (dd, *J* = 10.9, 3.6 Hz, 1H), 3.39 (ddd, *J* = 10.3, 8.4, 5.6 Hz, 1H), 2.93 (s, 3H), 2.83 (tt, *J* = 7.9, 4.7 Hz, 1H), 2.40-2.30 (m, 1H), 2.12-2.03 (m, 1H), 1.56-1.49 (m, 2H), 1.38 (d, *J* = 6.4 Hz, 6H), 1.27-1.19 (m, 2H); 590.22 [M+H]⁺ |
| 50 | 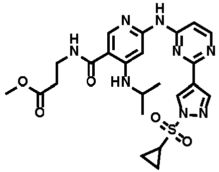 | methyl 3-(6-((2-(1-(cyclopropylsulfonyl )-1H-pyrazol-4-yl)pyrimidin-4-yl)amino)-4-(isopropylamino)nico tinamido)propanoate | ¹H NMR (400 MHz, DMSO) δ 10.17 (s, 1H), 8.63 (s, 1H), 8.50 (dd, *J* = 11.6, 6.3 Hz, 2H), 8.47-8.42 (m, 2H), 8.39 (s, 1H), 7.50 (s, 1H), 7.33 (s, 1H), 3.79-3.74 (m, 1H), 3.62 (s, 3H), 3.46-3.43 (m, 2H), 3.31-3.25 (m, 1H), 2.60-2.56 (m, 2H), 1.34 (d, *J* = 3.9 Hz, 2H), 1.27 (d, *J* = 6.2 Hz, 6H), 1.16 (d, *J* = 6.3 Hz, 2H); 529.2 [M+H]⁺ |
| 51 | 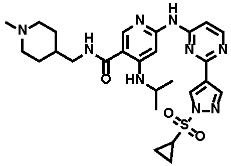 | 6-((2-(1-(cyclopropylsulfonyl )-1H-pyrazol-4-yl)pyrimidin-4-yl)amino)-4-(isopropylamino)-N-((1-methylpiperidin-4-yl)methyl)nicotinami de | ¹H NMR (400 MHz, DMSO) δ 10.16 (s, 1H), 8.62 (s, 1H), 8.55 (d, *J* = 7.2 Hz, 1H), 8.46-8.39 (m, 4H), 7.49 (s, 1H), 7.34 (s, 1H), 3.76 (dd, *J* = 13.0, 6.6 Hz, 1H), 3.28 (d, *J* = 4.8 Hz, 1H), 3.09 (t, *J* = 6.2 Hz, 2H), 2.77 (d, *J* = 10.4 Hz, 2H), 2.16 (s, 3H), 1.84 (s, 2H), 1.64 (d, *J* = 11.4 Hz, 2H), 1.49 (s, 1H), 1.34 (dd, *J* = 10.4, 5.9 Hz, 2H), 1.25 (t, *J* = 7.8 Hz, 8H), 1.17 (dd, *J* = 11.6, 2.9 Hz, 2H); 554.4 [M+H]⁺ |
| 52 | 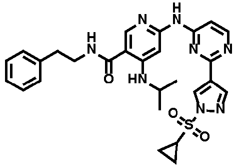 | 6-((2-(1-(cyclopropylsulfonyl )-1H-pyrazol-4-yl)pyrimidin-4-yl)amino)-4-(isopropylamino)-N-phenethylnicotinamid e | ¹H NMR (400 MHz, Chloroform-*d*) δ 8.64 (s, 1H), 8.42 (dd, *J* = 11.1, 5.2 Hz, 2H), 8.35 (d, *J* = 6.7 Hz, 1H), 8.01 (s, 1H), 7.35 (t, *J* = 7.3 Hz, 2H), 7.24 (s, 4H), 7.07 (d, *J* = 5.8 Hz, 1H), 6.01 (s, 1H), 3.83-3.77 (m, 1H), 3.68 (dd, *J* = 12.7, 6.7 Hz, 2H), 2.93 (t, *J* = 6.8 Hz, 2H), 2.83 (ddd, *J* = 11.1, 7.3, 3.9 Hz, 1H), 1.53 (dd, *J* = 4.7, 2.1 Hz, 2H), 1.36 (d, *J* = 6.3 Hz, 6H), 1.21 (d, *J* = 1.8 Hz, 2H); 547.2 [M+H]⁺ |
| 53 | 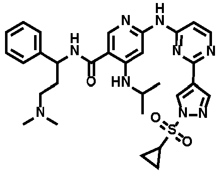 | 6-((2-(1-(cyclopropylsulfonyl )-1H-pyrazol-4-yl)pyrimidin-4-yl)amino)-N-(3-(dimethylamino)-1-phenylpropyl)-4-(isopropylamino)nico tinamide | ¹H NMR (400 MHz, Chloroform-*d*) δ 8.76 (d, *J* = 6.6 Hz, 1H), 8.62 (s, 1H), 8.43 (dd, *J* = 16.4, 7.0 Hz, 3H), 7.34 (dd, *J* = 14.4, 5.7 Hz, 5H), 7.26-7.23 (m, 2H), 7.05 (d, *J* = 5.7 Hz, 1H), 5.23 (dd, *J* = 11.2, 6.2 Hz, 1H), 3.76 (dd, *J* = 13.1, 6.6 Hz, 1H), 2.84 (ddd, *J* = 12.6, 8.0, 4.7 Hz, 1H), 2.50 (s, 6H), 2.30-2.17 (m, 2H), 1.80-1.57 (m, 2H), 1.55-1.52 (m, 2H), 1.32-1.28 (m, 6H), 1.22 (dd, *J* = 8.0, 1.8 Hz, 2H); 604.3 [M+H]⁺ |
| 54 | 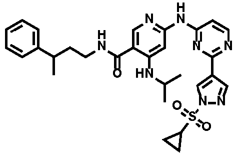 | 6-((2-(1-(cyclopropylsulfonyl )-1H-pyrazol-4-yl)pyrimidin-4-yl)amino)-4-(isopropylamino)-N-(3-phenylbutyl)nicotina mide | ¹H NMR (400 MHz, Chloroform-*d*) δ 8.63 (s, 1H), 8.52-8.38 (m, 3H), 7.76 (s, 1H), 7.38-7.27 (m, 3H), 7.25 (d, *J* = 8.0 Hz, 4H), 7.04 (t, *J* = 13.4 Hz, 1H), 5.76 (s, 1H), 3.78 (dq, *J* = 13.2, 6.4 Hz, 1H), 3.45 (td, *J* = 13.5, 6.5 Hz, 1H), 3.25 (td, *J* = 12.4, 7.0 Hz, 1H), 2.88-2.78 (m, 2H), 1.94 (ddd, *J* = 20.9, 13.5, 5.5 Hz, 2H), 1.54-1.51 (m, 2H), 1.34 (dd, *J* = 9.2, 6.7 Hz, 9H), 1.22 (dd, *J* = 7.9, 1.9 Hz, 2H); 575.3 [M+H]⁺ |
| 55 | 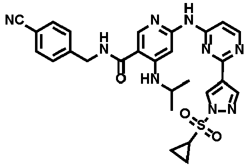 | N-(4-cyanobenzyl)-6-((2-(1-(cyclopropylsulfonyl )-1H-pyrazol-4-yl)pyrimidin-4-yl)amino)-4-(isopropylamino)nico tinamide | ¹H NMR (400 MHz, Chloroform-*d*) δ 8.63 (s, 1H), 8.42 (dd, *J* = 12.7, 6.9 Hz, 3H), 8.25 (s, 1H), 7.68-7.64 (m, 2H), 7.46 (d, *J* = 8.2 Hz, 2H), 7.33 (s, 1H), 7.05 (d, *J* = 5.8 Hz, 1H), 6.55 (s, 1H), 4.66 (d, *J* = 6.0 Hz, 2H), 3.81 (dd, *J* = 13.1, 6.5 Hz, 1H), 2.83 (td, *J* = 8.0, 4.0 Hz, 1H), 1.56-1.51 (m, 2H), 1.37 (d, *J* = 6.4 Hz, 6H), 1.23 (dd, *J* = 8.0, 1.8 Hz, 2H); 558.2 [M+H]⁺ |
| 56 | 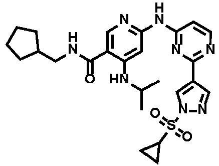 | N-(cyclopentylmethyl)-6-((2-(1-(cyclopropylsulfonyl )-1H-pyrazol-4-yl)pyrimidin-4-yl)amino)-4-(isopropylamino)-nicotinamide | ¹H NMR (400 MHz, Chloroform-*d*) δ 8.63 (s, 1H), 8.48-8.30 (m, 3H), 8.19 (s, 1H), 7.10 (d, *J* = 5.7 Hz, 1H), 3.78 (dd, *J* = 12.8, 6.4 Hz, 1H), 3.35 (dd, *J* = 7.2, 5.7 Hz, 2H), 2.88-2.77 (m, 1H), 2.23-2.08 (m, 1H), 1.70 (m, *J* = 18.9, 15.5, 9.8, 4.2 Hz, 8H), 1.53-1.48 (m, 2H), 1.35 (d, *J* = 6.3 Hz, 6H), 1.21 (dd, *J* = 7.5, 5.6 Hz, 2H); 525.3 [M+H]⁺ |
| 57 | 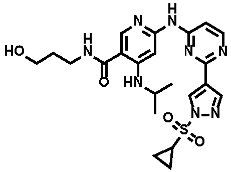 | 6-((2-(1-(cyclopropylsulfonyl )-1H-pyrazol-4-yl)pyrimidin-4-yl)amino)-N-(3-hydroxypropyl)-4-(isopropylamino)nico tinamide | ¹H NMR (400 MHz, Chloroform-*d*) δ 8.63 (s, 1H), 8.44 (dd, *J* = 10.3, 6.6 Hz, 3H), 8.18 (s, 1H), 7.28 (s, 1H), 7.09 (d, *J* = 5.8 Hz, 1H), 6.71 (s, 1H), 3.80 (dd, *J* = 11.9, 6.3 Hz, 3H), 3.59 (dd, *J* = 12.0, 5.8 Hz, 2H), 2.83 (ddd, *J* = 12.7, 8.0, 4.7 Hz, 1H), 1.82 (d, *J* = 5.5 Hz, 2H), 1.53 (dd, *J* = 4.8, 2.0 Hz, 2H), 1.36 (d, *J* = 6.4 Hz, 6H), 1.22 (dd, *J* = 8.0, 1.8 Hz, 2H); 501.2 [M+H]⁺ |
| 58 | 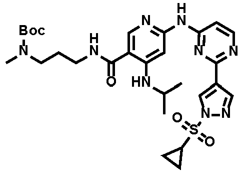 | tert-butyl (3-(6-((2-(1-(cyclopropylsulfonyl )-1H-pyrazol-4-yl)pyrimidin-4-yl)amino)-4-(isopropylamino)nico tinamido)propyl) (met hyl)carbamate | ¹H NMR (400 MHz, Chloroform-*d*) δ 8.64 (s, 1H), 8.50-8.33 (m, 3H), 7.85 (s, 1H), 7.10 (d, *J* = 5.7 Hz, 1H), 3.79 (dt, *J* = 13.0, 6.5 Hz, 1H), 3.38 (s, 4H), 2.86 (s, 3H), 2.85-2.80 (m, 1H), 1.74 (s, 2H), 1.53 (dd, *J* = 4.8, 2.1 Hz, 2H), 1.48 (s, 9H), 1.36 (d, *J* = 6.4 Hz, 6H), 1.22 (dd, *J* = 8.0, 1.8 Hz, 2H); 614.3 [M+H]⁺ |
| 59 | 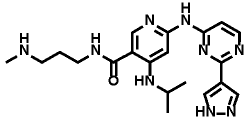 | 6-((2-(1H-pyrazol-4-yl)pyrimidin-4-yl)amino)-4-(isopropylamino)-N-(3-(methylamino)propyl) nicotinamide | 410.2 [M+H]⁺ |
| 60 | 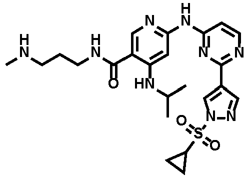 | 6-((2-(1-(cyclopropylsulfonyl )-1H-pyrazol-4-yl)pyrimidin-4-yl)amino)-4-(isopropylamino)-N-(3-(methylamino)propyl) nicotinamide | 514.3 [M+H]⁺ |
| 61 | 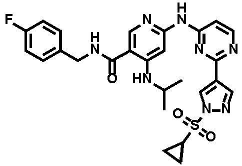 | 6-((2-(1-(cyclopropylsulfonyl )-1H-pyrazol-4-yl)pyrimidin-4-yl)amino)-N-(4-fluorobenzyl)-4-(isopropylamino)nico tinamide | ¹H NMR (400 MHz, Chloroform-*d*) δ 8.96 (s, 1H), 8.54 (s, 1H), 8.40 (d, *J* = 7.2 Hz, 1H), 8.36 (s, 1H), 8.28 (d, *J* = 5.8 Hz, 1H), 8.19 (s, 1H), 7.44 (s, 1H), 7.36-7.28 (m, 2H), 7.08-6.97 (m, 2H), 6.87-6.77 (m, 2H), 4.56 (d, *J* = 5.6 Hz, 2H), 3.82-3.70 (m, 1H), 2.82 (tt, *J* = 8.0, 4.7 Hz, 1H), 1.48 (tt, *J* = 6.0, 3.3 Hz, 2H), 1.34 (d, *J* = 6.3 Hz, 6H), 1.19 (qd, *J* = 6.2, 1.4 Hz, 2H); 551.2 [M+H]⁺ |
| 62 | 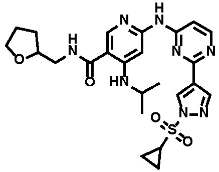 | 6-((2-(1-(cyclopropylsulfonyl )-1H-pyrazol-4-yl)pyrimidin-4-yl)amino)-4-(isopropylamino)-N-((tetrahydrofuran-2-yl)methyl)nicotinami de | ¹H NMR (400 MHz, Chloroform-*d*) δ 9.15 (s, 1H), 8.62 (s, 1H), 8.44 (s, 1H), 8.37 (dd, *J* = 6.8, 4.8 Hz, 2H), 8.29 (s, 1H), 7.58 (s, 1H), 7.15 (t, *J* = 5.8 Hz, 1H), 6.89 (d, *J* = 5.9 Hz, 1H), 4.17 (qd, *J* = 6.8, 2.9 Hz, 1H), 3.98 (dt, *J* = 8.4, 6.6 Hz, 1H), 3.88-3.78 (m, 2H), 3.65 (ddd, *J* = 14.0, 5.7, 3.1 Hz, 1H), 3.53 (dt, *J* = 14.0, 6.0 Hz, 1H), 2.84 (tt, *J* = 7.9, 4.7 Hz, 1H), 2.11-2.02 (m, 1H), 2.01-1.93 (m, 2H), 1.76-1.63 (m, 1H), 1.52 (qd, *J* = 5.5, 4.7, 2.4 Hz, 2H), 1.37 (d, *J* = 3.5 Hz, 3H), 1.35 (d, *J* = 3.6 Hz, 3H), 1.22 (dt, *J* = 7.8, 1.6 Hz, 2H); 527.2 [M+H]⁺ |
| 63 | 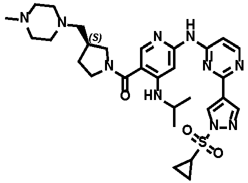 | (S)-(6-((2-(1-(cyclopropylsulfonyl )-1H-pyrazol-4-yl)pyrimidin-4-yl)amino)-4-(isopropylamino)pyri din-3-yl) (3-((4-methylpiperazin-1-yl)methyl)pyrrolidin-1-yl)methanone | 609.33 [M+H]⁺ |
| 64 | 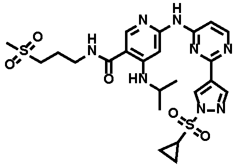 | 6-((2-(1-(cyclopropylsulfonyl )-1H-pyrazol-4-yl)pyrimidin-4-yl)amino)-4-(isopropylamino)-N-(3-(methylsulfonyl)prop yl)nicotinamide | ¹H NMR (400 MHz, Chloroform-*d*) δ 10.23 (s, 1H), 8.67 (d, *J* = 7.2 Hz, 1H), 8.62 (d, *J* = 0.6 Hz, 1H), 8.44 (d, *J* = 0.6 Hz, 1H), 8.41 (d, *J* = 5.8 Hz, 1H), 8.18 (s, 1H), 7.79 (s, 1H), 6.93 (d, *J* = 5.8 Hz, 1H), 6.62 (t, *J* = 5.8 Hz, 1H), 3.92-3.81 (m, 1H), 3.60 (q, *J* = 6.4 Hz, 2H), 3.16 (t, *J* = 7.3 Hz, 2H), 2.97 (s, 3H), 2.83 (tt, *J* = 7.9, 4.7 Hz, 1H), 2.28-2.15 (m, 2H), 1.57-1.49 (m, 2H), 1.40 (d, *J* = 6.4 Hz, 6H), 1.26-1.20 (m, 2H); 563.22 [M+H]⁺ |
| 65 | 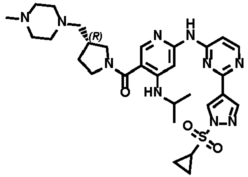 | (R)-(6-((2-(1-(cyclopropylsulfonyl )-1H-pyrazol-4-yl)pyrimidin-4-yl)amino)-4-(isopropylamino)pyri din-3-) (3-((4-methylpiperazin-1-yl)methyl)pyrrolidin -1-yl)methanone | ¹H NMR (400 MHz, Chloroform-*d*) δ 10.75 (s, 1H), 8.62 (s, 1H), 8.45 (s, 1H), 8.40 (d, *J* = 5.8 Hz, 1H), 7.89 (s, 1H), 7.86 (s, 1H), 7.38 (d, *J* = 7.2 Hz, 1H), 6.94 (d, *J* = 5.8 Hz, 1H), 4.20 (s, 4H), 3.85 (h, *J* = 6.5 Hz, 1H), 3.80-3.55 (m, 4H), 3.35 (dd, *J* = 11.5, 7.3 Hz, 1H), 2.83 (tt, *J* = 8.0, 4.7 Hz, 1H), 2.59 (s, 4H), 2.50-2.39 (m, 3H), 2.36 (s, 3H), 1.68 (dq, *J* = 12.6, 8.3 Hz, 1H), 1.55-1.50 (m, 2H), 1.38 (dd, *J* = 6.3, 2.3 Hz, 6H), 1.26-1.19 (m, 2H); 609.33 [M+H]⁺ |
| 66 | 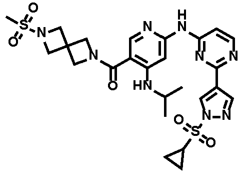 | (6-((2-(1-(cyclopropylsulfonyl )-1H-pyrazol-4-yl)pyrimidin-4-yl)amino)-4-(isopropylamino)pyri din-3-yl) (6-(methylsulfonyl)-2,6-diazaspiro[3.3]hepta n-2-yl)methanone | ¹H NMR (400 MHz, Chloroform-*d*) δ 9.79 (s, 1H), 8.62 (s, 1H), 8.44 (s, 1H), 8.41 (d, *J* = 5.8 Hz, 1H), 8.24 (d, *J* = 7.3 Hz, 1H), 7.91 (s, 1H), 7.72 (s, 1H), 6.95 (d, *J* = 5.8 Hz, 1H), 4.41 (s, 4H), 4.11 (s, 4H), 3.87-3.80 (m, 1H), 2.88 (s, 3H), 2.83 (tt, *J* = 8.0, 4.7 Hz, 1H), 1.55-1.50 (m, 2H), 1.38 (d, *J* = 6.4 Hz, 6H), 1.26-1.19 (m, 2H); 602.27 [M+H]⁺ |
| 67 | 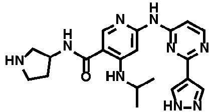 | 6-((2-(1H-pyrazol-4-yl)pyrimidin-4-yl)amino)-4-(isopropylamino)-N-(pyrrolidin-3-yl)nicotinamide | 408.26 [M+H]⁺ |
| 68 | 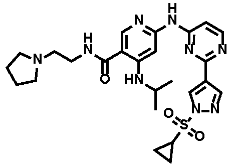 | 6-((2-(1-(cyclopropylsulfonyl )-1H-pyrazol-4-yl)pyrimidin-4-yl)amino)-4-(isopropylamino)-N-(2-(pyrrolidin-1-yl)ethyl)nicotinamid e | 540.3 [M+H]⁺ |
| 69 | 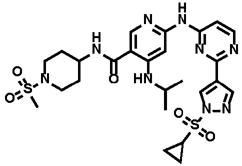 | 6-((2-(1-(cyclopropylsulfonyl )-1H-pyrazol-4-yl)pyrimidin-4-yl)amino)-4-(isopropylamino)-N-(1-(methylsulfonyl) pipe ridin-4-yl)nicotinamide | ¹H NMR (400 MHz, DMSO) δ 10.19 (s, 1H), 8.63 (s, 1H), 8.50 (d, *J* = 6.9 Hz, 1H), 8.48-8.40 (m, 3H), 8.26 (d, *J* = 7.4 Hz, 1H), 7.51 (s, 1H), 7.33 (s, 1H), 3.88 (s, 1H), 3.77 (dd, *J* = 13.2, 6.5 Hz, 1H), 3.57 (d, *J* = 11.9 Hz, 2H), 3.27 (dd, *J* = 7.7, 4.6 Hz, 1H), 2.89 (s, 3H), 2.82 (d, *J* = 9.7 Hz, 2H), 1.90 (d, *J* = 9.7 Hz, 2H), 1.63-1.54 (m, 2H), 1.37-1.31 (m, 2H), 1.27 (d, *J* = 6.3 Hz, 6H), 1.24-1.22 (m, 2H); 604.3 [M+H]⁺ |
| 70 | 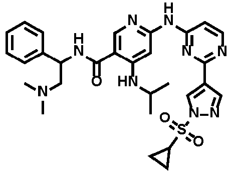 | 6-((2-(1-(cyclopropylsulfonyl )-1H-pyrazol-4-yl)pyrimidin-4-yl)amino)-N-(2-(dimethylamino)-1-phenylethyl)-4-(isopropylamino)nicotinamide | ¹H NMR (400 MHz, Chloroform-*d*) δ 8.63 (s, 1H), 8.48-8.40 (m, 3H), 8.35 (d, *J* = 7.1 Hz, 1H), 7.36 (d, *J* = 4.3 Hz, 4H), 7.28 (d, *J* = 4.2 Hz, 1H), 7.26-7.23 (m, 1H), 7.18 (d, *J* = 6.0 Hz, 2H), 4.98-4.86 (m, 1H), 3.75 (dd, *J* = 13.1, 6.6 Hz, 1H), 2.89-2.77 (m, 1H), 2.68 (t, *J* = 11.6 Hz, 1H), 2.49 (dd, *J* = 12.6, 4.9 Hz, 1H), 2.30 (s, 6H), 1.55-1.49 (m, 2H), 1.30 (t, *J* = 6.5 Hz, 6H), 1.22 (dd, *J* = 8.0, 1.8 Hz, 2H); 590.3 [M+H]⁺ |
| 71 | 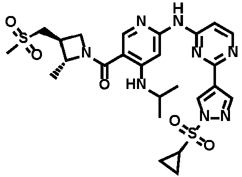 | (6-((2-(1-(cyclopropylsulfonyl )-1H-pyrazol-4-yl)pyrimidin-4-yl)amino)-4-(isopropylamino)pyri din-3-yl) ((2R,3S)-2-methyl-3-((methylsulfonyl)met hyl)azetidin-1-yl)methanone | ¹H NMR (400 MHz, Chloroform-*d*) δ 10.24 (s, 1H), 8.62 (s, 1H), 8.44 (s, 1H), 8.41 (d, *J* = 5.8 Hz, 1H), 8.17 (d, *J* = 7.1 Hz, 1H), 7.92 (s, 1H), 7.81 (s, 1H), 6.93 (d, *J* = 5.8 Hz, 1H), 4.64 (t, *J* = 9.0 Hz, 1H), 4.52 (p, *J* = 6.2 Hz, 1H), 4.01 (dd, *J* = 9.6, 5.9 Hz, 1H), 3.86 (h, *J* = 6.5 Hz, 1H), 3.41-3.27 (m, 2H), 2.96 (s, 3H), 2.89-2.80 (m, 2H), 1.58-1.50 (m, 5H), 1.39 (dd, *J* = 6.4, 4.3 Hz, 6H), 1.26-1.19 (m, 2H); 589.21 [M+H]⁺ |
| 72 | 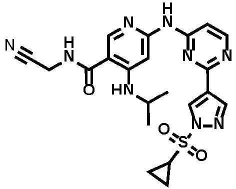 | N-(cyanomethyl)-6-((2-(1-(cyclopropylsulfonyl )-1H-pyrazol-4-yl)pyrimidin-4-yl)amino)-4-(isopropylamino)nico tinamide | ¹H NMR (400 MHz, Chloroform-*d*) δ 9.62 (s, 1H), 8.61 (d, *J* = 0.6 Hz, 1H), 8.46 (d, *J* = 7.3 Hz, 1H), 8.43 (s, 1H), 8.42 (d, *J* = 5.8 Hz, 1H), 8.18 (s, 1H), 7.73 (s, 1H), 6.92 (d, *J* = 5.8 Hz, 1H), 6.66 (t, *J* = 5.7 Hz, 1H), 4.31 (d, *J* = 5.6 Hz, 2H), 3.92-3.81 (m, 1H), 2.84 (tt, *J* = 8.0, 4.7 Hz, 1H), 1.56-1.50 (m, 2H), 1.40 (d, *J* = 6.4 Hz, 6H), 1.26-1.19 (m, 2H); 482.12 [M+H]⁺ |
| 73 | 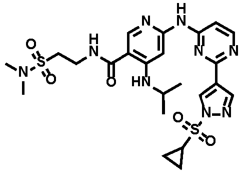 | 6-((2- (1-(cyclopropylsulfonyl )-1H-pyrazol-4-yl)pyrimidin-4-yl)amino)-N-(2-(N,N-dimethylsulfamoyl)et hyl)-4-(isopropylamino)nico tinamide | ¹H NMR (400 MHz, Chloroform-*d*) δ 8.91 (s, 1H), 8.63 (s, 1H), 8.45 (s, 1H), 8.43-8.38 (m, 2H), 8.16 (s, 1H), 7.53 (s, 1H), 7.01 (d, *J* = 5.8 Hz, 1H), 6.86 (t, *J* = 5.7 Hz, 1H), 3.91 (q, *J* = 5.8 Hz, 2H), 3.87-3.80 (m, 1H), 3.20-3.14 (m, 2H), 2.92 (s, 6H), 2.86-2.80 (m, 1H), 1.55-1.50 (m, 2H), 1.38 (d, *J* = 6.3 Hz, 6H), 1.25-1.19 (m, 2H); 578.18 [M+H]⁺ |
| 74 | 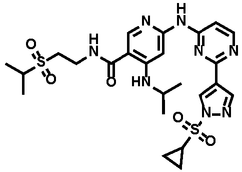 | 6-((2-(1-(cyclopropylsulfonyl )-1H-pyrazol-4-yl)pyrimidin-4-yl)amino)-4-(isopropylamino)-N-(2-(isopropylsulfonyl)e thyl)nicotinamide | ¹H NMR (400 MHz, Chloroform-*d*) δ 10.41 (s, 1H), 8.76 (d, *J* = 6.2 Hz, 1H), 8.56 (s, 1H), 8.43 (d, *J* = 5.8 Hz, 1H), 8.40 (s, 1H), 8.13 (s, 1H), 7.77 (s, 1H), 7.15-7.09 (m, 1H), 7.01 (d, *J* = 5.8 Hz, 1H), 3.98 (q, *J* = 5.8 Hz, 2H), 3.90-3.79 (m, 1H), 3.30-3.15 (m, 3H), 2.85 (tt, *J* = 7.9, 4.7 Hz, 1H), 1.57-1.51 (m, 2H), 1.43 (d, *J* = 6.9 Hz, 6H), 1.40 (d, *J* = 6.4 Hz, 6H), 1.27-1.21 (m, 2H); 577.21 [M+H]⁺ |
| 75 | 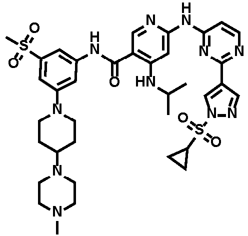 | 6-((2-(1-(cyclopropylsulfonyl )-1H-pyrazol-4-yl)pyrimidin-4-yl)amino)-4-(isopropylamino)-N-(3-(4-(4-methylpiperazin-1-yl)piperidin-1-yl)-5-(methylsulfonyl)phen yl)nicotinamide | 778.34 [M+H]⁺ |
| 76 | 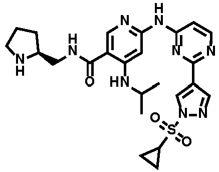 | (S)-6-((2-(1-(cyclopropylsulfonyl )-1H-pyrazol-4-yl)pyrimidin-4-yl)amino)-4-(isopropylamino)-N-(pyrrolidin-2-ylmethyl)nicotinamid e | ¹H NMR (400 MHz, Chloroform-*d*) δ 8.62 (d, *J* = 16.3 Hz, 1H), 8.41 (t, *J* = 12.1 Hz, 3H), 8.27 (s, 1H), 7.25 (s, 1H), 7.00 (d, *J* = 5.5 Hz, 1H), 3.75 (dd, *J* = 12.5, 6.1 Hz, 1H), 3.57 (d, *J* = 43.3 Hz, 2H), 3.29-3.15 (m, 1H), 3.03 (s, 2H), 2.84 (td, *J* = 7.9, 4.6 Hz, 1H), 1.90-1.77 (m, 4H), 1.53 (dd, *J* = 4.3, 2.5 Hz, 2H), 1.34 (t, *J* = 5.9 Hz, 6H), 1.22 (dd, *J* = 8.0, 1.7 Hz, 2H); 526.2 [M+H]⁺ |
| 77 | 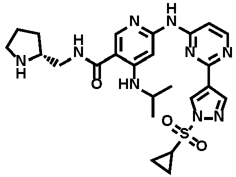 | (R)-6-((2-(1-(cyclopropylsulfonyl )-1H-pyrazol-4-yl)pyrimidin-4-yl)amino)-4-(isopropylamino)-N-(pyrrolidin-2-ylmethyl)nicotinamid e | ¹H NMR (400 MHz, Chloroform-*d*) δ 8.62 (d, *J* = 15.2 Hz, 1H), 8.45-8.35 (m, 3H), 8.27 (s, 1H), 7.24 (s, 1H), 7.01 (d, *J* = 9.1 Hz, 1H), 3.75 (d, *J* = 5.9 Hz, 1H), 3.56 (d, *J* = 49.5 Hz, 2H), 3.28-3.15 (m, 1H), 3.03 (s, 2H), 2.83 (dd, *J* = 8.2, 3.9 Hz, 1H), 1.87 (d, *J* = 7.0 Hz, 2H), 1.72 (dd, *J* = 12.1, 5.0 Hz, 2H), 1.53 (dd, *J* = 4.3, 2.6 Hz, 2H), 1.36-1.32 (m, 6H), 1.22 (dd, *J* = 8.0, 1.8 Hz, 2H); 526.3 [M+H]⁺ |
| 78 | 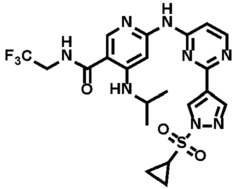 | 6-((2-(1-(cyclopropylsulfonyl )-1H-pyrazol-4-yl)pyrimidin-4-yl)amino)-4-(isopropylamino)-N-(2,2,2-trifluoroethyl)nicot inamide | ¹H NMR (400 MHz, Chloroform-*d*) δ 8.62 (s, 1H), 8.43 (s, 1H), 8.41 (d, *J* = 5.8 Hz, 1H), 8.30-8.25 (m, 2H), 8.05 (s, 1H), 7.34 (s, 1H), 7.03 (d, *J* = 5.8 Hz, 1H), 6.47 (s, 1H), 4.09 (qd, *J* = 9.0, 6.3 Hz, 2H), 3.80 (dq, *J* = 10.4, 5.2, 3.9 Hz, 1H), 2.84 (tt, *J* = 8.0, 4.7 Hz, 1H), 1.53 (qd, *J* = 6.1, 1.4 Hz, 2H), 1.36 (d, *J* = 6.3 Hz, 6H), 1.24-1.19 (m, 2H); 525.1 [M+H]⁺ |
| 79 | 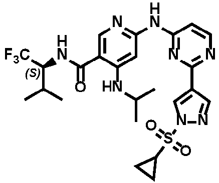 | (S)-6-((2-(1-(cyclopropylsulfonyl )-1H-pyrazol-4-yl)pyrimidin-4-yl)amino)-4-(isopropylamino)-N-(1,1,1-trifluoro-3-methylbutan-2-yl)nicotinamide | ¹H NMR (400 MHz, Chloroform-*d*) δ 8.57 (s, 1H), 8.53 (s, 1H), 8.40 (s, 1H), 8.37 (d, *J* = 5.9 Hz, 1H), 8.28 (s, 1H), 8.19 (d, *J* = 7.2 Hz, 1H), 7.39 (s, 1H), 6.96 (d, *J* = 5.9 Hz, 1H), 6.31 (d, *J* = 10.1 Hz, 1H), 4.80-4.66 (m, 1H), 3.77 (h, *J* = 6.6 Hz, 1H), 2.83 (tt, *J* = 8.4, 4.7 Hz, 1H), 2.27 (dq, *J* = 13.4, 6.9 Hz, 1H), 1.51 (dt, *J* = 7.7, 5.0 Hz, 2H), 1.35 (dd, *J* = 6.4, 3.2 Hz, 6H), 1.20 (d, *J* = 7.5 Hz, 2H), 1.08 (t, *J* = 7.1 Hz, 6H); 567.2 [M+H]⁺ |
| 80 | 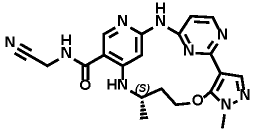 | (S)-N-(cyanomethyl)-1¹,6-dimethyl-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.89 (s, 1H), 8.45 (d, *J* = 7.3 Hz, 1H), 8.36 (d, *J* = 5.7 Hz, 1H), 8.35 (s, 1H), 8.13 (s, 1H), 6.51 (d, *J* = 5.8 Hz, 1H), 6.43-6.37 (m, 1H), 4.68 (td, *J* = 10.0, 3.2 Hz, 1H), 4.31 (dd, *J* = 9.2, 5.7 Hz, 2H), 4.28-4.23 (m, 1H), 4.12-4.05 (m, 1H), 3.80 (s, 3H), 2.20-2.13 (m, 2H), 1.47 (d, *J* = 6.6 Hz, 3H); 420.17 [M+H]⁺ |
| 81 | 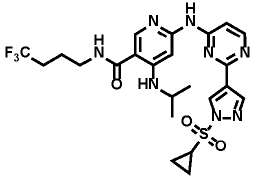 | 6-((2-(1-(cyclopropylsulfonyl )-1H-pyrazol-4-yl)pyrimidin-4-yl)amino)-4-(isopropylamino)-N-(4,4,4-trifluorobutyl)nicot inamide | ¹H NMR (400 MHz, Chloroform-*d*) δ 8.64 (s, 1H), 8.46-8.31 (m, 3H), 8.18 (s, 1H), 7.29 (s, 1H), 7.08 (d, *J* = 5.7 Hz, 1H), 6.15 (s, 1H), 3.80 (dd, *J* = 12.8, 6.5 Hz, 1H), 3.49 (dd, *J* = 13.3, 6.9 Hz, 2H), 2.88-2.79 (m, 1H), 2.28-2.11 (m, 2H), 1.91 (dt, *J* = 14.8, 7.4 Hz, 2H), 1.53 (dd, *J* = 4.7, 2.2 Hz, 2H), 1.37 (d, *J* = 6.3 Hz, 6H), 1.22 (dd, *J* = 7.9, 1.9 Hz, 2H); 553.2 [M+H]⁺ |
| 82 | 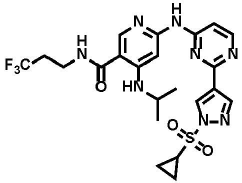 | 6-((2-(1-(cyclopropylsulfonyl )-1H-pyrazol-4-yl)pyrimidin-4-yl)amino)-4-(isopropylamino)-N-(3,3,3-trifluoropropyl)nico tinamide | ¹H NMR (400 MHz, Chloroform-*d*) δ 8.64 (s, 1H), 8.41 (dd, *J* = 25.8, 19.8 Hz, 3H), 8.18 (s, 1H), 7.28 (s, 1H), 7.08 (d, *J* = 5.7 Hz, 1H), 6.30 (s, 1H), 3.80 (dd, *J* = 13.1, 6.4 Hz, 1H), 3.72-3.63 (m, 2H), 2.89-2.78 (m, 1H), 2.54-2.41 (m, 2H), 1.54 (dd, *J* = 6.0, 3.8 Hz, 2H), 1.37 (d, *J* = 6.4 Hz, 6H), 1.22 (dt, *J* = 7.0, 3.5 Hz, 2H); 539.2 [M+H]⁺ |
| 83 | 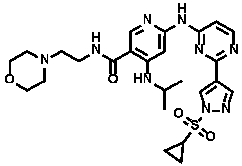 | 6-((2-(1-(cyclopropylsulfonyl )-1H-pyrazol-4-yl)pyrimidin-4-yl)amino)-4-(isopropylamino) -N-(2-morpholinoethyl)nico tinamide | ¹H NMR (400 MHz, Chloroform-*d*) δ 10.97 (s, 1H), 8.80 (d, *J* = 7.3 Hz, 1H), 8.61 (s, 1H), 8.44 (s, 1H), 8.41 (d, *J* = 5.8 Hz, 1H), 8.13 (s, 1H), 7.90 (s, 1H), 6.93 (d, *J* = 5.8 Hz, 2H), 3.94-3.82 (m, 1H), 3.76 (t, *J* = 4.7 Hz, 4H), 3.52 (q, *J* = 5.5 Hz, 2H), 2.84 (tt, *J* = 7.9, 4.7 Hz, 1H), 2.65 (t, *J* = 5.9 Hz, 2H), 2.56 (s, 4H), 1.58-1.49 (m, 2H), 1.40 (d, *J* = 6.4 Hz, 6H), 1.26-1.20 (m, 2H); 556.25 [M+H]⁺ |
| 84 | 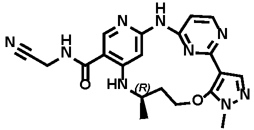 | (R)-N-(cyanomethyl)-1¹,6-dimethyl-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 9.52 (s, 1H), 8.50 (d, *J* = 7.3 Hz, 1H), 8.36 (d, *J* = 5.7 Hz, 1H), 8.35 (s, 1H), 8.15 (s, 1H), 8.13 (s, 1H), 6.71 (t, 1H), 6.55 (d, *J* = 5.7 Hz, 1H), 4.65 (td, *J* = 9.7, 2.9 Hz, 1H), 4.30 (dd, *J* = 9.0, 5.6 Hz, 2H), 4.27-4.20 (m, 1H), 4.07 (dt, *J* = 9.3, 4.6 Hz, 1H), 3.79 (s, 3H), 2.19-2.13 (m, 2H), 1.46 (d, *J* = 6.6 Hz, 3H); 420.13 [M+H]⁺ |
| 85 | 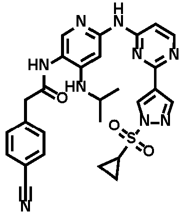 | 2-(4-cyanophenyl)-N-(6-((2-(1-(cyclopropylsulfonyl )-1H-pyrazol-4-yl)pyrimidin-4-yl)amino)-4-(isopropylamino)pyri din-3-yl)acetamide | ¹H NMR (400 MHz, DMSO) δ 9.96 (s, 1H), 9.33 (s, 1H), 8.61 (s, 1H), 8.44 (s, 1H), 8.39 (t, *J* = 5.9 Hz, 1H), 7.82 (dd, *J* = 8.4, 1.8 Hz, 3H), 7.57 (d, *J* = 8.3 Hz, 2H), 7.34 (d, *J* = 12.2 Hz, 2H), 5.39 (s, 1H), 3.81 (s, 2H), 3.71 (dq, *J* = 12.5, 6.2 Hz, 1H), 3.26 (td, *J* = 7.8, 3.9 Hz, 1H), 1.33 (dd, *J* = 10.5, 5.9 Hz, 2H), 1.26 (dd, *J* = 10.6, 4.5 Hz, 8H); 558.2 [M+H]⁺ |
| 86 | 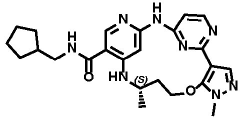 | (S) -N-(cyclopentylmethyl)-1¹,6-dimethyl-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.48 (d, *J* = 7.4 Hz, 1H), 8.34 (d, *J* = 5.7 Hz, 1H), 8.29 (s, 1H), 8.12 (d, *J* = 1.6 Hz, 2H), 7.66 (s, 1H), 6.38 (d, *J* = 5.7 Hz, 1H), 6.07 (s, 1H), 4.72 (td, *J* = 9.3, 4.1 Hz, 1H), 4.28-4.17 (m, 1H), 4.06 (dt, *J* = 9.2, 4.3 Hz, 1H), 3.79 (s, 3H), 3.40-3.30 (m, 2H), 2.21-2.08 (m, 2H), 1.86-1.78 (m, 3H), 1.70-1.63 (m, 2H), 1.62-1.55 (m, 2H), 1.44 (d, *J* = 6.7 Hz, 3H), 1.31-1.27 (m, 2H); 463.24 [M+H]⁺ |
| 87 | 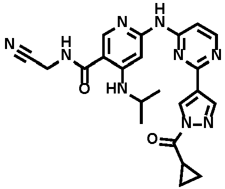 | N-(cyanomethyl)-6-((2-(1-(cyclopropanecarbony l)-1H-pyrazol-4-yl)pyrimidin-4-yl)amino)-4-(isopropylamino)nico tinamide | ¹H NMR (400 MHz, DMSO-*d*₆) δ 8.74 (d, *J* = 5.7 Hz, 1H), 8.58 (s, 1H), 8.16 (d, *J* = 2.6 Hz, 1H), 7.79 (d, *J* = 1.6 Hz, 1H), 7.71 (d, *J* = 5.7 Hz, 1H), 6.99 (s, 1H), 6.53 (dd, *J* = 2.7, 1.6 Hz, 1H), 4.30 (d, *J* = 5.3 Hz, 2H), 3.78 (dt, *J* = 13.1, 6.6 Hz, 1H), 3.32 (s, 7H), 1.62 (td, *J* = 7.8, 4.1 Hz, 1H), 1.18 (s, 2H), 1.16 (s, 2H), 1.13 (d, *J* = 6.3 Hz, 1H), 1.06-1.01 (m, 2H), 0.90-0.85 (m, 2H).; 446.2 [M+H]⁺ |
| 88 | 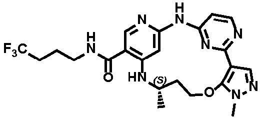 | (S)-1¹,6-dimethyl-N-(4,4,4-trifluorobutyl)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 9.32 (s, 1H), 8.58 (d, *J* = 7.4 Hz, 1H), 8.34 (d, *J* = 5.7 Hz, 1H), 8.33 (s, 1H), 8.12 (s, 1H), 8.09 (s, 1H), 6.52 (d, *J* = 5.7 Hz, 1H), 6.27 (t, *J* = 5.6 Hz, 1H), 4.68 (td, *J* = 9.6, 3.2 Hz, 1H), 4.27-4.19 (m, 1H), 4.07 (dt, *J* = 9.2, 4.4 Hz, 1H), 3.82-3.75 (m, 3H), 3.49 (q, *J* = 7.1, 6.7 Hz, 2H), 2.27-2.12 (m, 4H), 1.91 (p, *J* = 7.3 Hz, 2H), 1.45 (d, *J* = 6.7 Hz, 3H); 491.21 [M+H]⁺ |
| 89 | 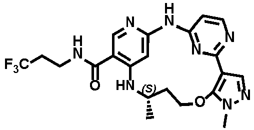 | (S)-1¹,6-dimethyl-N-(3,3,3-trifluoropropyl)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.40 (d, *J* = 7.4 Hz, 1H), 8.33 (d, *J* = 5.7 Hz, 1H), 8.29 (s, 1H), 8.14 (s, 1H), 8.12 (s, 1H), 7.71 (s, 1H), 6.36 (d, *J* = 5.7 Hz, 1H), 6.32 (t, *J* = 6.1 Hz, 1H), 4.72 (td, *J* = 9.4, 4.1 Hz, 1H), 4.24 (dt, *J* = 10.9, 6.7 Hz, 1H), 4.07 (dt, *J* = 9.2, 4.3 Hz, 1H), 3.79 (s, 3H), 3.68 (q, *J* = 6.3 Hz, 2H), 2.47 (qt, *J* = 10.8, 6.5 Hz, 2H), 2.18-2.11 (m, 2H), 1.45 (d, *J* = 6.6 Hz, 3H); 477.13 [M+H]⁺ |
| 90 | 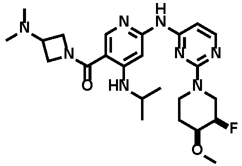 | (3-(dimethylamino)azeti din-1-yl) (6-((2-(cis-3-fluoro-4-methoxypiperidin-1-yl)pyrimidin-4-yl)amino)-4-(isopropylamino)pyri din-3-yl)methanone | ¹H NMR (400 MHz, Chloroform-*d*) δ 8.06 (d, *J* = 5.6 Hz, 1H), 8.02 (s, 1H), 7.93 (d, *J* = 7.5 Hz, 1H), 7.27 (s, 1H), 6.24 (d, *J* = 5.6 Hz, 1H), 4.85-4.68 (m, 1H), 4.44 (dt, *J* = 13.9, 7.0 Hz, 1H), 4.26 (s, 1H), 4.12 (q, *J* = 7.2 Hz, 4H), 3.85-3.55 (m, 5H), 3.09 (ddd, *J* = 12.4, 7.1, 5.2 Hz, 1H), 2.19 (s, 4H), 2.05 (s, 2H), 1.95 (d, *J* = 52.3 Hz, 4H), 1.30-1.27 (m, 6H); 487.3 [M+H]⁺ |
| 91 | 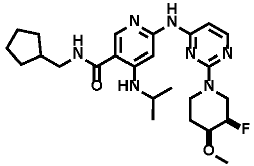 | N-(cyclopentylmethyl)-6-((2-(cis-3-fluoro-4-methoxypiperidin-1-yl)pyrimidin-4-yl)amino)-4-(isopropylamino)nico tinamide | ¹H NMR (400 MHz, Chloroform-*d*) δ 8.29 (d, *J* = 7.6 Hz, 1H), 8.13 (s, 1H), 8.05 (dd, *J* = 5.6, 1.4 Hz, 1H), 7.32 (s, 1H), 6.21 (dd, *J* = 5.7, 1.8 Hz, 1H), 6.09 (d, *J* = 5.9 Hz, 1H), 4.77 (ddd, *J* = 48.1, 6.5, 3.1 Hz, 1H), 4.44 (dt, *J* = 13.9, 7.1 Hz, 1H), 4.21-4.10 (m, 1H), 3.77 (dtd, *J* = 26.9, 13.4, 4.7 Hz, 2H), 3.67-3.53 (m, 1H), 3.49 (s, 3H), 3.33 (dd, *J* = 7.3, 5.6 Hz, 2H), 2.15 (hept, *J* = 7.6 Hz, 1H), 1.99 (dtt, *J* = 11.9, 7.8, 3.5 Hz, 1H), 1.80 (ddd, *J* = 12.5, 7.3, 3.9 Hz, 3H), 1.72-1.50 (m, 4H), 1.29 (dd, *J* = 6.3, 2.0 Hz, 6H), 1.27-1.24 (m, 2H); 486.3 [M+H]⁺ |
| 92 | 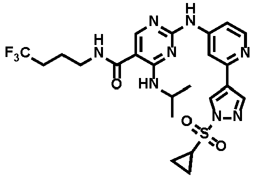 | 2-((2-(1-(cyclopropylsulfonyl )-1H-pyrazol-4-yl)pyridin-4-yl)amino)-4-(isopropylamino)-N-(4,4,4-trifluorobutyl)pyrim idin-5-carboxamide | ¹H NMR (400 MHz, Chloroform-*d*) δ 9.43 (s, 1H), 8.97 (d, *J* = 7.5 Hz, 1H), 8.47 (s, 1H), 8.45 (d, *J* = 5.7 Hz, 1H), 8.31 (d, *J* = 0.7 Hz, 1H), 8.16 (d, *J* = 2.0 Hz, 1H), 8.14 (s, 1H), 7.40 (dd, *J* = 5.7, 2.1 Hz, 1H), 6.12 (t, *J* = 5.8 Hz, 1H), 4.47-4.36 (m, 1H), 3.48 (q, *J* = 6.8 Hz, 2H), 2.82 (tt, *J* = 8.0, 4.7 Hz, 1H), 2.27-2.15 (m, 2H), 1.90 (p, *J* = 7.3 Hz, 2H), 1.55-1.49 (m, 2H), 1.38 (d, *J* = 6.5 Hz, 6H), 1.24-1.17 (m, 2H); 553.20 [M+H]⁺ |
| 93 | 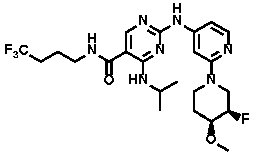 | 2-((2-((3R,4S)-3-fluoro-4-methoxypiperidin-1-yl)pyridin-4-yl)amino)-4-(isopropylamino)-N-(4,4,4-trifluorobutyl)pyrim idine-5-carboxamide | ¹H NMR (400 MHz, Chloroform-*d*) δ 8.77 (d, *J* = 7.7 Hz, 1H), 8.16 (s, 1H), 8.02 (d, *J* = 5.6 Hz, 1H), 7.64 (s, 1H), 7.42 (d, *J* = 1.4 Hz, 1H), 6.68 (dd, *J* = 5.6, 1.7 Hz, 1H), 6.00 (t, *J* = 5.3 Hz, 1H), 4.89-4.71 (m, 1H), 4.45-4.34 (m, 1H), 4.17-4.05 (m, 1H), 3.79 (ddd, *J* = 11.9, 7.3, 3.7 Hz, 1H), 3.66-3.43 (m, 7H), 2.25-2.12 (m, 2H), 2.07-1.98 (m, 1H), 1.93-1.79 (m, 4H), 1.31 (d, *J* = 6.5 Hz, 6H); 514.20 [M+H]⁺ |
| 94 | 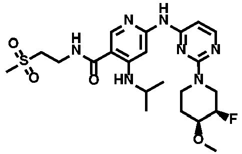 | 6-((2-((3R,4S)-3-fluoro-4-methoxypiperidin-1-yl)pyrimidin-4-yl)amino)-4-(isopropylamino)-N-(2-(methylsulfonyl)ethy l)nicotinamide | ¹H NMR (400 MHz, Chloroform-*d*) δ 8.27 (d, *J* = 7.6 Hz, 1H), 8.18 (s, 1H), 8.06 (d, *J* = 5.6 Hz, 1H), 7.41 (s, 1H), 7.35 (s, 1H), 6.83 (t, *J* = 5.9 Hz, 1H), 6.20 (d, *J* = 5.6 Hz, 1H), 4.77 (ddt, 1H), 4.42 (dt, *J* = 14.0, 7.1 Hz, 1H), 4.18-4.09 (m, 1H), 3.98-3.90 (m, 2H), 3.88-3.71 (m, 2H), 3.67-3.55 (m, 2H), 3.50 (s, 3H), 3.37-3.31 (m, 2H), 3.00 (s, 3H), 2.06-1.95 (m, 1H), 1.84-1.75 (m, 1H), 1.30 (dd, *J* = 6.3, 2.0 Hz, 6H); 510.18 [M+H]⁺ |
| 95 | 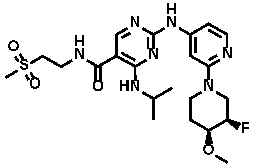 | 2-((2-((3R,4S)-3-fluoro-4-methoxypiperidin-1-yl)pyridin-4-yl)amino)-4-(isopropylamino)-N-(2-(methylsulfonyl)ethy l)pyrimidin-5-carboxamide | ¹H NMR (400 MHz, Chloroform-*d*) δ 8.88-8.81 (m, 2H), 8.15 (s, 1H), 8.03 (d, *J* = 5.7 Hz, 1H), 7.42 (s, 1H), 6.86 (t, *J* = 5.2 Hz, 1H), 6.77 (dd, *J* = 5.7, 1.4 Hz, 1H), 4.80 (ddt, *J* = 48.2, 6.3, 2.4 Hz, 1H), 4.45-4.35 (m, 1H), 4.10 (dt, *J* = 13.8, 7.1 Hz, 1H), 3.93 (q, *J* = 5.8 Hz, 2H), 3.78 (ddd, *J* = 11.7, 7.3, 3.3 Hz, 1H), 3.64-3.51 (m, 2H), 3.49 (s, 3H), 3.43 (dd, *J* = 16.0, 7.1 Hz, 1H), 3.35-3.29 (m, 2H), 3.01 (s, 3H), 2.08-1.98 (m, 1H), 1.87-1.79 (m, 1H), 1.32 (d, *J* = 6.5 Hz, 6H); 510.23 [M+H]⁺ |
| 96 | 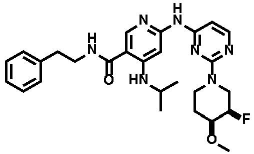 | 6-((2-(cis-3-fluoro-4-methoxypiperidin-1-yl)pyrimidin-4-yl)amino)-4-(isopropylamino)-N-phenethylnicotinamid e | ¹H NMR (400 MHz, Chloroform-*d*) δ 8.28 (d, *J* = 7.6 Hz, 1H), 8.03 (d, *J* = 5.6 Hz, 1H), 7.98 (s, 1H), 7.78 (s, 1H), 7.33 (dd, *J* = 5.1, 2.6 Hz, 2H), 7.25-7.20 (m, 3H), 6.17 (d, *J* = 5.6 Hz, 1H), 6.06 (t, *J* = 5.8 Hz, 1H), 4.77 (ddt, *J* = 48.0, 6.0, 2.7 Hz, 1H), 4.43 (dt, *J* = 13.9, 7.1 Hz, 1H), 4.15 (ddd, *J* = 11.1, 6.8, 3.7 Hz, 1H), 3.86-3.71 (m, 2H), 3.69-3.54 (m, 5H), 3.49 (s, 3H), 2.91 (t, *J* = 6.9 Hz, 2H), 2.02-1.74 (m, 2H), 1.29 (dd, *J* = 6.4, 2.1 Hz, 6H); 508.3 [M+H]⁺ |
| 97 | 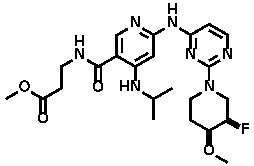 | methyl 3-(6-((2-(cis-3-fluoro-4-methoxypiperidin-1-yl)pyrimidin-4-yl)amino)-4-(isopropylamino)nico tinamido)propanoate | ¹H NMR (400 MHz, Chloroform-*d*) δ 8.16 (s, 1H), 8.05 (d, *J* = 5.6 Hz, 1H), 7.80 (s, 1H), 7.35 (s, 1H), 6.79 (t, *J* = 6.0 Hz, 1H), 6.19 (d, *J* = 5.6 Hz, 1H), 4.77 (ddt, *J* = 48.1, 6.2, 2.7 Hz, 1H), 4.44 (dt, *J* = 14.0, 7.1 Hz, 1H), 4.20-4.09 (m, 1H), 3.89-3.76 (m, 1H), 3.72 (s, 4H), 3.70-3.54 (m, 4H), 3.50 (s, 3H), 2.65 (t, *J* = 5.9 Hz, 2H), 2.05-1.74 (m, 2H), 1.29 (dd, *J* = 6.3, 2.1 Hz, 6H); 490.2 [M+H]⁺ |
| 98 | 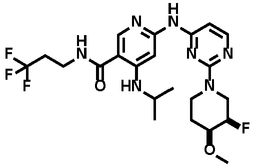 | 6-((2-(cis-3-fluoro-4-methoxypiperidin-1-yl)pyrimidin-4-yl)amino)-4-(isopropylamino)-N-(3,3,3-trifluoropropyl)nico tinamide | ¹H NMR (400 MHz, Chloroform-*d*) δ 8.28 (d, *J* = 7.6 Hz, 1H), 8.19-8.10 (m, 1H), 8.05 (d, *J* = 5.6 Hz, 1H), 7.38 (s, 1H), 6.37 (t, *J* = 5.9 Hz, 1H), 6.19 (d, *J* = 5.6 Hz, 1H), 4.77 (ddd, *J* = 48.0, 6.5, 3.1 Hz, 1H), 4.43 (dt, *J* = 14.0, 7.1 Hz, 1H), 4.14 (ddd, *J* = 13.0, 6.8, 3.7 Hz, 1H), 3.85-3.71 (m, 2H), 3.69-3.58 (m, 4H), 3.50 (s, 3H), 2.80 (s, 1H), 2.46 (qt, *J* = 10.8, 6.5 Hz, 2H), 2.06-1.92 (m, 1H), 1.79 (ddt, *J* = 13.9, 7.3, 3.7 Hz, 1H), 1.30 (dd, *J* = 6.3, 2.3 Hz, 6H); 500.2 [M+H]⁺ |
| 99 | 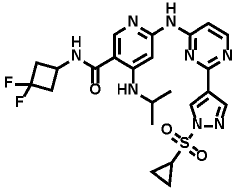 | 6-((2-(1-(cyclopropylsulfonyl )-1H-pyrazol-4-yl)pyrimidin-4-yl)amino)-N-(3,3-difluorocyclobutyl)-4-(isopropylamino)nico tinamide | ¹H NMR (400 MHz, Chloroform-*d*) δ 8.59 (s, 1H), 8.41 (s, 1H), 8.38 (d, *J* = 5.8 Hz, 1H), 8.28 (d, *J* = 7.3 Hz, 1H), 8.21 (s, 1H), 8.13 (s, 1H), 7.33 (s, 1H), 6.97 (d, *J* = 5.8 Hz, 1H), 6.45 (s, 1H), 4.45-4.32 (m, 1H), 3.77 (dq, *J* = 12.9, 6.4 Hz, 1H), 3.09 (dqd, *J* = 15.3, 8.0, 3.6 Hz, 2H), 2.83 (tt, *J* = 7.9, 4.7 Hz, 1H), 2.61 (qd, *J* = 14.2, 6.4 Hz, 2H), 1.55-1.48 (m, 2H), 1.35 (d, *J* = 6.3 Hz, 6H), 1.24-1.20 (m, 2H); 533.3 [M+H]⁺ |
| 100 | 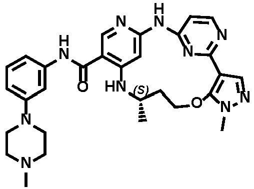 | (S)-1¹,6-dimethyl-N-(3-(4-methylpiperazin-1-yl)phenyl)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.35 (d, *J* = 5.7 Hz, 1H), 8.32 (d, *J* = 4.1 Hz, 2H), 8.27 (d, *J* = 7.4 Hz, 1H), 8.13 (s, 1H), 7.67 (s, 1H), 7.40 (s, 1H), 7.28-7.23 (m, 1H), 7.20-7.18 (m, 1H), 6.95 (dd, *J* = 7.7, 1.6 Hz, 1H), 6.75 (dd, *J* = 8.0, 2.4 Hz, 1H), 6.35 (d, *J* = 5.8 Hz, 1H), 4.73 (td, *J* = 9.6, 3.3 Hz, 1H), 4.30-4.23 (m, 1H), 4.11-4.05 (m, 1H), 3.80 (s, 3H), 3.26 (t, *J* = 5.1 Hz, 4H), 2.59 (t, 4H), 2.36 (s, 3H), 2.21-2.08 (m, 2H), 1.45 (d, *J* = 6.6 Hz, 3H); 555.42 [M+H]⁺ |
| 101 | 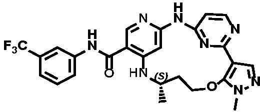 | (S)-1¹,6-dimethyl-N-(3-(trifluoromethyl)phe nyl)-1¹H-9-oxa-3,5-diaza-2(2,4)-pyrimidina-4(2,4)-pyridina-1(4,5)-pyrazolacyclononaphane-4⁵-carboxamide | ¹H NMR (400 MHz, Chloroform-*d*) δ 8.35 (d, *J* = 5.7 Hz, 1H), 8.32 (d, *J* = 4.1 Hz, 2H), 8.27 (d, *J* = 7.4 Hz, 1H), 8.13 (s, 1H), 7.67 (s, 1H), 7.40 (s, 1H), 7.28-7.23 (m, 1H), 7.20-7.18 (m, 1H), 6.95 (dd, *J* = 7.7, 1.6 Hz, 1H), 6.75 (dd, *J* = 8.0, 2.4 Hz, 1H), 6.35 (d, *J* = 5.8 Hz, 1H), 4.73 (td, *J* = 9.6, 3.3 Hz, 1H), 4.30-4.23 (m, 1H), 4.11-4.05 (m, 1H), 3.80 (s, 3H), 3.26 (t, *J* = 5.1 Hz, 4H), 2.59 (t, 4H), 2.36 (s, 3H), 2.21-2.08 (m, 2H), 1.45 (d, *J* = 6.6 Hz, 3H); 525.28 [M+H]⁺ |
| 102 | 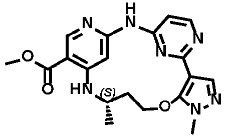 | methyl (S)-1¹,6-dimethyl-1¹H-9-oxa-3,5-diaza-2(2,4)-pyrimidina-4(2,4)-pyridina-1(4,5)-pyrazolacyclononapha ne-4⁵-carboxylate | ¹H NMR (400 MHz, Chloroform-*d*) δ 8.84 (s, 1H), 8.58 (s, 1H), 8.38-8.31 (m, 2H), 8.27 (d, *J* = 7.5 Hz, 1H), 8.13 (s, 1H), 6.50 (d, *J* = 5.7 Hz, 1H), 4.73 (td, *J* = 9.2, 4.5 Hz, 1H), 4.29 (dt, *J* = 11.7, 6.5 Hz, 1H), 4.08 (dt, *J* = 9.8, 5.2 Hz, 1H), 3.87 (s, 3H), 3.80 (s, 3H), 2.19-2.13 (m, 1H), 1.47 (d, *J* = 6.7 Hz, 3H); 396 [M+H]⁺ |
| 103 | 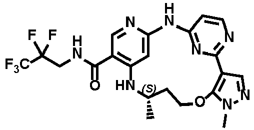 | (S)-1¹,6-dimethyl-N-(2,2,3,3,3-pentafluoropropyl)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.35 (d, *J* = 5.7 Hz, 1H), 8.33-8.27 (m, 2H), 8.19 (s, 1H), 8.13 (s, 1H), 7.79 (s, 1H), 6.41 (d, *J* = 5.7 Hz, 1H), 6.33 (s, 1H), 4.71 (td, *J* = 9.4, 3.5 Hz, 1H), 4.28-4.22 (m, 1H), 4.21-4.04 (m, 3H), 3.80 (s, 3H), 2.15-2.11 (m, 2H), 1.45 (d, *J* = 6.7 Hz, 3H); 513.17 [M+H]⁺ |
| 104 | 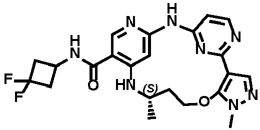 | (S)-N-(3,3-difluorocyclobutyl)-1¹,6-dimethyl-1¹H-9-oxa-3,5-diaza-2(2,4)-pyrimidina-4(2,4)-pyridina-1(4,5)-pyrazolacyclononapha ne-4⁵-carboxamide | 471.3[M+H]⁺ |
| 105 | 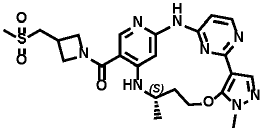 | (S)-(1¹,6-dimethyl-1¹H-9-oxa-3,5-diaza-2(2,4)-pyrimidina-4(2,4)-pyridina-1(4,5)-pyrazolacyclononapha ne-4⁵-yl)(3-((methylsulfonyl)met hyl)azetidin-1-yl)methanone | ¹H NMR (400 MHz, Chloroform-*d*) δ 10.12 (s, 1H), 8.41-8.29 (m, 3H), 8.13 (s, 1H), 7.87 (s, 1H), 6.62 (d, *J* = 5.7 Hz, 1H), 4.70 (td, *J* = 9.7, 3.0 Hz, 1H), 4.60-4.50 (m, 2H), 4.28-4.14 (m, 3H), 4.08 (dt, *J* = 9.2, 4.4 Hz, 1H), 3.80 (s, 3H), 3.39 (d, *J* = 6.7 Hz, 2H), 3.37-3.29 (m, 1H), 2.96 (s, 3H), 2.14-2.12 (m, 2H), 1.45 (d, *J* = 6.6 Hz, 3H); 513.17 [M+H]⁺ |
| 106 | 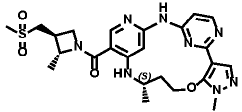 | ((S)-1¹,6-dimethyl-1¹H-9-oxa-3,5-diaza-2(2,4)-pyrimidina-4(2,4)-pyridina-1(4,5)-pyrazolacyclononapha ne-4⁵-yl)((2R,3S)-2-methyl-3-((methylsulfonyl)met hyl)azetidin-1-yl)methanone | 527.3[M+H]⁺ |
| 107 | 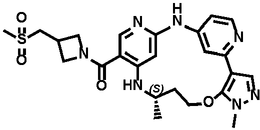 | (S)-(1¹,6-dimethyl-1¹H-9-oxa-3,5-diaza-2,4(2,4)-dipyridina-1(4,5)-pyrazolacyclononapha ne-4⁵-yl)(3-((methylsulfonyl)met hyl)azetidin-1-yl)methanone | 512.3[M+H]⁺ |
| 108 | 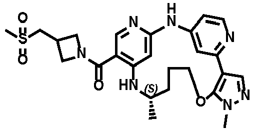 | (S)-(1¹,6-dimethyl-1¹H-10-oxa-3,5-diaza-2,4(2,4)-dipyridina-1(4,5)-pyrazolacyclodecapha ne-4⁵-yl)(3-((methylsulfonyl)met hyl)azetidin-1-yl)methanone | 526.3 [M+H]⁺ |
| 109 | 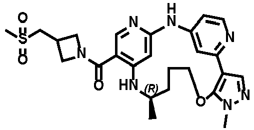 | (R)-(1¹,6-dimethyl-1¹H-10-oxa-3,5-diaza-2,4(2,4)-dipyridina-1(4,5)-pyrazolacyclodecapha ne-4⁵-yl)(3-((methylsulfonyl)met hyl)azetidin-1-yl)methanone | 526.3 [M+H]⁺ |
| 110 | 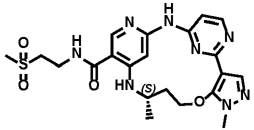 | (S)-1¹, 6-dimethyl-N-(2-(methylsulfonyl)ethy l)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 8.46 (d, *J* = 7.3 Hz, 1H), 8.38 (s, 1H), 8.34 (d, *J* = 5.7 Hz, 1H), 8.31 (s, 1H), 8.14 (s, 1H), 8.12 (s, 1H), 6.92 (t, *J* = 5.3 Hz, 1H), 6.45 (d, *J =* 5.7 Hz, 1H), 4.71 (td, *J* = 9.5, 3.5 Hz, 1H), 4.24 (dq, *J* = 10.6, 3.2 Hz, 1H), 4.08 (dt, *J* = 9.3, 4.3 Hz, 1H), 3.95 (q, *J* = 5.8 Hz, 2H), 3.80 (s, 3H), 3.38-3.32 (m, 2H), 3.01 (s, 3H), 2.15-2.13 (m, 2H), 1.45 (d, *J* = 6.6 Hz, 3H); 487.13 [M+H]⁺ |
| 111 | 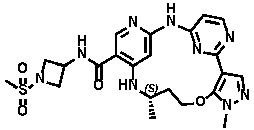 | (S)-1¹, 6-dimethyl-N-(1-(methylsulfonyl)azet idin-3-yl)-1¹H-9-oxa-3,5-diaza-2(2,4)-pyrimidina-4(2,4)-pyridina-1(4,5)-pyrazolacyclononapha ne-4⁵-carboxamide | 514.3 [M+H]⁺ |
| 112 | 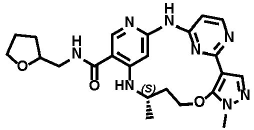 | (6S)-1¹,6-dimethyl-N-((tetrahydrofuran-2-yl)methyl)-1¹H-9-oxa-3,5-diaza-2(2,4)-pyrimidina-4(2,4)-pyridina-1(4,5)-pyrazolacyclononapha ne-4⁵-carboxamide | 465.3 [M+H]⁺ |
| 113 | 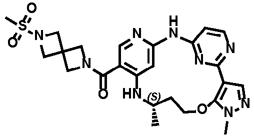 | (S)-(1¹,6-dimethyl-1¹H-9-oxa-3,5-diaza-2(2,4)-pyrimidina-4(2,4)-pyridina-1(4,5)-pyrazolacyclononapha ne-4⁵-yl)(6-(methylsulfonyl)-2,6-diazaspiro[3.3]hepta n-2-yl)methanone | 540.3 [M+H]⁺ |
| 114 | 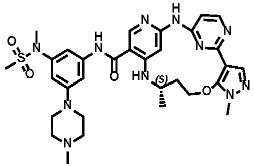 | (S)-1¹, 6-dimethyl-N-(3-(N-methylmethylsulfonam ido)-5-(4-methylpiperazin-1-yl)phenyl)-1¹H-9-oxa-3,5-diaza-2(2,4)-pyrimidina-4(2,4)-pyridina-1(4,5)-pyrazolacyclononapha ne-4⁵-carboxamide | 662.34 [M+H]⁺ |
| 115 | 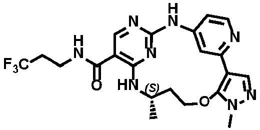 | (S)-1¹, 6-dimethyl-N-(3,3,3-trifluoropropyl)-1¹H-9-oxa-3,5-diaza-4(2,4)-pyrimidina-2(2,4)-pyridina-1(4,5)-pyrazolacyclononapha ne-4⁵-carboxamide | 477.3 [M+H]⁺ |
| 116 | 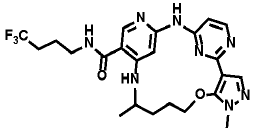 | 1¹,6-dimethyl-N-(4,4,4-trifluorobutyl)-1¹H-10-oxa-3,5-diaza-2(2,4)-pyrimidina-4(2,4)-pyridina-1(4,5)-pyrazolacyclodecapha ne-4⁵-carboxamide | 505.3 [M+H]⁺ |
| 117 | 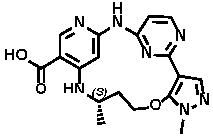 | (S)-1¹,6-dimethyl-1¹H-9-oxa-3,5-diaza-2(2,4)-pyrimidina-4(2,4)-pyridina-1(4,5)-pyrazolacyclononapha ne-4⁵-carboxylic acid | 382.3 [M+H]⁺ |
| 118 | 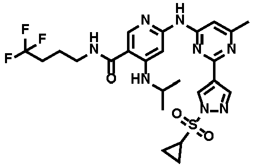 | 6-((2-(1-(cyclopropylsulfonyl )-1H-pyrazol-4-yl)-6-methylpyrimidin-4-yl)amino)-4-(isopropylamino)-N-(4,4,4-trifluorobutyl)nicot inamide | ¹H NMR (400 MHz, Chloroform-d) δ 10.16 (s, 1H), 8.67 (d, *J* = 7.1 Hz, 1H), 8.61 (s, 1H), 8.44 (s, 1H), 8.18 (s, 1H), 7.81 (s, 1H), 6.76 (s, 1H), 6.36 (d, *J* = 24.3 Hz, 1H), 3.86 (h, *J* = 6.6 Hz, 1H), 3.48 (q, *J* = 6.7 Hz, 2H), 2.83 (tt, *J* = 7.9, 4.7 Hz, 1H), 2.48 (s, 3H), 2.28-2.18 (m, 2H), 1.91 (p, *J* = 7.3 Hz, 2H), 1.52 (qd, *J* = 6.0, 1.3 Hz, 2H), 1.39 (dd, *J* = 6.4, 1.1 Hz, 6H), 1.21 (qd, *J* = 6.2, 1.3 Hz, 2H); 567.20 [M+H]⁺ |
| 119 | 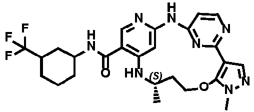 | (6S)-1¹,6-dimethyl-N-(3-(trifluoromethyl)cyc lohexyl)-1¹H-9-oxa-3,5-diaza-2(2,4)-pyrimidina-4(2,4)-pyridina-1(4,5)-pyrazolacyclononapha ne-4⁵-carboxamide | 531.3 [M+H]⁺ |
| 120 | 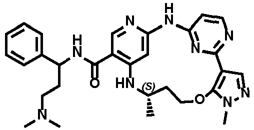 | (6S)-N-(3-(dimethylamino)-1-phenylpropyl)-1¹,6-dimethyl-1¹H-9-oxa-3,5-diaza-2(2,4)-pyrimidina-4(2,4)-pyridina-1(4,5)-pyrazolacyclononapha ne-4⁵-carboxamide | 542.3 [M+H]⁺ |
| 121 | 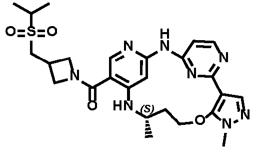 | (S)-(1¹,6-dimethyl-1¹H-9-oxa-3,5-diaza-2(2,4)-pyrimidina-4(2,4)-pyridina-1(4,5)-pyrazolacyclononapha ne-4⁵-yl)(3-((isopropylsulfonyl) methyl)azetidin-1-yl)methanone | 541.25 [M+H]⁺ |
| 122 | 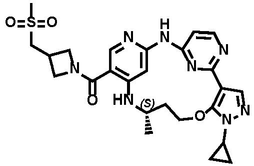 | (S)-(1¹-cyclopropyl-6-methyl-1¹H-9-oxa-3,5-diaza-2(2,4)-pyrimidina-4(2,4)-pyridina-1(4,5)-pyrazolacyclononapha ne-4⁵-yl)(3-((methylsulfonyl)met hyl)azetidin-1-yl)methanone | 539.3 [M+H]⁺ |
| 123 | 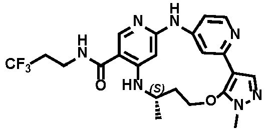 | (S)-1¹, 6-dimethyl-N-(3,3,3-trifluoropropyl)-1¹H-9-oxa-3,5-diaza-2,4(2,4)-dipyridina-1(4,5)-pyrazolacyclononapha ne-4⁵-carboxamide | ¹H NMR (400 MHz, DMSO-d6) δ 8.67 (s, 1H), 8.52-8.43 (m, 2H), 8.26 (d, *J* = 6.2 Hz, 1H), 8.03 (s, 1H), 7.84 (s, 1H), 6.98 (s, 1H), 6.72 (s, 1H), 4.49-4.43 (m, 1H), 4.10 (s, 1H), 3.83 (s, 3H), 3.46 (q, *J* = 6.6 Hz, 2H), 2.60-2.52 (m, 2H), 2.33-2.22 (m, 2H), 1.90-1.81 (m, 1H), 1.36 (d, *J =* 6.4 Hz, 3H); 476.22 [M+H]⁺ |
| 124 | 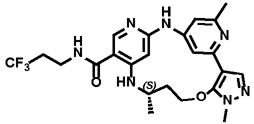 | (S)-1¹,2⁶,6-trimethyl-N-(3,3,3-trifluoropropyl)-1¹H-9-oxa-3,5-diaza-2,4(2,4)-dipyridina-1(4,5)-pyrazolacyclononapha ne-4⁵-carboxamide | ¹H NMR (400 MHz, Chloroform-*d*) δ 8.69 (s, 1H), 8.37 (d, *J* = 6.5 Hz, 1H), 8.13 (s, 1H), 8.04 (s, 1H), 7.57-7.53 (m, 1H), 6.60 (s, 1H), 6.56 (d, *J* = 2.0 Hz, 1H), 6.43-6.37 (m, 1H), 4.30-4.19 (m, 2H), 3.79 (s, 3H), 3.71-3.62 (m, 3H), 2.51 (s, 3H), 2.50-2.39 (m, 3H), 2.01-1.91 (m, 1H), 1.43 (d, *J* = 6.5 Hz, 3H); 490.20 [M+H]⁺ |
| 125 | 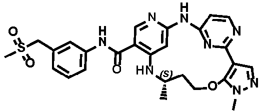 | (S)-1¹, 6-dimethyl-N-(3-((methylsulfonyl)met hyl)phenyl)-1¹H-9-oxa-3,5-diaza-2(2,4)-pyrimidina-4(2,4)-pyridina-1(4,5)-pyrazolacyclononapha ne-4⁵-carboxamide | ¹H NMR (400 MHz, Chloroform-*d*) δ 9.40 (s, 1H), 8.44 (d, *J* = 7.3 Hz, 1H), 8.38-8.34 (m, 2H), 8.29 (s, 1H), 8.13 (s, 1H), 8.05 (s, 1H), 7.71 (s, 1H), 7.60 (d, J = 8.1 Hz, 1H), 7.43 (t, *J* = 7.9 Hz, 1H), 7.21 (d, *J* = 7.6 Hz, 1H), 6.54 (d, *J =* 5.7 Hz, 1H), 4.68 (td, *J* = 9.7, 2.9 Hz, 1H), 4.30-4.24 (m, 3H), 4.08 (dt, *J* = 9.4, 4.5 Hz, 1H), 3.79 (s, 3H), 2.83 (s, 3H), 2.18-2.14 (m, 2H), 1.45 (d, *J* = 6.6 Hz, 3H); 549.24 [M+H]⁺ |
| 126 | 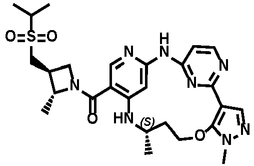 | ((S)-1¹,6-dimethyl-1¹H-9-oxa-3,5-diaza-2(2,4)-pyrimidina-4(2,4)-pyridina-1(4,5)-pyrazolacyclononapha ne-4⁵-yl)((2R,38)-3-((isopropylsulfonyl) methyl)-2-methylazetidin-1-yl)methanone | 555.3 [M+H]⁺ |
| 127 | 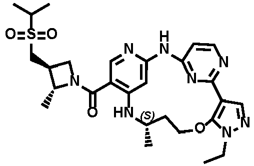 | ((S)-1¹-ethyl-6-methyl-1¹H-9-oxa-3,5-diaza-2(2,4)-pyrimidina-4(2,4)-pyridina-1(4,5)-pyrazolacyclononapha ne-4⁵-yl)((2R,3S)-3-((isopropylsulfonyl) methyl)-2-methylazetidin-1-yl)methanone | 569.3 [M+H]⁺ |
| 128 | 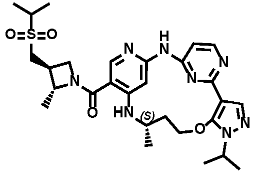 | ((S)-1¹-isopropyl-6-methyl-1¹H-9-oxa-3,5-diaza-2(2,4)-pyrimidina-4(2,4)-pyridina-1(4,5)-pyrazolacyclononapha ne-4⁵-yl)((2R,3S)-3-((isopropylsulfonyl) methyl)-2-methylazetidin-1-yl)methanone | 583.3 [M+H]⁺ |
| 129 | 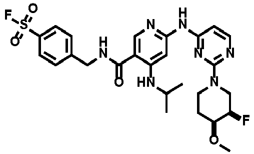 | 4-((6-((2-(cis-3-fluoro-4-methoxypiperidin-1-yl)pyrimidin-4-yl)amino)-4-(isopropylamino)nico tinamido)methyl)benz enesulfonyl fluoride | ¹H NMR (400 MHz, Chloroform-*d*) δ 8.46 (d, *J* = 7.2 Hz, 1H), 8.39 (s, 1H), 8.01 (d, *J* = 6.0 Hz, 1H), 7.93 (d, *J* = 8.4 Hz, 2H), 7.62 (d, *J* = 8.2 Hz, 2H), 6.40 (s, 1H), 6.31 (d, *J* = 6.1 Hz, 1H), 5.45 (d, *J* = 5.7 Hz, 2H), 5.30 (s, 3H), 4.12 (q, *J* = 7.2 Hz, 2H), 3.58-3.48 (m, 3H), 3.45 (s, 3H), 2.05 (s, 3H), 1.24 (d, *J* = 1.9 Hz, 6H); 576.25 [M+H]⁺ |
| 130 | 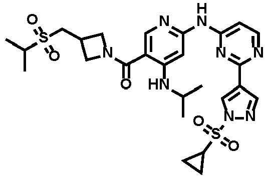 | (6-((2-(1-(cyclopropylsulfonyl )-1H-pyrazol-4-yl)pyrimidin-4-yl)amino)-4-(isopropylamino)pyri din-3-yl) (3-((isopropylsulfonyl) methyl)azetidin-1-yl)methanone | ¹H NMR (400 MHz, Chloroform-d) δ 8.64 (s, 1H), 8.44 (d, *J* = 5.8 Hz, 2H), 8.11 (d, *J* = 7.2 Hz, 1H), 8.04 (d, *J* = 9.6 Hz, 1H), 7.23-7.09 (m, 2H), 4.56 (s, 2H), 4.18 (s, 2H), 3.78 (dd, *J =* 13.2, 6.6 Hz, 1H), 3.47-3.32 (m, 1H), 3.28 (d, *J* = 7.6 Hz, 2H), 3.10 (dt, *J* = 13.8, 6.9 Hz, 1H), 2.89-2.78 (m, 1H), 1.53 (dd, *J* = 4.8, 2.1 Hz, 2H), 1.43 (d, *J* = 6.9 Hz, 6H), 1.36 (d, *J* = 6.4 Hz, 6H), 1.22 (dd, *J* = 8.0, 1.9 Hz, 2H); 603.3 [M+H]⁺ |
| 131 | 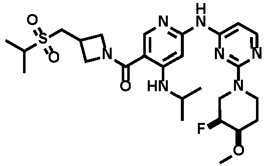 | (6-((2-(cis-3-fluoro-4-methoxypiperidin-1-yl)pyrimidin-4-yl)amino)-4-(isopropylamino)pyri din-3-yl) (3-((isopropylsulfonyl) methyl)azetidin-1-yl)methanone | ¹H NMR (400 MHz, Chloroform-*d*) δ 8.07 (d, *J* = 5.6 Hz, 2H), 8.00 (s, 1H), 7.31 (s, 1H), 6.25 (d, *J* = 5.6 Hz, 1H), 4.88-4.66 (m, 1H), 4.53 (d, *J* = 8.5 Hz, 2H), 4.42 (dt, *J* = 14.0, 7.1 Hz, 1H), 4.24-4.02 (m, 3H), 3.87-3.70 (m, 2H), 3.67-3.56 (m, 2H), 3.50 (s, 3H), 3.43-3.32 (m, 1H), 3.27 (d, *J* = 7.6 Hz, 2H), 3.16-3.02 (m, 1H), 2.05-1.92 (m, 1H), 1.84-1.76 (m, 1H), 1.42 (d, J = 6.9 Hz, 6H), 1.29 (dd, *J* = 6.3, 1.8 Hz, 6H); 564.3 [M+H]⁺ |
| 132 | 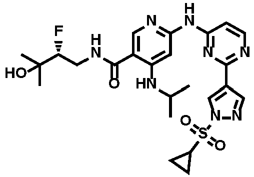 | (R)-6-((2-(1-(cyclopropylsulfonyl )-1H-pyrazol-4-yl)pyrimidin-4-yl)amino)-N-(2-fluoro-3-hydroxy-3-methylbutyl)-4-(isopropylamino)nico tinamide | ¹H NMR (400 MHz, Chloroform-d) δ 9.11 (d, *J* = 7.4 Hz, 1H), 8.74 (s, 1H), 8.61 (s, 1H), 8.39 (s, 1H), 8.35 (d, *J* = 5.5 Hz, 2H), 7.41 (s, 1H), 6.99 (d, *J* = 5.8 Hz, 1H), 4.64-4.45 (m, 1H), 4.12 (q, *J* = 7.1 Hz, 1H), 3.95-3.58 (m, 4H), 2.89 (tt, *J* = 8.0, 4.7 Hz, 1H), 1.52 (tt, *J* = 6.0, 3.2 Hz, 2H), 1.34-1.31 (m, 12H), 1.26 (d, *J* = 2.1 Hz, 2H); 547.30 [M+H]⁺ |
| 133 | 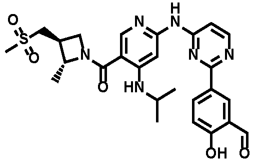 | 2-hydroxy-5-(4-((4-(isopropylamino)-5-((2R,3S)-2-methyl-3-((methylsulfonyl)met hyl)azetidine-1-carbonyl)pyridin-2-yl)amino)pyrimidin-2-yl)benzaldehyde | ¹H NMR (400 MHz, Chloroform-*d*) δ 11.26 (s, 1H), 10.02 (s, 1H), 8.70 (d, *J* = 2.2 Hz, 1H), 8.62 (dd, *J* = 8.8, 2.2 Hz, 1H), 8.50 (d, *J* = 5.7 Hz, 1H), 8.14 (s, 1H), 8.05 (s, 1H), 7.29 (s, 1H), 7.17 (d, *J* = 5.7 Hz, 1H), 7.07 (d, *J* = 8.7 Hz, 1H), 4.65 (t, *J* = 8.9 Hz, 1H), 4.56-4.48 (m, 1H), 4.02 (q, *J* = 5.5 Hz, 1H), 3.85-3.76 (m, 1H), 3.41-3.27 (m, 2H), 2.95 (s, 3H), 2.90-2.81 (m, 1H), 2.12 (s, 1H), 1.55 (d, *J* = 6.2 Hz, 3H), 1.33 (t, *J* = 6.1 Hz, 6H); 539.31 [M+H]⁺ |
| 134 | 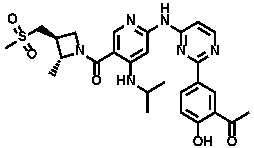 | 1-(2-hydroxy-5-(4-((4-(isopropylamino)-5-((2R,3S)-2-methyl-3-((methylsulfonyl)methyl)azetidine-1-carbonyl)pyridin-2-yl)amino)pyrimidin-2-yl)phenyl)ethan-1-one | ¹H NMR (400 MHz, Chloroform-*d*) δ 12.58 (s, 1H), 8.89 (d, *J* = 2.1 Hz, 1H), 8.55 (dd, *J* = 8.7, 2.1 Hz, 1H), 8.49 (d, *J* = 5.7 Hz, 1H), 8.03 (s, 2H), 7.35 (s, 1H), 7.14 (d, *J* = 5.8 Hz, 1H), 7.04 (d, *J* = 8.8 Hz, 1H), 4.65 (t, *J* = 9.0 Hz, 1H), 4.53 (dt, *J* = 11.8, 6.2 Hz, 1H), 4.04-3.97 (m, 1H), 3.86-3.76 (m, 1H), 3.41-3.27 (m, 2H), 2.95 (s, 3H), 2.85 (dd, *J* = 13.3, 6.1 Hz, 1H), 2.78 (s, 3H), 2.12 (s, 1H), 1.55 (d, *J* = 6.2 Hz, 3H), 1.33 (t, *J* = 5.9 Hz, 6H); 553.26 [M+H]⁺ |

### <Experimental Example 1> Evaluation of EGFR C797S enzyme inhibition activity

The compounds according to the present invention were reacted together with purified human EGFR (d746-750/T790M/C797S) (668-end, SignalChem) enzyme and a specific substrate and evaluated for enzyme inhibitory activity as described below. The reaction buffer consisted of 40 mM Tris-HCl pH 7.4, 20 mM MgCl₂, 0.5 mg/ml BSA, and 50 µM DTT, and all reactions of the test substances were performed in reaction buffers. The compounds were serially diluted in 12 steps from a 10 mM DMSO stock, and the enzyme activity was measured at the final compound concentrations of 10, 3, 1, 0.3, 0.1, 0.03, 0.01, 0.003, 0.001, 0.0003, 0.0001, and 0 µM. In the assay, human EGFR (d746-750/T790M/C797S) (3 ng) enzyme was reacted with purified ATP (5 µM) and enzyme substrate (0.2 µg) at 25°C for 1 hour, and the enzyme activity was determined using the in vitro ADP-GloTM kinase assay (Promega). The enzyme activity inhibition was measured via luminescence by reacting the enzyme activity reaction solution, ADP-Glo reaction solution, and enzyme activity detection solution in a 2:2:1 ratio. Based on the luminescence of the enzyme activity of the solvent control group that was not treated with the compound, the degree of the enzyme activity inhibition depending on the treatment concentration of each compound was calculated, wherein the concentration of each compound that inhibited the enzyme activity by 50% was determined as the IC₅₀ (nM) value, which was obtained using GraphPad Prism 8.4.3 (GraphPad software Inc., San Diego). Results thereof are summarized in Table 3 below.

**[Table 3]**

| Example compound | EGFR DTC Enzyme | Example compound | EGFR DTC Enzyme | Example compound | EGFR DTC Enzyme | Example compound | EGFR DTC Enzyme |
|---|---|---|---|---|---|---|---|
| 1 | A | 40 | B | 61 | B | 83 | A |
| 2 | A | 41 | A | 62 | A | 84 | A |
| 6 | A | 42 | A | 64 | B | 85 | A |
| 7 | A | 43 | A | 65 | A | 86 | A |
| 9 | A | 44 | B | 66 | A | 96 | B |
| 10 | A | 45 | B | 67 | A | 97 | A |
| 11 | B | 46 | B | 68 | A | 98 | A |
| 13 | B | 47 | B | 69 | A | 99 | B |
| 14 | A | 48 | A | 70 | A | 100 | A |
| 15 | B | 49 | A | 71 | A | 101 | B |
| 17 | A | 50 | A | 72 | A | 102 | A |
| 22 | B | 51 | A | 73 | A | 105 | A |
| 25 | B | 52 | A | 74 | A | 110 | A |
| 26 | B | 53 | A | 75 | A | 115 | A |
| 28 | B | 54 | B | 76 | A | 116 | A |
| 32 | B | 55 | B | 77 | A | 117 | A |
| 34 | A | 56 | B | 78 | A | 119 | A |
| 35 | A | 57 | A | 79 | B | 120 | A |
| 36 | A | 58 | B | 80 | A | | |
| 37 | A | 59 | A | 81 | A | | |
| 39 | A | 60 | A | 82 | A | | |

| | | | | | | | |
|---|---|---|---|---|---|---|---|
| (A: IC₅₀ < 50 nM; B: 50 nM ≤ IC₅₀ < 500 nM; C: 500 nM ≤ IC₅₀ < 5000 nM; D: 5000 nM ≤ IC₅₀) | | | | | | | |

### <Experimental Example 2> Evaluation of inhibitory activity on proliferation of Ba/F3 cell overexpressing EGFR C797S

The following experiments were performed to evaluate the inhibitory activity of the compounds according to the present invention on the proliferation of Ba/F3 expressing EGFR Del19/T790M/C797S mutations.

Ba/F3 cells were cultured in RPMI-1640 medium supplemented with 10% fetal bovine serum (FBS), and 3000 cells were seeded into each well of a white clear bottom 96 well plate (Corning) 24 hours prior to compound treatment. The compounds were diluted in dimethyl sulfoxide (3-fold dilution, total of 12 concentrations) and injected at 1 µl each so that the final concentration was 0.2 nM to 5 µM. After 72 hours post treatment with compounds, living cells were reacted in a 37°C, CO₂ incubator for 72 hours, followed by 10 minutes at room temperature using Cell Titer-Glo luminescent cell-viability reagent (Promega), and the luminescence intensity thereof was measured using a reader (SynergyNeo2, Biotek). The result values were calculated as a cell growth rate (%) compared to the control group. GI₅₀ values were calculated using the GraphPad Prism version 8.4.3 (GraphPad software Inc., San Diego) program. The results are summarized in Table 4 below.

**[Table 4]**

| Example compound | Ba/F3 (EGFR DTC) | Example compound | Ba/F3 (EGFR DTC) | Example compound | Ba/F3 (EGFR DTC) | Example compound | Ba/F3 (EGFR DTC) |
|---|---|---|---|---|---|---|---|
| 1 | B | 46 | B | 76 | B | 103 | A |
| 6 | B | 47 | B | 78 | B | 105 | B |
| 7 | B | 48 | A | 79 | B | 110 | B |
| 9 | B | 49 | B | 80 | A | 114 | A |
| 10 | B | 50 | B | 81 | A | 115 | A |
| 13 | B | 51 | B | 82 | A | 116 | A |
| 15 | B | 53 | B | 83 | A | 119 | A |
| 17 | B | 55 | B | 86 | A | 120 | B |
| 24 | B | 56 | B | 87 | B | 121 | A |
| 25 | B | 57 | B | 88 | A | 123 | A |
| 27 | B | 58 | B | 89 | A | 124 | B |
| 29 | B | 61 | B | 91 | B | 125 | B |
| 30 | B | 62 | A | 92 | B | 130 | A |
| 32 | B | 64 | B | 93 | B | 131 | B |
| 33 | B | 65 | B | 94 | B | 132 | A |
| 34 | B | 66 | B | 95 | B | 133 | B |
| 35 | B | 68 | B | 96 | B | 134 | B |
| 36 | B | 70 | B | 97 | B | | |
| 39 | B | 71 | B | 98 | A | | |
| 40 | B | 72 | A | 99 | A | | |
| 41 | B | 73 | B | 100 | A | | |
| 42 | B | 74 | B | 102 | A | | |

| | | | | | | | |
|---|---|---|---|---|---|---|---|
| (A: GI₅₀ < 500 nM; B: 500 nM ≤ GI₅₀ < 5000 nM; C: 5000 nM ≤ GI₅₀) | | | | | | | |

As shown in Tables 3 and 4 above, it can be seen that the compounds according to Examples of the present invention exhibited high inhibitory activity against enzymes or cell lines including the EGFR C797S mutations.

As described above, the present invention has been described in detail through preferred Preparation Examples, Examples and Experimental Examples, but the scope of the present invention is not limited to the specific compounds according to Examples of the present invention and should be interpreted based on the attached claims. Further, those skilled in the art should understand that various modifications and variations can be made without departing from the scope of the present invention.

## Claims

1. A compound represented by the following Chemical Formula A, a stereoisomer thereof, or a pharmaceutically acceptable salt thereof: 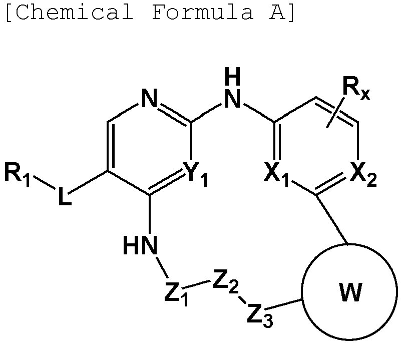 in Chemical Formula A above,
X₁ and X₂ are each independently CH or N;
Rx is -H, -C₁₋₆alkyl, -C₁₋₆hydroxyalkyl, -NH₂, -OH, -O-C₁₋₆alkyl, or -halo;
Y₁ is CH or N;
ring W is a 5-6 membered heteroaryl or phenyl {wherein at least one H of the 5-6 membered heteroaryl or phenyl ring may be substituted with -C₁₋₆alkyl, -C₁₋₆alkenyl, -C₁₋₆alkynyl, -C₁₋₆alkyl-cycloalkyl, -C₁₋₆hydroxyalkyl, -OH, -O-C₁₋₆alkyl, =O, -halo, or cycloalkyl, and -CH₂- of the heterocycloalkyl ring may be substituted with -S(=O)₂-};
Z₁ is - (CH₂CH₂)-, - (CH₂CH₂CH₂)-, or - (CH₂CH₂CH₂CH₂)-{wherein at least one H of the -(CH₂CH₂)-, -(CH₂CH₂CH₂), or - (CH₂CH₂CH₂CH₂)- may be substituted with -C₁₋₃alkyl};
Z₂ is -(CH₂)- or -O-;
Z₃ is a bond (null), -(CH₂)-, or -(CH₂CH₂)-,
L is -C(=O)-, -C(=O)-NRₐ-, -C(=O)-O-, -NRₐ-C(=O)-, or - O-C(=O)-;
R₁ is -H, -C₁₋₆alkyl, -C₁₋₆cyanoalkyl, -C₁₋₆aminoalkyl, - C₁₋₆hydroxyalkyl, -C₁₋₆alkyl-O-C₁₋₆alkyl, -C₁₋₆hydroxyhaloalkyl, -C₁₋₆haloalkyl, - (CH₂)m-C(=O)-OC₁₋₆alkyl, -(CH₂)m-S(=O)₂-R_{b}, - (CH₂)m-cycloalkyl, -(CH₂)m-heterocycloalkyl, -(CH₂)m-aryl, or -(CH₂)m-heteroaryl {wherein at least one H of the -(CH₂)m-cycloalkyl, -(CH₂)m-heterocycloalkyl, -(CH₂)m-aryl, or - (CH₂)m-heteroaryl may be substituted with -C₁₋₆alkyl, -C₁-₆cyanoalkyl, -C₁₋₆aminoalkyl, -C₁₋₆hydroxyalkyl, -C₁₋₆haloalkyl, -CN, -NO₂, -NH₂, -NH-C₁₋₆alkyl, -N(C₁₋₆alkyl) (C₁₋₆alkyl), -OH, -O-C₁₋₆alkyl, =O, -S(=O)₂-C₁₋₆alkyl, -S(=O)₂-halo, -S(=O)₂-cycloalkyl, -C₁₋₆alkyl-S(=O)₂-C₁₋₆alkyl, -NR_{c}-S(=O)₂-C₁₋₆alkyl, -halo, - (CH₂)n-cycloalkyl, - (CH₂)n-heterocycloalkyl, - (CH₂)n-aryl, or - (CH₂)n-heteroaryl [wherein at least one H of the (CH₂)n-cycloalkyl, -(CH₂)n-heterocycloalkyl, -(CH₂)n-aryl, or -(CH₂)n-heteroaryl ring may be substituted with - C₁₋₆alkyl, -C₁₋₆aminoalkyl, -C₁₋₆hydroxyalkyl, -C₁₋₆haloalkyl, =O, -S(=O)₂-C₁₋₆alkyl, -C₁₋₆alkyl-S(=O)₂-C₁₋₆alkyl, -halo, cycloalkyl, or heterocycloalkyl], and -CH₂- of the -(CH₂)ₘ-cycloalkyl or -(CH₂)m-heterocycloalkyl may be substituted with -S(=O)₂-};
Rₐ is -H or -C₁₋₆alkyl;
R_{b} is -H, -C₁₋₆alkyl, -NH₂, -NH-C₁₋₆alkyl, or -N(C₁₋₆alkyl)(C₁₋₆alkyl);
R_{c} is -H or -C₁₋₆alkyl; and
m and n are each independently 0, 1, 2, or 3.

2. The compound represented by Chemical Formula A, the stereoisomer thereof, or the pharmaceutically acceptable salt thereof of claim 1, wherein
X₁ and X₂ are each independently CH or N {wherein at least one of X₁ or X₂ is N};
Rx is -H, or -C₁₋₆alkyl;
Y₁ is CH or N;
ring W is a 5-6 membered heteroaryl {wherein at least one H of the 5-6 membered heteroaryl ring may be substituted with -C₁₋₆alkyl or cycloalkyl};
Z₁ is - (CH₂CH₂)-, - (CH₂CH₂CH₂)-, or - (CH₂CH₂CH₂CH₂)-{wherein at least one H of the -(CH₂CH₂)-, -(CH₂CH₂CH₂), or - (CH₂CH₂CH₂CH₂)- may be substituted with -C₁₋₃alkyl};
Z₂ is -(CH₂)- or -O-;
Z₃ is a bond (null), -(CH₂)-, or -(CH₂CH₂)-,
L is -C(=O)-, -C(=O)-NRₐ-, or -C(=O)-O-;
R₁ is -H, -C₁₋₆alkyl, -C₁₋₆cyanoalkyl, -C₁₋₆haloalkyl, - (CH₂)m-S(=O)₂-R_{b}, - (CH₂)m-cycloalkyl, - (CH₂)m-heterocycloalkyl, or -(CH₂)m-aryl {wherein at least one H of the -(CH₂)m-cycloalkyl, -(CH₂)m-heterocycloalkyl, or -(CH₂)m-aryl may be substituted with -C₁₋₆alkyl, -C₁₋₆cyanoalkyl, -C₁-₆aminoalkyl, -C₁₋₆hydroxyalkyl, -C₁₋₆haloalkyl, -S(=O)₂-C₁-₆alkyl, -C₁₋₆alkyl-S(=O)₂-C₁₋₆alkyl, -NR_{c}-S(=O)₂-C₁₋₆alkyl, - halo, or -(CH₂)n-heterocycloalkyl [wherein at least one H of the -(CH₂)n-heterocycloalkyl ring may be substituted with - C₁₋₆alkyl or heterocycloalkyl], and -CH₂- of the -(CH₂)m-cycloalkyl or -(CH₂)m-heterocycloalkyl may be substituted with -S(=O)₂-};
Rₐ is -H or -C₁₋₆alkyl;
R_{b} is -H or -C₁₋₆alkyl;
R_{c} is -H or -C₁₋₆alkyl; and
m and n are each independently 0, 1, 2, or 3.

3. The compound represented by Chemical Formula A, the stereoisomer thereof, or the pharmaceutically acceptable salt thereof of claim 1, wherein
ring W is 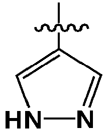 or phenyl {wherein at least one H of the 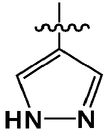 or phenyl ring may be substituted with -C₁₋₆alkyl, -C₁₋₆hydroxyalkyl, -O-C₁₋₆alkyl, -S (=O) ₂-C₁₋₆alkyl, or -S(=O)₂-cycloalkyl}.

4. The compound, the stereoisomer thereof or the pharmaceutically acceptable salt thereof of claim 1, wherein
the compound represented by Chemical Formula A is selected from the group consisting of the following compounds:
| |
|---|
| (S)-N-(cyanomethyl)-1¹,6-dimethyl-1¹H-9-oxa-3,5-diaza-2(2,4)-pyrimidina-4(2,4)-pyridina-1(4,5)-pyrazolacyclononaphane-4⁵-carboxamide |
| (R)-N-(cyanomethyl)-1¹, 6-dimethyl-1¹H-9-oxa-3,5-diaza-2(2,4)-pyrimidina-4(2,4)-pyridina-1(4,5)-pyrazolacyclononaphane-4⁵-carboxamide |
| (S)-N-(cyclopentylmethyl)-1¹,6-dimethyl-1¹H-9-oxa-3,5-diaza-2(2,4)-pyrimidina-4 (2,4)-pyridina-1(4,5)-pyrazolacyclononaphane-4⁵-carboxamide |
| (S)-1¹,6-dimethyl-N-(4,4,4-trifluorobutyl)-1¹H-9-oxa-3,5-diaza-2(2,4)-pyrimidina-4(2,4)-pyridina-1(4,5)-pyrazolacyclononaphane-4⁵-carboxamide |
| (S)-1¹,6-dimethyl-N-(3,3,3-trifluoropropyl)-1¹H-9-oxa-3,5-diaza-2(2,4)-pyrimidina-4(2,4)-pyridina-1(4,5)-pyrazolacyclononaphane-4⁵-carboxamide |
| (S)-1¹,6-dimethyl-N-(3-(4-methylpiperazin-1-yl)phenyl)-1¹H-9-oxa-3,5-diaza-2(2,4)-pyrimidina-4(2,4)-pyridina-1(4,5)-pyrazolacyclononaphane-4⁵-carboxamide |
| (S)-1¹,6-dimethyl-N-(3-(trifluoromethyl)phenyl)-1¹H-9-oxa-3,5-diaza-2(2,4)-pyrimidina-4(2,4)-pyridina-1(4,5)-pyrazolacyclononaphane-4⁵-carboxamide |
| methyl (S)-1¹,6-dimethyl-1¹H-9-oxa-3,5-diaza-2(2,4)-pyrimidina-4(2,4)-pyridina-1(4,5)-pyrazolacyclononaphane-4⁵-carboxylate |
| (S)-1¹,6-dimethyl-N-(2,2,3,3,3-pentafluoropropyl)-1¹H-9-oxa-3,5-diaza-2(2,4)-pyrimidina-4(2,4)-pyridina-1(4,5)-pyrazolacyclononaphane-4⁵-carboxamide |
| (S)-N-(3,3-difluorocyclobutyl)-1¹,6-dimethyl-1¹H-9-oxa-3,5-diaza-2(2,4)-pyrimidina-4(2,4)-pyridina-1(4,5)-pyrazolacyclononaphane-4⁵-carboxamide |
| (S)-(1¹,6-dimethyl-1¹H-9-oxa-3,5-diaza-2(2,4)-pyrimidina-4(2,4)-pyridina-1(4,5)-pyrazolacyclononaphane-4⁵-yl)(3-((methylsulfonyl)methyl)azetidin-1-yl)methanone |
| ((S)-1¹,6-dimethyl-1¹H-9-oxa-3,5-diaza-2(2,4)-pyrimidina-4(2,4)-pyridina-1(4,5)-pyrazolacyclononaphane-4⁵-yl)((2R,3S)-2-methyl-3-((methylsulfonyl)methyl)azetidin-1-yl)methanone |
| (S)-(1¹,6-dimethyl-1¹H-9-oxa-3,5-diaza-2,4(2,4)-dipyridina-1(4,5)-pyrazolacyclononaphane-4⁵-yl)(3-((methylsulfonyl)methyl)azetidin-1-yl)methanone |
| (S)-(1¹,6-dimethyl-1¹H-10-oxa-3,5-diaza-2,4(2,4)-dipyridina-1(4,5)-pyrazolacyclodecaphane-4⁵-yl)(3-((methylsulfonyl)methyl)azetidin-1-yl)methanone |
| (R)-(1¹,6-dimethyl-1¹H-10-oxa-3,5-diaza-2,4(2,4)-dipyridina-1(4,5)-pyrazolacyclodecaphane-4⁵-yl)(3-((methylsulfonyl)methyl)azetidin-1-yl)methanone |
| (S)-1¹,6-dimethyl-N-(2-(methylsulfonyl)ethyl)-1¹H-9-oxa-3,5-diaza-2(2,4)-pyrimidina-4(2,4)-pyridina-1(4,5)-pyrazolacyclononaphane-4⁵-carboxamide |
| (S)-1¹,6-dimethyl-N-(1-(methylsulfonyl)azetidin-3-yl)-1¹H-9-oxa-3,5-diaza-2(2,4)-pyrimidina-4(2,4)-pyridina-1(4,5)-pyrazolacyclononaphane-4⁵-carboxamide |
| (6S)-1¹,6-dimethyl-N-((tetrahydrofuran-2-yl)methyl)-1¹H-9-oxa-3,5-diaza-2(2,4)-pyrimidina-4(2,4)-pyridina-1(4,5)-pyrazolacyclononaphane-4⁵-carboxamide |
| (S)-(1¹,6-dimethyl-1¹H-9-oxa-3,5-diaza-2(2,4)-pyrimidina-4(2,4)-pyridina-1(4,5)-pyrazolacyclononaphane-4⁵-yl)(6-(methylsulfonyl)-2,6-diazaspiro[3.3]heptan-2-yl)methanone |
| (S)-1¹,6-dimethyl-N-(3-(N-methylmethylsulfonamido)-5-(4-methylpiperazin-1-yl)phenyl)-1¹H-9-oxa-3,5-diaza-2(2,4)-pyrimidina-4(2,4)-pyridina-1(4,5)-pyrazolacyclononaphane-4⁵ carboxamide |
| (S)-1¹,6-dimethyl-N-(3,3,3-trifluoropropyl)-1¹H-9-oxa-3,5-diaza-4(2,4)-pyrimidina-2(2,4)-pyridina-1(4,5)-pyrazolacyclononaphane-4⁵-carboxamide |
| 1¹,6-dimethyl-N-(4,4,4-trifluorobutyl)-1¹H-10-oxa-3,5-diaza-2(2,4)-pyrimidina-4 (2,4)-pyridina-1(4,5)-pyrazolacyclodecaphane-4⁵-carboxamide |
| (S)-1¹,6-dimethyl-1¹H-9-oxa-3,5-diaza-2(2,4)-pyrimidina-4(2,4)-pyridina-1(4,5)-pyrazolacyclononaphane-4⁵-carboxylic acid |
| (6S)-1¹,6-dimethyl-N-(3-(trifluoromethyl)cyclohexyl)-1¹H-9-oxa-3,5-diaza-2(2,4)-pyrimidina-4(2,4)-pyridina-1(4,5)-pyrazolacyclononaphane-4⁵-carboxamide |
| (6S)-N-(3-(dimethylamino)-1-phenylpropyl)-1¹,6-dimethyl-1¹H-9-oxa-3,5-diaza-2(2,4)-pyrimidina-4(2,4)-pyridina-1(4,5)-pyrazolacyclononaphane-4⁵-carboxamide |
| (S)-(1¹,6-dimethyl-1¹H-9-oxa-3,5-diaza-2(2,4)-pyrimidina-4(2,4)-pyridina-1(4,5)-pyrazolacyclononaphane-4⁵-yl)(3-((isopropylsulfonyl)methyl)azetidin-1-yl)methanone |
| (S)-(1¹-cyclopropyl-6-methyl-1¹H-9-oxa-3,5-diaza-2(2,4)-pyrimidina-4(2,4)-pyridina-1(4,5)-pyrazolacyclononaphane-4⁵ yl)(3-((methylsulfonyl)methyl)azetidin-1-yl)methanone |
| (S)-1¹,6-dimethyl-N-(3,3,3-trifluoropropyl)-1¹H-9-oxa-3,5-diaza-2,4(2,4)-dipyridina-1(4,5)-pyrazolacyclononaphane-4⁵-carboxamide |
| (S)-1¹,2⁶,6-trimethyl-N-(3,3,3-trifluoropropyl)-1¹H-9-oxa-3,5-diaza-2,4(2,4)-dipyridina-1(4,5)-pyrazolacyclononaphane-4⁵-carboxamide |
| (S)-1¹,6-dimethyl-N-(3-((methylsulfonyl)methyl)phenyl)-1¹H-9-oxa-3,5-diaza-2(2,4)-pyrimidina-4(2,4)-pyridina-1(4,5)-pyrazolacyclononaphane-4⁵-carboxamide |
| ((S)-1¹,6-dimethyl-1¹H-9-oxa-3,5-diaza-2(2,4)-pyrimidina-4(2,4)-pyridina-1(4,5)-pyrazolacyclononaphane-4⁵-yl)((2R,3S)-3-((isopropylsulfonyl)methyl)-2-methylazetidin-1-yl)methanone |
| ((S)-1¹-ethyl-6-methyl-1¹H-9-oxa-3,5-diaza-2(2,4)-pyrimidina-4(2,4)-pyridina-1(4,5)-pyrazolacyclononaphane-4⁵-yl)((2R,3S)-3-((isopropylsulfonyl)methyl)-2-methylazetidin-1-yl)methanone |
| ((S)-1¹-isopropyl-6-methyl-1¹H-9-oxa-3,5-diaza-2(2,4)-pyrimidina-4(2,4)-pyridina-1(4,5)-pyrazolacyclononaphane-4⁵-yl)((2R,3S)-3-((isopropylsulfonyl)methyl)-2-methylazetidin-1-yl)methanone |

5. A pharmaceutical composition comprising:
the compound represented by Chemical Formula A, the stereoisomer thereof, or the pharmaceutically acceptable salt thereof according to any one of claims 1 to 4, and a pharmaceutically acceptable additive.

6. A pharmaceutical composition for preventing or treating cancer, comprising:
the compound, the stereoisomer thereof, or the pharmaceutically acceptable salt thereof according to any one of claims 1 to 4 as an active ingredient.

7. The pharmaceutical composition of claim 6, wherein the pharmaceutical composition inhibits EGFR.

8. The pharmaceutical composition of claim 7, wherein the pharmaceutical composition inhibits at least any one selected from the group consisting of EGFR Del19/C797S, EGFR L858R/C797S, EGFR Del19/T790M/C797S, and EGFR L858R/T790M/C797S.

9. The pharmaceutical composition of claim 6, wherein the cancer is at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, nonsmall cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone tumor, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid tumor, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, hematological cancer, and thymic carcinoma.

10. Use of the compound represented by Chemical Formula A, the stereoisomer thereof, or the pharmaceutically acceptable salt thereof according to any one of claims 1 to 4, for use in the manufacture of a medicament for the treatment or prevention of EGFR-related diseases.

11. A method for treating or preventing EGFR-related diseases, comprising administering to a subject in need thereof a therapeutically effective amount of the compound represented by Chemical Formula A, the stereoisomer thereof, or the pharmaceutically acceptable salt thereof according to any one of claims 1 to 4.

12. A method for treating or preventing at least one disease, comprising administering to a subject in need thereof a therapeutically effective amount of the compound represented by Chemical Formula A, the stereoisomer thereof, or the pharmaceutically acceptable salt thereof according to any one of claims 1 to 4, the disease being selected from the group consisting of the following diseases:
pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, nonsmall cell lung cancer, tongue cancer, astrocytoma, small cell lung cancer, pediatric brain cancer, pediatric lymphoma, pediatric leukemia, small bowel cancer, meningioma, esophageal cancer, glioma, renal pelvis cancer, renal cancer, heart cancer, duodenal cancer, malignant soft tissue tumor, malignant bone tumor, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid tumor, gastrointestinal stromal tumor, Wilms' tumor, breast cancer, sarcoma, penile cancer, pharyngeal cancer, gestational choriocarcinoma, cervical cancer, endometrial cancer, uterine sarcoma, prostate cancer, metastatic bone cancer, metastatic brain cancer, mediastinal cancer, rectal cancer, rectal carcinoid tumor, vaginal cancer, spinal cord cancer, vestibular schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, hematological cancer, and thymic carcinoma.
